(19) Europäisches Patentamt
European Patent Office
Office européen des brevets

(11) EP 1 363 161 B1

(12) EUROPEAN PATENT SPECIFICATION

(45) Date of publication and mention of the grant of the patent:
**12.09.2007 Bulletin 2007/37**

(51) Int Cl.:
*G03C 7/30* (2006.01)

(21) Application number: **03011016.7**

(22) Date of filing: **16.05.2003**

# (54) Silver halide color photographic lightsensitive material

Farbphotographisches lichtempfindliches Silberhalogenidmaterial

Matériau photographique couleur à l'halogénure d'argent sensible à la lumière

(84) Designated Contracting States:
**AT BE BG CH CY CZ DE DK EE ES FI FR GB GR HU IE IT LI LU MC NL PT RO SE SI SK TR**

(30) Priority: **17.05.2002 JP 2002143552**

(43) Date of publication of application:
**19.11.2003 Bulletin 2003/47**

(73) Proprietor: **FUJIFILM Corporation**
**Minato-ku**
**Tokyo (JP)**

(72) Inventors:
- **Mikoshiba, Hisashi**
  **Minami-Ashigara-shi,**
  **Kanagawa (JP)**
- **Kato, Yasuhiro**
  **Minami-Ashigara-shi,**
  **Kanagawa (JP)**
- **Matsuda, Naoto**
  **Minami-Ashigara-shi,**
  **Kanagawa (JP)**

(74) Representative: **HOFFMANN EITLE**
**Patent- und Rechtsanwälte**
**Arabellastrasse 4**
**81925 München (DE)**

(56) References cited:
**US-A- 5 418 122**

- **PATENT ABSTRACTS OF JAPAN vol. 018, no. 482 (C-1247), 8 September 1994 (1994-09-08) -& JP 06 157442 A (FUJI PHOTO FILM CO LTD), 3 June 1994 (1994-06-03)**
- **LATTERMANN, G. ET AL: "Liquid crystalline piperazine and triazacyclononane derivatives" JOURNAL OF THE CHEMICAL SOCIETY, CHEMICAL COMMUNICATIONS (1992), (15), 1091-2 , XP009014884**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 363 161 B1

Printed by Jouve, 75001 PARIS (FR)

## Description

The present invention relates to a bis type amide compound of novel structure. More particularly, the present invention relates to a bis type amide compound which exhibits excellent performance as a high-boiling organic solvent being superior in dissolving power, dispersibility, dispersion stability, etc., and relates to a silver halide color photographic lightsensitive material which contains this bis type amide compound.

It is common practice to use photographically useful compounds of low solubility in water by dissolving each thereof in an appropriate oil-droplet forming agent, namely, a high-boiling organic solvent, subsequently dispersing the resultant solution in a solution of hydrophilic organic colloid, for example, gelatin in the presence of a surfactant, and thereafter incorporating the thus obtained dispersion in a hydrophilic organic colloid layer or layers. Generally, a phthalic ester compound and a phosphoric ester compound are used as such a high-boiling organic solvent.

It is demanded that the high-boiling organic solvent have the following wide range of capabilities. The high-boiling organic solvent must be excellent in the dissolving power of photographically useful compounds, affinity with gelatin, dispersibility and dispersion stability therein, etc.; should not lower the reactivity of photographically useful compounds (color forming performance of coupler, or redox reactivity with redox compounds such as a color mixing prevention agent); must be able to optimally regulate the hue of dye formed by a color forming reaction; and must be excellent in the chemical stability of itself. Further, the high-boiling organic solvent should not accelerate the decomposition of dispersed photographically useful compounds; should not accelerate the fading of formed dye by light, humidity and heat; should not accelerate such a phenomenon (stain) that a photographically useful compound contained in the lightsensitive material, or a compound which is present in a processing solution and which, after processing, remains in the lightsensitive material is decomposed by light, humidity or heat to result in dyeing; should not deteriorate the emulsion and the storability of latent image; must be inexpensive; and must be easily available.

Phosphoric ester and phthalic ester compounds have been employed as the high-boiling organic solvent which can satisfy the above requirements for capabilities. However, in place thereof, it is proposed to employ amide compounds which, due to high polarity, are excellent in the capability of regulating the absorption waveform of formed dye and color forming properties. In particular, as a high-boiling organic solvent of high performance, compounds having a plurality of amido bonds in each molecule have been proposed in Jpn. Pat. Appln. KOKAI Publication No. (hereinafter referred to as JP-A-) 2-262654 and JP-A-8-110624. However, these amide compounds have a drawback in that the capability of regulating the absorption waveform of formed dye is still unsatisfactory.

In color lightsensitive materials, sensitizing dyes are used for spectral sensitization. The sensitizing dyes are needed at the exposure but must be completely decolorized after the processing. When the decolorization is incomplete, tint attributed to the sensitizing dyes would remain on white background, thereby posing a problem from the viewpoint of faithful color reproduction. This is known as residual color. The degree of residual color differs depending on the type of added high-boiling organic solvent. It has been found that the compounds of the present invention which will be described in detail below are superior to the known amide compounds in the effect of improvement to residual color.

In silver halide color photographic lightsensitive materials, it is well known that an aromatic primary amine color developing agent oxidized with exposed silver halides as an oxidizing agent reacts with a coupler, thereby producing a dye, such as indophenol, indoaniline, indamine, azomethine, phenoxazine or phenazine, so that an image is formed. In this photography, the subtractive color process is employed, and each color image is formed by yellow, magenta and cyan dyes.

For formation of a cyan dye image among these, it is common practice to employ phenol or naphthol couplers. However, the dyes formed from such couplers exhibit undesirable absorption in the region from yellow to magenta, thereby posing a problem of deteriorating the color reproduction. Thus, it is demanded to resolve this problem.

Especially in recent years, demands on the system in which image information is digitized and subjected to image processing and in which thereafter a silver halide color photographic lightsensitive material is exposed according to the information, known as the digital photography, are increasing. Particularly in this system, there is a strong demand for a silver halide color photographic lightsensitive material of large color reproduction range wherein formed dyes do not exhibit the above undesirable absorption.

On the other hand, high saturation and large color reproduction range are demanded on reversal photographic films. Since the method of emphasizing an interlayer effect has a drawback of, for example, deterioration in processing dependence, it is now demanded to realize the high saturation and large color reproduction range by the use of a coupler of excellent hue.

As means for solving this problem, there have been proposed heterocyclic compounds as described in, for example, U.S.P.'s 4,728,598 and 4,873,183 and EP 0249453A2. However, the couplers described therein have fatal drawbacks such as low coupling activity and poor dye durability. As a coupler which overcomes these problems, there have been proposed pyrrolotriazole couplers as described in U.S.P. 5,256,526 and EP 0545300. Although these couplers are excellent in hue and coupling activity, absorption bands attributed to association of formed dyes are present in the couplers, thereby posing a critical technical task of controlling the association condition so as to obtain desired absorption

waveforms. Moreover, it has been revealed that color photographic lightsensitive materials wherein these couplers are employed are not satisfactory in color image durability, thereby necessitating further improvement.

JP 06157442 discloses 2,6-dialkoxy-4-carbamoylphenol derivatives and antioxidant compositions comprising the same. These compounds may be used for preventing deterioration of dyes, coloring matter and synthetic polymers.

US 5,418,122 discloses silver halide color photographic materials comprising a support with at least one layer containing *inter alia* 2,6-dialkoxy-4-carbamoylphenol derivatives. These compounds improve the fastness of photographic materials.

G. Latterman et al., Journal of the Chemical Society, Chem. Communications, 1992, p. 1091-1092, discloses liquid crystalline piperazine and triazacyclonane derivatives. This publication describes the mesophase behaviour of the corresponding ammonium salts.

The first object of the present invention is to provide a high-boiling organic solvent which exhibits high dissolving power and which is excellent in dispersibility and dispersion stability, and further to provide a lightsensitive material containing the high-boiling organic solvent. The second object of the present invention is to provide a high-boiling organic solvent which improves durability of color images and staining, and further to provide a lightsensitive material containing the high-boiling organic solvent. The third object of the present invention is to provide a high-boiling organic solvent which exhibits high capability of regulating the absorption waveform of color forming dyes, and further to provide a lightsensitive material containing the high-boiling organic solvent. The fourth object of the present invention is to control the state of association of formed dyes derived from pyrrolotriazole couplers so as to realize desired absorption waveforms. The fifth object of the present invention is to provide a lightsensitive material capable of forming images of high durability.

The inventors have conducted extensive and intensive studies with respect to the relationship between the structures of bis type amide compounds and the photographic performance realized thereby. As a result, a compound of unknown completely novel structure represented by the following general formula (I) has been found. Further, it has been found that the above objects can be attained by incorporating the compound of the following general formula (I) combined with a pyrrolotriazole coupler of the following general formula (II) in a lightsensitive material. That is, the above objects of the present invention have been attained by the following means.

(1) A silver halide color photographic lightsensitive material, characterized by comprising a compound of the following general formula (I):

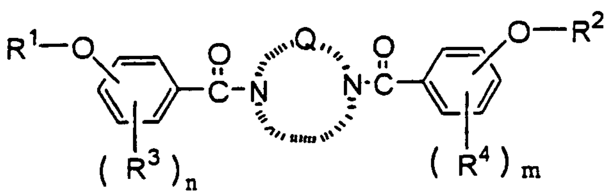

(I)

wherein:

each of $R^1$ and $R^2$ independently represents a substituted or unsubstituted alkyl group having 1 to 30 carbon atoms, or a substituted or unsubstituted alkenyl group having 3 to 30 carbon atoms;
each of $R^3$ and $R^4$ independently represents an unsubstituted alkyl group having 1 to 10 carbon atoms, a halogen atom or an unsubstituted alkoxy group having 1 to 10 carbon atoms;
each of n and m is independently an integer of 0 to 3; and
Q represents an atomic group needed to form a 5-membered or 6-membered heterocycle in cooperation with two nitrogen atoms.

(2) The silver halide color photographic lightsensitive material according to item (1) above, characterized in that a coupler of the following general formula (II) is contained.

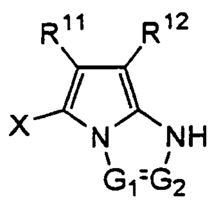

(II)

In the formula (II), X represents a hydrogen atom, or a group which may be split off at a coupling reaction with products of oxidation of an aromatic primary amine color developing agent. Each of $R^{11}$ and $R^{12}$ independently represents an electron withdrawing group whose Hammett substituent constant σp value is 0.20 or greater, provided that the sum of $R^{11}$ and $R^{12}$ σp values is 0.65 or greater. Either of $G_1$ and $G_2$ is a nitrogen atom while the rest is -C ($R^{13}$)=, wherein $R^{13}$ represents a hydrogen atom or a substituent.

(3) A compound of the following general formula (I) .

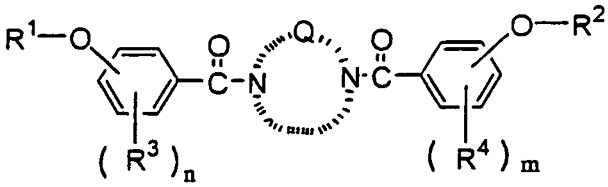

(I)

In the formula (I), each of $R^1$ and $R^2$ independently represents an unsubstituted alkyl group having 10 to 20 carbon atoms. Each of $R^3$ and $R^4$ independently represents an unsubstituted alkyl group having 1 to 10 carbon atoms, a halogen atom or an unsubstituted alkoxy group having 1 to 10 carbon atoms;

each of n and m is independently an integer of 0 to 2; and

Q represents an atomic group needed to form a 5-membered or 6-membered heterocycle in cooperation with two nitrogen atoms;

provided that the following compounds are not included:

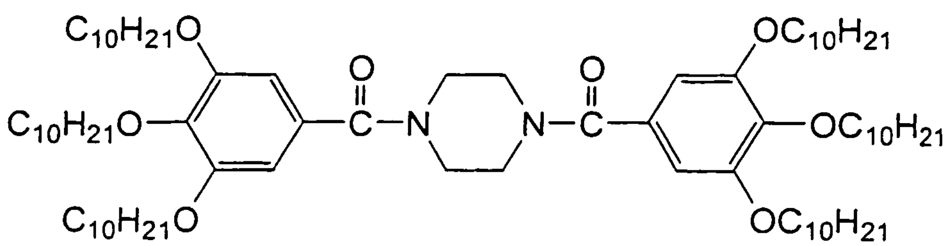

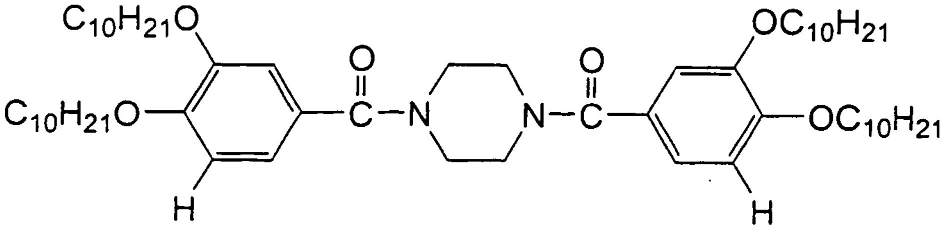

(4) A method of forming an image, characterized by comprising subjecting the silver halide color photographic lightsensitive material according to item (2) above to processes including reversal processing.

(5) The silver halide color photographic lightsensitive material according to item (2) above, wherein each of $R^1$ and

$R^2$ independently represents an unsubstituted alkyl group having 10 to 20 carbon atoms.

(6) The silver halide color photographic lightsensitive material according to item (1) or (2) above, wherein m = n = O.

(7) The compound according to item (3) above, wherein m = n = O.

The present invention will be described in detail below. First, the expression "Hammett substituent constant σp value" used herein will be briefly described. Hammett's rule is a rule of thumb advocated by L. P. Hammett in 1935 for quantitatively considering the effect of substituents on the reaction or equilibrium of benzene derivatives, and the appropriateness thereof is now widely recognized. The substituent constant determined in the Hammett's rule involves σp value and σm value. These values can be found in a multiplicity of general publications, and are detailed in, for example, "Lange's Handbook of Chemistry" 12th edition by J. A. Dean, 1979 (Mc Graw-Hill) and "Kagaku no Ryoiki" special issue, no. 122, p.p. 96 to 103, 1979 (Nankodo). Although in the present invention, substituents are limited by the Hammett substituent constant σp or described thereby, this should not be construed as limitation to only substituents whose values are known from literature and can be found in the above publications, and should naturally be construed as including substituents whose values, even if unknown from literature, would be included in stated ranges when measured according to the Hammett's rule. Further, although the compounds represented by the general formula (II) of the present invention, or the general formula (III) described later as being preferred, are not benzene derivatives, the σp value is used, irrespective of the position of substitution, as a scale for evaluating the electronic effect of substituents thereof. In the present invention, the σp value will be used in the above meaning below. The terminology "lipophilicity" used in the present invention refers to the property of compound whose solubility in water at room temperature is 10% or less.

Herein, the heterocycle refers to a ring having a heteroatom therein. The heterocycles include those having aromaticity, and further may have substituents. As the heteroatom, there can be mentioned N, S, O or P. Herein, the substituents and the substituents which may be had by the alkyl group, alkenyl group, alkynyl group, cycloalkyl group, cycloalkenyl group, aryl group and heterocycle are not limited as long as they are capable of substitution unless otherwise specified. For example, the substituents can be any of an alkyl group, an alkenyl group, an alkynyl group, a cycloalkyl group, a cycloalkenyl group, an aryl group, a heterocyclic group, an acyl group, an acyloxy group, an acylamino group, an alkyloxy group, an aryloxy group, a heterocyclic oxy group, an alkyloxycarbonyl group, an aryloxycarbonyl group, a heterocyclic oxycarbonyl group, an alkylcarbamoyl group, an arylcarbamoyl group, an alkylsulfonyl group, an arylsulfonyl group, an alkylsulfamoyl group, an arylsulfamoyl group, an alkylsulfonamido group, an arylsulfonamido group, an alkylamino group, an arylamino group, an alkylsulfinyl group, an arylsulfinyl group, an alkylthio group, an arylthio group, a mercapto group, a hydroxy group, a cyano group, a nitro group, a hydroxyamino group, a halogen atom and the like.

The compounds represented by the general formula (I) of the present invention (hereinafter also referred to as "amide compounds of the present invention") will be described in detail below.

Each of $R^1$ and $R^2$ independently represents a substituted or unsubstituted alkyl group having 1 to 30 carbon atoms, or a substituted or unsubstituted alkenyl group having 3 to 30 carbon atoms. As appropriate substituents, there can be mentioned, for example, a halogen atom, an alkyl group, an alkenyl group, an alkynyl group, a cycloalkyl group, a cycloalkenyl group, an aryl group, a heterocyclic group, a cyano group, a hydroxy group, a nitro group, a carboxy group, a sulfo group, an amino group, an alkyloxy group, an aryloxy group, an acylamino group, an alkylamino group, an anilino group, a ureido group, a sulfamoylamino group, an alkylthio group, an arylthio group, an alkyloxycarbonylamino group, a sulfonamido group, a carbamoyl group, a sulfamoyl group, a sulfonyl group, an alkyloxycarbonyl group, a heterocyclic oxy group, an azo group, an acyloxy group, a carbamoyloxy group, a silyloxy group, an aryloxycarbonylamino group, an imido group, a heterocyclic thio group, a sulfinyl group, a phosphonyl group, an aryloxycarbonyl group, an acyl group and the like. Further, there can be mentioned the substituents represented by for example, a halogen atom (e.g., a chlorine atom or a bromine atom); an alkyl group, an alkenyl group, an alkynyl group, a cycloalkyl group and a cycloalkenyl group (e.g., a linear or branched alkyl group having 1 to 32 carbon atoms, an aralkyl group having 7 to 38 carbon atoms, an alkenyl group having 2 to 32 carbon atoms, a linear or branched alkynyl group having 2 to 32 carbon atoms, a linear or branched cycloalkyl group having 3 to 32 carbon atoms and a linear or branched cycloalkenyl group having 3 to 32 carbon atoms, such as methyl, ethyl, propyl, isopropyl, t-butyl, tridecyl, 2-methanesulfonylethyl, 3-(3-pentadecylphenoxy)propyl, 3-{4-{2-[4-(4-hydroxyphenylsulfonyl)phenoxy]dodecanamido}phenyl}propy 1, 2-ethoxytridecyl, trifluoromethyl, cyclopentyl, and 3-(2,4-di-t-amylphenoxy)propyl); an aryl group (e.g., phenyl, 4-t-butylphenyl, 2,4-di-t-amylphenyl or 4-tetradecanamidophenyl); a heterocyclic group (e.g., imidazolyl, pyrazolyl, triazolyl, 2-furyl, 2-thienyl, 2-pyrimidinyl or 2-benzothiazolyl); a cyano group, a hydroxy group, a nitro group, a carboxy group, a sulfo group and an amino group; an alkyloxy group (e.g., methoxy, ethoxy, 2-methoxyethoxy, 2-dodecylethoxy or 2-methanesulfonylethoxy); an aryloxy group (e.g., phenoxy, 2-methylphenoxy, 4-t-butylphenoxy, 3-nitrophenoxy, 3-t-butyloxycarbamoylphenoxy or 3-methoxycarbamoylphenoxy); an acylamino group (e.g., acetamido, benzamido, tetradecanamido, 2-(2,4-di-t-amylphenoxy)butanamido, 4-(3-t-butyl-4-hydroxyphenoxy)butanamido or 2-{4-(4-hydroxyphenylsulfonyl)phenoxy}decanamido); an alkylamino group (e.g., methylamino, butylamino, dodecylamino, diethylamino or methylbutylamino); an anilino group (e.g., phenylamino, 2-chloroanilino, 2-chloro-5-tetradecanaminoanilino, 2-chloro-5-dodecyloxycarbonylanilino, N-acetylanilino or 2-chloro-5-{2-(3-t-butyl-4-hydroxyphenoxy)dodecanamido}anilino); a ureido group (e.g., phenylureido, methylureido or N,N-dibutylureido); a sulfamoylamino group (e.g., N,N-dipropylsulfamoylamino or N-methyl-N-decylsulfamoylamino); an alkylthio group (e.g., methylthio, octylthio, tetradecylthio, 2-phenoxyethylthio, 3-phenoxypropylthio or 3-(4-t-butylphenoxy)propylthio); an arylthio group (e.g., phenylthio, 2-butoxy-5-t-octylphenylthio, 3-pentadecylphenylthio, 2-carboxyphenylthio or 4-tetradecanamidophenylthio); an alkyloxycarbonylamino group (e.g., methoxycarbonylamino or tetradecyloxycarbonylamino); a sulfonamido group (e.g., methanesulfonamido, hexadecanesulfonamido, benzenesulfonamido, p-toluenesulfonamido, octadecanesulfonamido or 2-methoxy-5-t-butylbenzenesulfonamido); a carbamoyl group (e.g., N-ethylcarbamoyl, N,N-dibutylcarbamoyl, N-(2-dodecyloxyethyl)carbamoyl, N-methyl-N-dodecylcarbamoyl or N-{3-(2,4-di-t-amylphenoxy)propyl}carbamoyl); a sulfamoyl group (e.g., N-ethylsulfamoyl, N,N-dipropylsulfamoyl, N-(2-dodecyloxyethyl)sulfamoyl, N-ethyl-N-dodecylsulfamoyl or N,N-diethylsulfamoyl); a sulfonyl group (e.g., methanesulfonyl, octanesulfonyl, benzenesulfonyl or toluenesulfonyl); an alkyloxycarbonyl group (e.g., methoxycarbonyl, butyloxycarbonyl, dodecyloxycarbonyl or octadecyloxycarbonyl); a heterocyclic oxy group (e.g., 1-phenyltetrazol-5-oxy or 2-tetrahydropyranyloxy); an azo group (e.g., phenylazo, 4-methoxyphenylazo, 4-pivaloylaminophenylazo or 2-hydroxy-4-propanoylphenylazo); an acyloxy group (e.g., acetoxy); a carbamoyloxy group (e.g., N-methylcarbamoyloxy or N-phenylcarbamoyloxy); a silyloxy group (e.g., trimethylsilyloxy or dibutylmethylsilyloxy); an aryloxycarbonylamino group (e.g., phenoxycarbonylamino); an imido group (e.g., N-succinimido, N-phthalimido or 3-octadecenylsuccinimido); a heterocyclic thio group (e.g., 2-benzothiazolylthio or 2,4-diphenoxy-1,3,5-triazole-6-thio or 2-pyridylthio); a sulfinyl group (e.g., dodecanesulfinyl, 3-pentadecylphenylsulfinyl or 3-phenoxypropylsulfinyl); a phosphonyl group (e.g., phenoxyphosphonyl, octyloxyphosphonyl or phenylphosphonyl); an aryloxycarbonyl group (e.g., phenoxycarbonyl); and an acyl group (e.g., acetyl, 3-phenylpropanoyl, benzoyl or 4-dodecyloxybenzoyl).

The alkyl of a group having alkyl moiety among the above substituents refers to a linear or branched alkyl or cycloalkyl. The substituted alkyl groups comprehend an aralkyl, an alkenyl, an alkynyl and a cycloalkenyl.

Accordingly, the alkyloxycarbonyl groups comprehend linear or branched alkyloxycarbonyl, aralkyloxycarbonyl, alkenyloxycarbonyl, alkynyloxycarbonyl, cycloalkyloxycarbonyl and cycloalkenoxycarbonyl groups.

Examples of the groups represented by $R^1$ and $R^2$ include methyl, ethyl, isopropyl, n-butyl, n-propyl, n-pentyl, n-hexyl, n-heptyl, 3-methoxybutyl, 2-cyanoethyl, 2-chloroethyl, n-octyl, 2-ethyl-1-hexyl, n-decyl, prenyl, geranyl and oleyl. It is preferred that $R^1$ and $R^2$ represent unsubstituted alkyl groups having 10 to 20 carbon atoms. Further, it is preferred that $R^1$ and $R^2$ represent the same groups.

Each of $R^3$ and $R^4$ independently represents an unsubstituted alkyl group having 1 to 10 carbon atoms, a halogen atom or an unsubstituted alkoxy group having 1 to 10 carbon atoms.

More specifically, the substituents represented by $R^3$ and $R^4$ can be, for example, a halogen atom (e.g., a chlorine atom or a bromine atom); an alkyl group such as methyl, ethyl, propyl, isopropyl, t-butyl, an alkyloxy group, e.g., methoxy, ethoxy.

The alkyl of a group having alkyl moiety among the above substituents refers to a linear or branched alkyl or cycloalkyl.

It is preferred that $R^3$ and $R^4$ represent the same groups.

Q represents an atomic group needed to form a 5-membered or 6-membered heterocycle in cooperation with two nitrogen atoms. In particular, the heterocycle is preferably in the form of a 6-membered ring, more preferably a piperazine ring.

Each of n and m is independently an integer of 0 to 3, preferably 0 to 2. More preferably, each of n and m is 0 or 1. 0 is most preferred. It is preferred that n and m be the same integers. Most preferably, both n and m are 0.

The compounds of the general formula (I) preferably have nondiffusing groups (known as ballasting groups) from the viewpoint of nondiffusivity. Among them, the compounds wherein $R^1$ and $R^2$ represent ballasting groups are preferred.

From the viewpoint of nondiffusivity, the molecular weight of the amide compound of the present invention is preferably 400 or greater, more preferably 450 or greater. Further, the molecular weight of the amide compound of the present invention is still more preferably 500 or greater, most preferably 530 or greater.

With respect to the upper limit of the molecular weight of the amide compound of the present invention, from the viewpoint of the number of polar groups per unit mass, the molecular weight is preferably 800 or less, more preferably 750 or less, still more preferably 700 or less, and most preferably 650 or less.

Therefore, the molecular weight of the amide compound of the present invention is preferably in the range of 400 to 800, more preferably 450 to 750, still more preferably 500 to 700, and most preferably 530 to 650.

Specific examples of the compounds represented by the general formula (I) of the present invention will be set out below, which however in no way limit the scope of the present invention.

1.

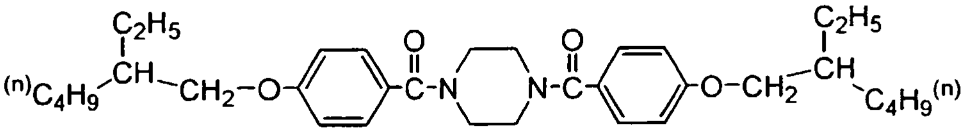

2.

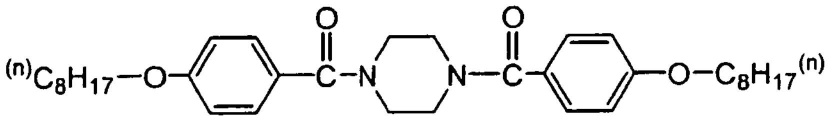

3.

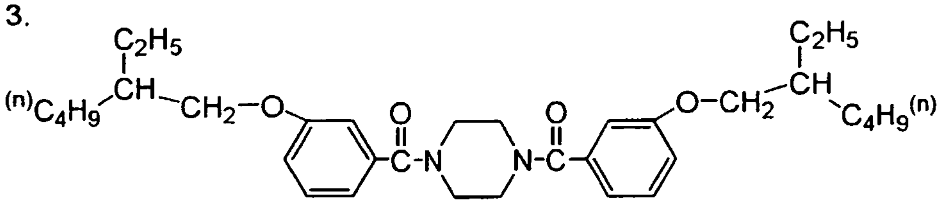

4.

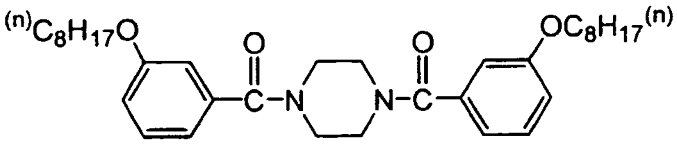

5.

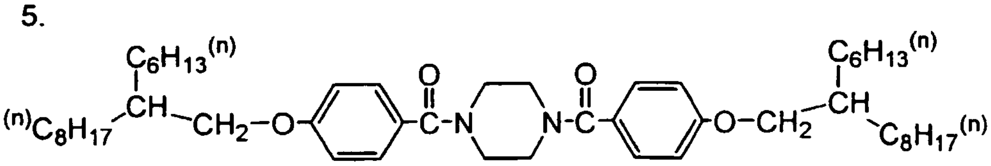

6.

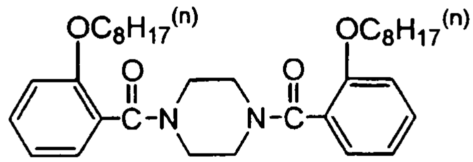

7.

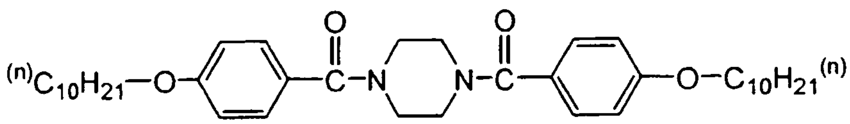

8.

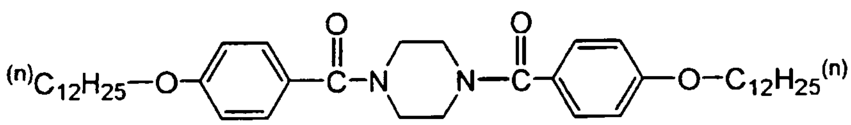

9.

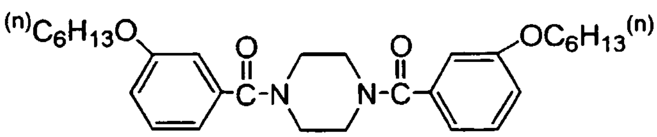

10.

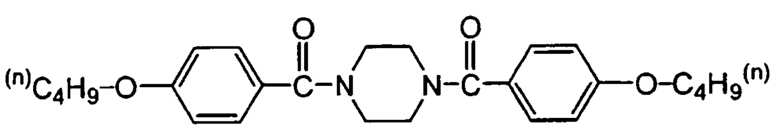

11.

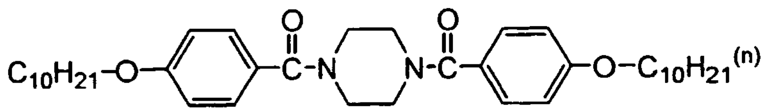

12.

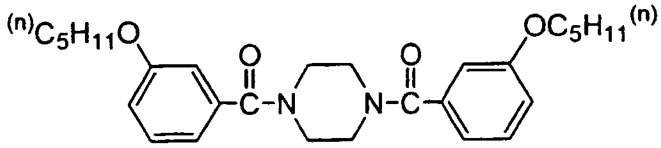

13.

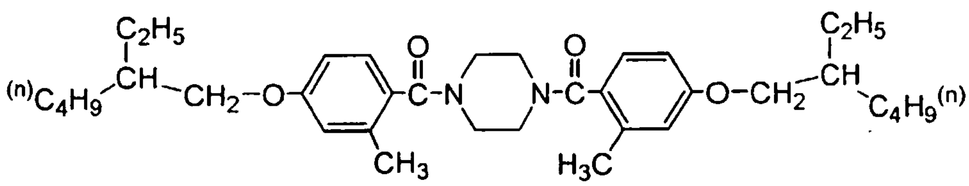

14.

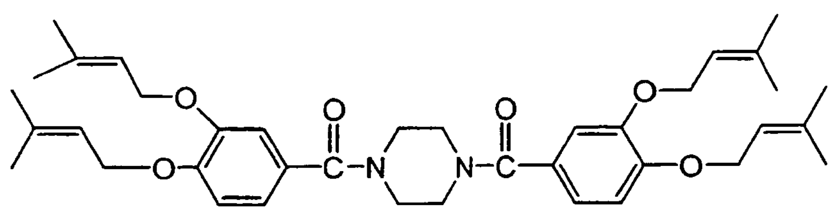

15.

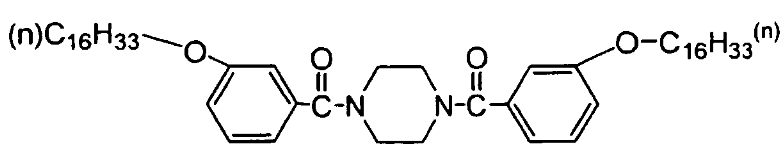

16.

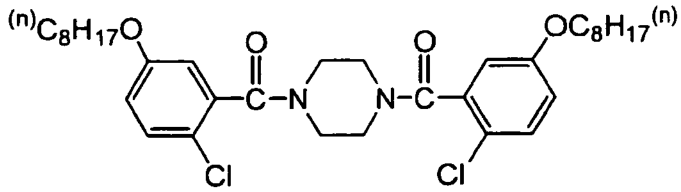

17.

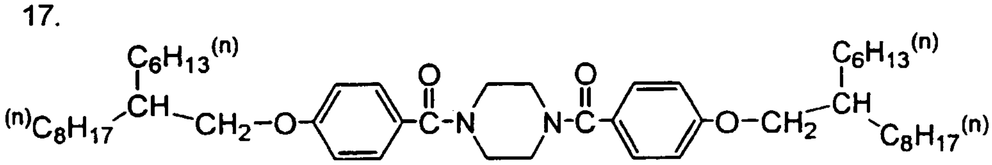

18.

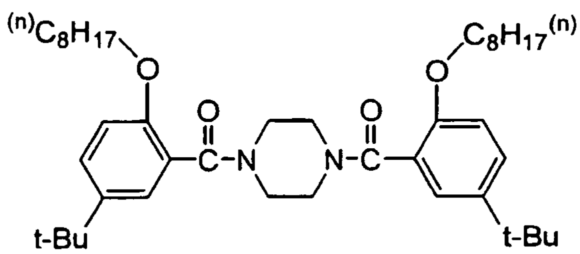

19.

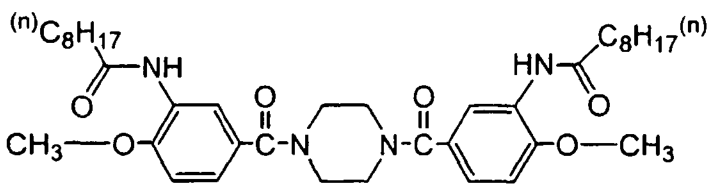

20.

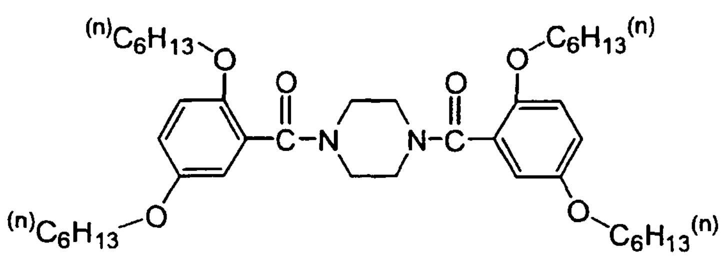

21.

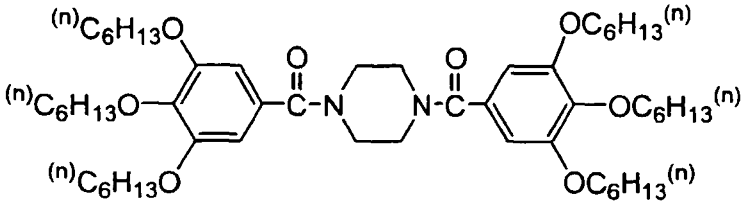

22.

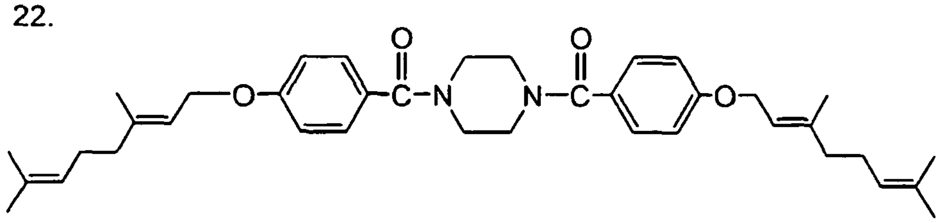

23.

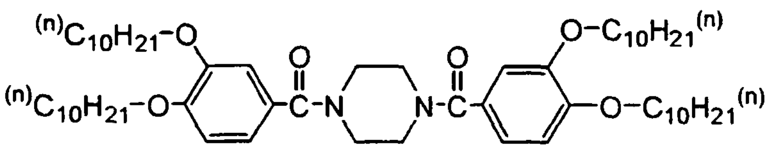

24.

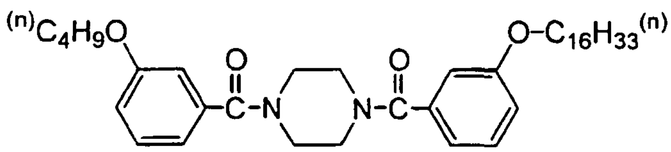

The cyan couplers of the general formula (II) for use in the present invention (hereinafter also referred to as "cyan couplers of the present invention") will be described in detail below.

In the cyan couplers of the present invention, X represents a hydrogen atom, or a group which can be split off at a coupling reaction with products of oxidation of an aromatic primary amine color developing agent. The split off group is preferably, for example, any of a halogen atom, an alkyloxy group, an aryloxy group, an acyloxy group, an alkyl- or arylsulfonyloxy group, an acylamino group, an alkyl- or arylsulfonamido group, an alkyloxycarbonyloxy group, an aryloxycarbonyloxy group, an alkylthio group, an arylthio group, a heterocyclic thio group, a carbamoylamino group, a carbamoyloxy group, a heterocyclic carbonyloxy group, a 5-membered or 6-membered nitrogenous heterocyclic group, an imido group and an arylazo group. These groups may further have substituents as exemplified above with respect to $R^3$ and $R^4$ of the general formula (I).

More specifically, X represents, for example, a hydrogen atom; a halogen atom (e.g., a fluorine atom, a chlorine atom or a bromine atom); an alkyloxy group (e.g., ethoxy, dodecyloxy, methoxyethylcarbamoylmethoxy, carboxypropyloxy, methanesulfonylethoxy or ethoxycarbonylmethoxy); an aryloxy group (e.g., 4-methylphenoxy, 4-chlorophenoxy, 4-methoxyphenoxy, 4-carboxyphenoxy, 3-ethoxycarbonylphenoxy, 3-acetylaminophenoxy or 2-carboxyphenoxy); an acyloxy group (e.g., acetoxy, tetradecanoyloxy or benzoyloxy); an alkyl- or arylsulfonyloxy group (e.g., methanesulfonyloxy or toluenesulfonyloxy); an acylamino group (e.g., dichloroacetylamino or heptafluorobutyrylamino); an alkyl- or arylsulfonamido group (e.g., methanesulfonylamino, trifluoromethanesulfonylamino or p-toluenesulfonylamino); an alkyloxycarbonyloxy group (e.g., ethoxycarbonyloxy or benzyloxycarbonyloxy); an aryloxycarbonyloxy group (e.g., phenoxycarbonyloxy); an alkylthio group, an arylthio group or a heterocyclic thio group (e.g., dodecylthio, 1-carboxydodecylthio, phenylthio, 2-butoxy-5-t-octylphenylthio or tetrazolylthio); a carbamoylamino group (e.g., N-methylcarbamoylamino or N-phenylcarbamoylamino); a carbamoyloxy group (e.g., N,N-diethylcarbamoyloxy, N-ethylcarbamoyloxy or N-ethyl-N-phenylcarbamoyloxy); a heterocyclic carbonyloxy group (e.g., morpholinocarbonyloxy or piperidinocarbonyloxy); a 5-membered or 6-membered nitrogenous heterocyclic group (e.g., imidazolyl, pyrazolyl, triazolyl, tetrazolyl or 1,2-dihydro-2-oxo-1-pyridyl); an imido group (e.g., succinimido or hydantoinyl); or an arylazo group (e.g., phenylazo or 4-methoxyphenylazo). Apart from these, X may be in the form of a bis type coupler obtained by condensation of an aldehyde or ketone 4-equivalent coupler as a split off group bonded through a carbon atom. Further, X may contain a photographically useful group, such as a development inhibitor or a development accelerator.

Preferably, X represents a hydrogen atom, a halogen atom, an alkyloxy group having 1 to 32 carbon atoms, an aryloxy group having 6 to 32 carbon atoms, an alkylthio group having 1 to 32 carbon atoms, an arylthio group having 6 to 32 carbon atoms, a heterocyclic thio group having 2 to 32 carbon atoms, an alkyloxycarbonyloxy group having 2 to 32 carbon atoms, an aryloxycarbonyloxy group having 7 to 32 carbon atoms, a carbamoyloxy group having 1 to 32 carbon atoms, a heterocyclic carbonyloxy group having 3 to 32 carbon atoms, or a 5-membered or 6-membered nitrogenous heterocyclic group having 2 to 32 carbon atoms, the heterocyclic group capable of bonding with a coupling active site at a nitrogen atom. More preferably, X represents a hydrogen atom, a halogen atom, an alkylthio group, an arylthio group, an alkyloxycarbonyloxy group, an aryloxycarbonyloxy group, a carbamoyloxy group or a heterocyclic carbonyloxy group. Still more preferably, X represents a hydrogen atom, a halogen atom, an arylthio group, a carbamoyloxy group or a heterocyclic carbonyloxy group. Yet still more preferably, X represents a hydrogen atom or a heterocyclic carbonyloxy group. Most preferably, X represents a hydrogen atom.

In the cyan coupler of the present invention, both $R^{11}$ and $R^{12}$ represent an electron withdrawing group whose Hammett substituent constant σp value is 0.20 or greater, provided that the sum of $R^{11}$ and $R^{12}$ σp values is 0.65 or greater. The cyan coupler satisfying this requirement realizes color formation into cyan images. The sum of $R^{11}$ and $R^{12}$ σp values is preferably 0.70 or greater, and the upper limit thereof is about 2.0.

$R^{11}$ and $R^{12}$ will be described in detail below.

Each of $R^{11}$ and $R^{12}$ represents an electron withdrawing group of 0.20 or greater Hammett substituent constant σp value, preferably an electron withdrawing group of 0.30 or greater Hammett substituent constant σp value. The upper limit thereof is an electron withdrawing group of 1.0 or less Hammett substituent constant σp value.

As examples of $R^{11}$ and $R^{12}$ groups which are electron withdrawing groups of 0.20 or greater σp value, there can be mentioned an acyl group, an acyloxy group, a carbamoyl group, an alkyloxycarbonyl group, an aryloxycarbonyl group, a cyano group, a nitro group, a dialkylphosphono group, a diarylphosphono group, a diarylphosphinyl group, an alkylsulfinyl group, an arylsulfinyl group, an alkylsulfonyl group, an arylsulfonyl group, a sulfonyloxy group, an acylthio group, a sulfamoyl group, a thiocyanato group, a thiocarbonyl group, a halogenated alkyl group, a halogenated alkoxy group, a halogenated aryloxy group, a halogenated alkylamino group, a halogenated alkylthio group, an aryl group substituted with another electron withdrawing group of 0.20 or greater σp value, a heterocyclic group, a halogen atom, an azo group and a selenocyanato group.

$R^{11}$ and $R^{12}$ will be described in greater detail below. As the electron withdrawing group of 0.20 or greater σp value, there can be specifically mentioned an acyl group (e.g., acetyl, 3-phenylpropanoyl, benzoyl or 4-dodecyloxybenzoyl); an acyloxy group (e.g., acetoxy); a carbamoyl group (e.g., carbamoyl, N-ethylcarbamoyl, N-phenylcarbamoyl, N,N-dibutylcarbamoyl, N-(2-dodecyloxyethyl)carbamoyl, N-(4-n-pentadecanamido)phenylcarbamoyl, N-methyl-N-dodecylcarbamoyl or N-{3-(2,4-di-t-amylphenoxy)propyl}carbamoyl); an alkyloxycarbonyl group (e.g., methoxycarbonyl, ethoxycarbonyl, isopropyloxycarbonyl, tert-butyloxycarbonyl, isobutyloxycarbonyl, butyloxycarbonyl, dodecyloxycarbonyl, octadecyloxycarbonyl, cyclohexyloxycarbonyl or cyclohexenoxycarbonyl); an aryloxycarbonyl group (e.g., phenoxycarbonyl); a cyano group; a nitro group; a dialkylphosphono group (e.g., dimethylphosphono); a diarylphosphono group (e.g., diphenylphosphono); a diarylphosphinyl group (e.g., diphenylphosphinyl); an alkylsulfinyl group (e.g., 3-phenoxypropylsulfinyl); an arylsulfinyl group (e.g., 3-pentadecylphenylsulfinyl); an alkylsulfonyl group (e.g., methanesulfonyl or octanesulfonyl); an arylsulfonyl group (e.g., benzenesulfonyl or toluenesulfonyl); a sulfonyloxy group (e.g., methanesulfonyloxy or toluenesulfonyloxy); an acylthio group (e.g., acetylthio or benzoylthio); a sulfamoyl group (e.g., N-ethylsulfamoyl, N,N-dipropylsulfamoyl, N-(2-dodecyloxyethyl)sulfamoyl, N-ethyl-N-dodecylsulfamoyl or N,N-diethylsulfamoyl); a thiocyanato group; a thiocarbonyl group (e.g., methylthiocarbonyl or phenylthiocarbonyl); a halogenated alkyl group (e.g., trifluoromethane or heptafluoropropane); a halogenated alkyloxy group (e.g., trifluoromethyloxy); a halogenated aryloxy group (e.g., pentafluorophenyloxy); a halogenated alkylamino group (e.g., N,N-di(trifluoromethyl)amino); a halogenated alkylthio group (e.g., difluoromethylthio or 1,1,2,2-tetrafluoroethylthio); an aryl group substituted with another electron withdrawing group of 0.20 or greater σp value (e.g., 2,4-dinitrophenyl, 2,4,6-trichlorophenyl or pentachlorophenyl); a heterocyclic group (e.g., 2-benzoxazolyl, 2-benzothiazolyl, 1-phenyl-2-benzimidazolyl, 5-chloro-1-tetrazolyl or 1-pyrrolyl); a halogen atom (e.g., a chlorine atom or a bromine atom); an azo group (e.g., phenylazo); or a selenocyanato group.

The groups capable of further having a substituent among these substituents may further have the above substituents.

As preferred examples of $R^{11}$ and $R^{12}$ groups, there can be mentioned an acyl group having 2 to 32 carbon atoms, an acyloxy group having 2 to 32 carbon atoms, a carbamoyl group having 1 to 32 carbon atoms, an alkyloxycarbonyl group having 2 to 32 carbon atoms, an aryloxycarbonyl group having 7 to 32 carbon atoms, a cyano group, a nitro group, an alkylsulfinyl group having 1 to 32 carbon atoms, an arylsulfinyl group having 6 to 32 carbon atoms, an alkylsulfonyl group having 1 to 32 carbon atoms, an arylsulfonyl group having 6 to 32 carbon atoms, a sulfamoyl group having 0 to 32 carbon atoms, a halogenated alkyl group having 1 to 32 carbon atoms, a halogenated alkyloxy group having 1 to 32 carbon atoms, a halogenated alkylthio group having 1 to 32 carbon atoms, a halogenated aryloxy group having 7 to 32 carbon atoms, an aryl group having 7 to 32 carbon atoms substituted with two or more other electron withdrawing groups of 0.20 or greater σp value, and a 5 to 8-membered heterocyclic group having 1 to 36 carbon atoms wherein a nitrogen atom, an oxygen atom or a sulfur atom is contained.

As more preferred examples of $R^{11}$ and $R^{12}$ groups, there can be mentioned an alkyloxycarbonyl group having 2 to 32 carbon atoms, a nitro group, a cyano group, an arylsulfonyl group having 6 to 32 carbon atoms, a carbamoyl group having 1 to 32 carbon atoms and a halogenated alkyl group having 1 to 32 carbon atoms. $R^{11}$ most preferably represents a cyano group. $R^{12}$ especially preferably represents an alkyloxycarbonyl group having 2 to 32 carbon atoms, and most preferably represents a branched alkyloxycarbonyl group having 4 to 32 carbon atoms (in particular, a cycloalkyloxycarbonyl group).

In the cyan coupler of the present invention, each of $G_1$ and $G_2$ represents a nitrogen atom or a substituent of the formula $-C(R^{13})=$, provided that either of $G_1$ and $G_2$ is a nitrogen atom while the rest is a substituent of the formula $-C(R^{13})=$.

$R^{13}$ represents a hydrogen atom or a substituent. The substituent is as defined above with respect to $R^3$ and $R^4$, and examples thereof are also as mentioned there.

$R^{13}$ preferably represents a substituted or unsubstituted alkyl group having 1 to 30 carbon atoms, or a substituted or unsubstituted aryl group having 6 to 30 carbon atoms.

It is preferred that the cyan coupler of the present invention and the amide compound of the present invention be added to the same layer of a silver halide lightsensitive material. Further, the cyan coupler of the present invention is preferably added to a red-sensitive silver halide emulsion layer of a silver halide lightsensitive material. In that instance, it is preferred that the cyan coupler of the present invention be a so-called incorporated coupler. For this purpose, it is preferred that at least one of the $R^{11}$, $R^{12}$, $R^{13}$ and X groups be a so-called ballasting group (preferably, the total number of carbon atoms thereof is 10 or greater). With respect to the ballasting group, it is preferred that the total number of carbon atoms be in the range of 10 to 50. It is especially preferred that the ballasting group be had at $R^{13}$.

An especially preferred combination of groups for the cyan coupler of the general formula (II) according to the present invention is realized when X is a hydrogen atom, $R^{11}$ is a cyano group, $R^{12}$ is a branched alkyloxycarbonyl group having 4 to 32 carbon atoms, $G_1$ is a nitrogen atom, and $G_2$ is -C(wherein $R^{13}$ is preferably a substituted or unsubstituted aryl group having 6 to 30 carbon atoms.

Most preferred combination of groups for the cyan coupler of the general formula (II) according to the present invention is realized when X is a hydrogen atom, $R^{11}$ is a cyano group, $R^{12}$ is a cycloalkyloxycarbonyl group having 6 to 32 carbon atoms, $G_1$ is a nitrogen atom, and $G_2$ is $-C(R^{13})=$ wherein $R^{13}$ is preferably a substituted or unsubstituted aryl group having 6 to 30 carbon atoms.

Still preferably, the cyan coupler of the general formula (II) has the structure represented by the following general formula (III).

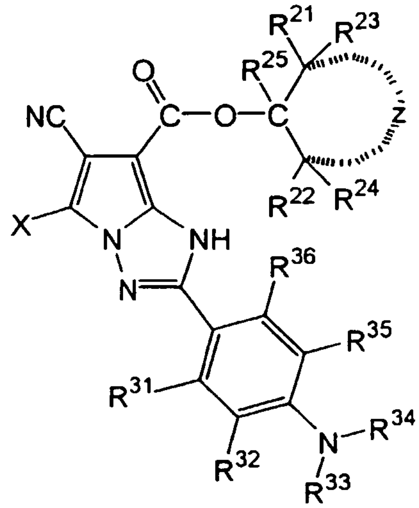

(III)

In the general formula (III), X has the same meaning as that of X of the general formula (II). Each of $R^{31}$, $R^{32}$, $R^{35}$ and $R^{36}$ independently represents a hydrogen atom or a substituent. $R^{33}$ represents a substituted or unsubstituted alkyl group, a substituted or unsubstituted alkenyl group, a substituted or unsubstituted alkynyl group, a substituted or unsubstituted aryl group, a substituted or unsubstituted cycloalkyl group, or a substituted or unsubstituted heterocyclic group. $R^{34}$ represents a hydrogen atom, or a substituent capable of bonding at a carbon atom. $R^{33}$ and $R^{34}$ may be bonded with each other to thereby form a ring.

Specifically, as the alkyl group, alkenyl group, alkynyl group, cycloalkyl group or cycloalkenyl group represented by $R^{33}$, there can be mentioned a linear or branched alkyl group having 1 to 32 carbon atoms, an aralkyl group having 7 to 32 carbon atoms, an alkenyl group having 2 to 32 carbon atoms, an alkynyl group having 2 to 32 carbon atoms, a cycloalkyl group having 3 to 32 carbon atoms, or a cycloalkenyl group having 3 to 32 carbon atoms. More specifically, there can be mentioned, for example, methyl, ethyl, propyl, isopropyl, t-butyl, tridecyl, 2-methanesulfonylethyl, 3-(3-pentadecylphenoxy)propyl, 3-{4-{2-[4-(4-hydroxyphenylsulfonyl)phenoxy]dodecanamido}phenyl}propy 1, 2-ethoxytridecyl, trifluoromethyl, cyclopentyl, or 3-(2,4-di-t-amylphenoxy)propyl. As the aryl group, aryls having 6 to 36 carbon atoms are preferred, and monocyclic aryls are more preferred. Examples thereof include phenyl, 4-t-butylphenyl, 2-methylphenyl, 2,4,6-trimethylphenyl, 2-methoxyphenyl, 4-methoxyphenyl, 2,6-dichlorophenyl, 2-chlorophenyl and 2,4-dichlorophenyl. As the heterocyclic group, a 5 to 8-membered ring group having 1 to 36 carbon atoms wherein a nitrogen atom, an oxygen atom or a sulfur atom is contained is preferred. A 5-membered or 6-membered ring bonded at a nitrogen atom is more preferred. This ring may form a condensed ring in cooperation with a benzene ring or another heterocycle. As such a heterocyclic group, there can be mentioned, for example, imidazolyl, pyrazolyl, triazolyl, piperidino, pyrrolidyl, pyrrolyl, morpholino, pyrazolidyl, or thiazolidyl. Among these, pyrrolidyl is preferred.

Among these substituents, those capable of further having a substituent may further be substituted with groups as exemplified above with respect to $R^3$ and $R^4$.

As preferred $R^{33}$, there can be mentioned a substituted or unsubstituted alkyl group, a substituted or unsubstituted alkenyl group, or a substituted or unsubstituted cycloalkyl group.

$R^{34}$ represents a hydrogen atom, or a substituent capable of bonding at a carbon atom. As the substituent capable of bonding at a carbon atom, there can be mentioned, for example, a substituted or unsubstituted alkyl group, a substituted or unsubstituted alkenyl group, a substituted or unsubstituted alkynyl group, a substituted or unsubstituted cycloalkyl group, a substituted or unsubstituted cycloalkenyl group, a substituted or unsubstituted aryl group, a substituted or unsubstituted acyl group, a substituted or unsubstituted alkyloxycarbonyl group, a substituted or unsubstituted aryloxycarbonyl group, or a substituted or unsubstituted carbamoyl group.

More specifically, $R^{34}$ can be a hydrogen atom, and the alkyl, alkenyl, alkynyl, cycloalkyl and cycloalkenyl groups represented by $R^{34}$ can be a linear or branched alkyl group having 1 to 32 carbon atoms, an aralkyl group having 7 to 32 carbon atoms, an alkenyl group having 2 to 32 carbon atoms, an alkynyl group having 2 to 32 carbon atoms, a cycloalkyl group having 3 to 32 carbon atoms and a cycloalkenyl group having 3 to 32 carbon atoms. Still more specifically, they can be, for example, methyl, ethyl, propyl, isopropyl, t-butyl, tridecyl, 2-methanesulfonylethyl, 3-(3-pentadecylphenoxy)propyl, 3-{4-{2-[4-(4-hydroxyphenylsulfonyl)phenoxy]dodecanamido}phenyl}propy 1, 2-ethoxytridecyl, trifluoromethyl, cyclopentyl and 3-(2,4-di-t-amylphenoxy)propyl. With respect to the aryl group, one having 6 to 36 carbon atoms is preferred, and monocyclic one is more preferred. The aryl group can be, for example, phenyl, 4-t-butylphenyl, 2-methylphenyl, 2,4,6-trimethylphenyl, 2-methoxyphenyl, 4-methoxyphenyl, 2,6-dichlorophenyl, 2-chlorophenyl, 2,4-dichlorophenyl or the like. The acyl group is preferably one having 2 to 32 carbon atoms, and can be, for example, acetyl, pivaloyl, octanoyl or benzoyl. Examples of the alkyloxycarbonyl, aryloxycarbonyl and carbamoyl groups can be, for example, those described above with respect to groups employed for substitution of $R^1$ and $R^2$.

Among these substituents, those capable of further having a substituent may further have substituents exemplified above with respect to groups employed for substitution of $R^3$ and $R^4$.

As preferred $R^{34}$, there can be mentioned a substituted or unsubstituted alkyl group, a substituted or unsubstituted alkenyl group, a substituted or unsubstituted alkynyl group, a substituted or unsubstituted cycloalkyl group, a substituted or unsubstituted cycloalkenyl group, or a substituted or unsubstituted aryl group.

$R^{33}$ and $R^{34}$ may be bonded with each other to thereby form a 5 or 6-membered ring having a bonding with nitrogen atom. This nitrogenous ring can be, for example, any of imidazolyl, pyrazolyl, triazolyl, piperidyl, piperidino, pyrrolidinyl, pyrrolyl, morpholyl, morpholino, pyrazolidinyl, thiazolidinyl, pyrazolinyl, piperadinyl and the like. These nitrogenous rings may form a condensed ring in cooperation with a benzene ring or another heterocycle.

With respect to $R^{33}$ and $R^{34}$, those which form a ring structure are preferred to those which do not form any ring structure. In particular, groups which form a 6-membered ring having a bonding with nitrogen atom are preferred, and those which form morpholino, a piperadinyl substituted with an acyl group, piperidino or a piperidino substituted with a carboxy group are more preferred.

$R^{21}$, $R^{22}$, $R^{23}$, $R^{24}$ and $R^{25}$ may be identical with or different from each other. Each thereof represents a hydrogen atom or a substituent. As a preferred substituent, there can be mentioned a substituted or unsubstituted alkyl group, a substituted or unsubstituted alkenyl group, a substituted or unsubstituted alkynyl group, a substituted or unsubstituted cycloalkyl group, a substituted or unsubstituted cycloalkenyl group, or a substituted or unsubstituted aryl group. More preferred substituents are as follows.

Each of $R^{21}$ and $R^{22}$ preferably represents an alkyl group, an alkenyl group, an alkynyl group, a cycloalkyl group or a cycloalkenyl group. For example, each of $R^{21}$ and $R^{22}$ represents a linear, branched or cyclic alkyl group having 1 to 36 carbon atoms, an aralkyl group having 7 to 36 carbon atoms, an alkenyl group having 2 to 36 carbon atoms, an alkynyl group having 2 to 36 carbon atoms, or a cycloalkenyl group having 3 to 36 carbon atoms. Specifically, each of $R^{21}$ and $R^{22}$ represents, for example, methyl, ethyl, propyl, isopropyl, t-butyl, t-amyl, t-octyl, tridecyl, cyclopentyl or cyclohexyl. With respect to the number of carbon atoms of these alkyl, alkenyl, alkynyl, cycloalkyl and cycloalkenyl groups, it is preferred that the upper limit thereof be 12. More preferably, each of $R^{21}$ and $R^{22}$ represents an unsubstituted alkyl group having 1 to 8 carbon atoms. Each of $R^{23}$, $R^{24}$ and $R^{25}$ preferably represents a hydrogen atom, an alkyl group, an alkenyl group, an alkynyl group, a cycloalkyl group or a cycloalkenyl group. As the alkyl, alkenyl, alkynyl, cycloalkyl and cycloalkenyl groups, there can be mentioned those set out above with respect to $R^{21}$ and $R^{22}$. Most preferably, each of $R^{23}$, $R^{24}$ and $R^{25}$ represents a hydrogen atom.

Z represents a nonmetallic atomic group required for forming a 5 to 8-membered ring. This ring may have a substituent, and may be a saturated ring or have an unsaturated bond. As a preferred nonmetallic atom, there can be mentioned a nitrogen atom, an oxygen atom, a sulfur atom or a carbon atom. More preferably, the nonmetallic atom is a carbon atom.

As the ring formed by Z, there can be mentioned, for example, a cyclopentane ring, a cyclohexane ring, a cycloheptane ring, a cyclooctane ring, a cyclohexene ring, a piperazine ring, an oxane ring or a thiane ring. These rings may have any of the above-mentioned substituents.

Among the rings formed by Z, an unsubstituted or substituted cyclohexane ring is preferred. A cyclohexane ring having its 4-position substituted with an alkyl group having 1 to 24 carbon atoms (may have any of the above-mentioned substituents) is especially preferred.

It is preferred that the cyan coupler of the general formula (III) for use in the present invention have an oil solubility increasing group in its molecule so as to be easily dissolved in high-boiling organic solvents, and further that this coupler per se and the dye formed by oxidation coupling between this coupler and a color reducing agent (developer) be nondiffusive in hydrophilic colloid layers.

An especially preferred combination of groups for the cyan coupler of the general formula (III) according to the present invention is realized when X is a hydrogen atom; each of $R^{21}$ and $R^{22}$ is t-butyl; each of $R^{23}$, $R^{24}$ and $R^{25}$ is a hydrogen atom; Z is one for forming a cyclohexane ring wherein the 4-position is an alkyl group having 1 to 8 carbon atoms; $R^{33}$ and $R^{34}$ are those for forming a ring structure (in particular, preferably groups forming a 6-membered ring having a bonding with nitrogen atom, for example, morpholino, a piperazinyl substituted with an acyl group, or a piperidino substituted with a carboxy group); $R^{35}$ is a substituted or unsubstituted alkylsulfonylamino group having 1 to 32 carbon atoms, a substituted or unsubstituted arylsulfonylamino group having 6 to 30 carbon atoms, or a nitro group; and each of $R^{36}$, $R^{31}$ and $R^{32}$ is preferably a hydrogen atom or a substituted arylsulfonylamino group having 6 to 30 carbon atoms. The substituent had by the arylsulfonylamino group is preferably an alkoxy group having 1 to 32 carbon atoms, an alkyl group having 1 to 32 carbon atoms, a sulfonylamino group having 1 to 32 carbon atoms, an acylamino group having 1 to 32 carbon atoms, or a halogen atom.

Specific examples of the cyan couplers of the present invention will be set out below, which are however not limitative.

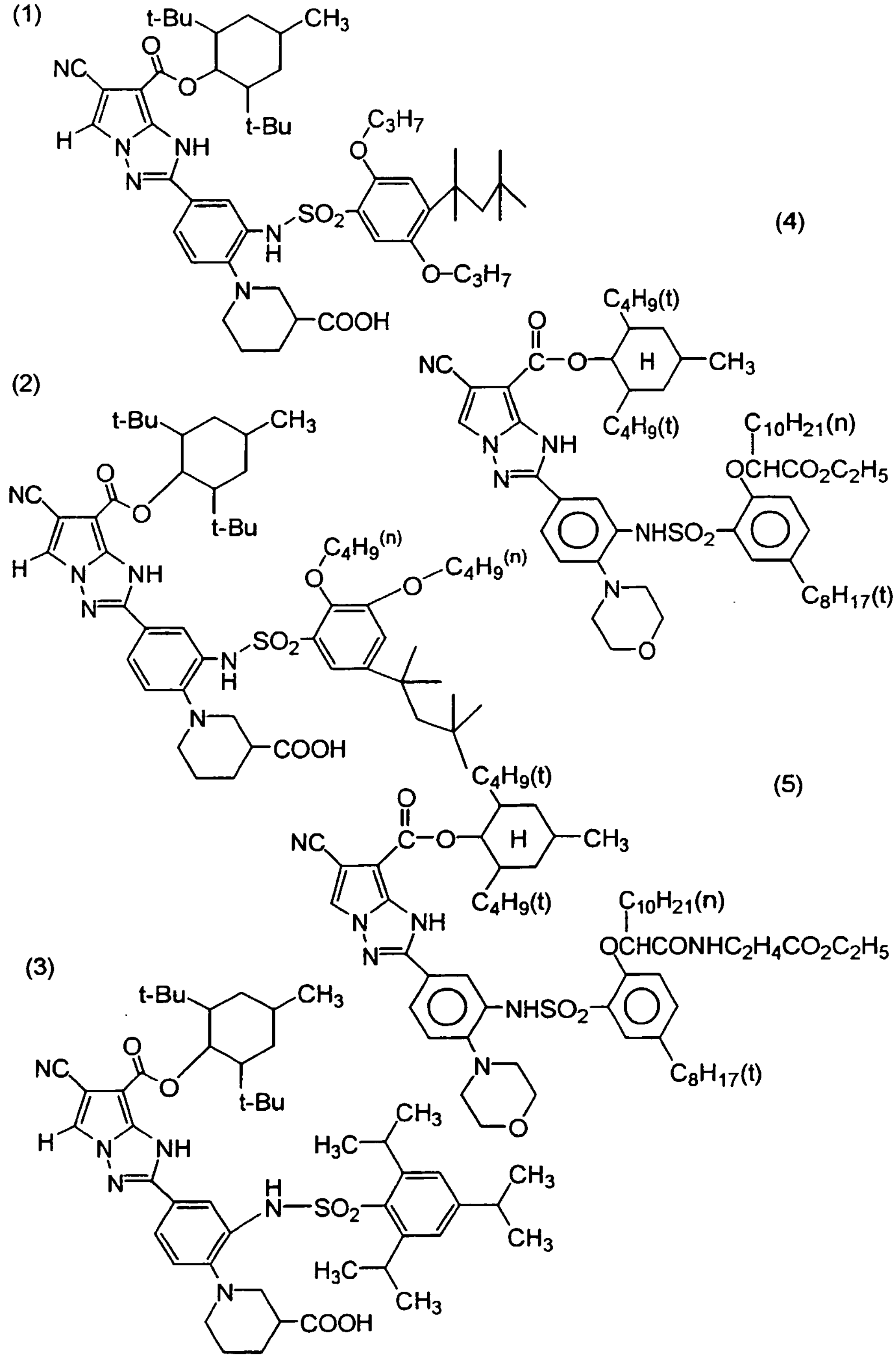

(1)

(4)

(2)

(5)

(3)

(6)

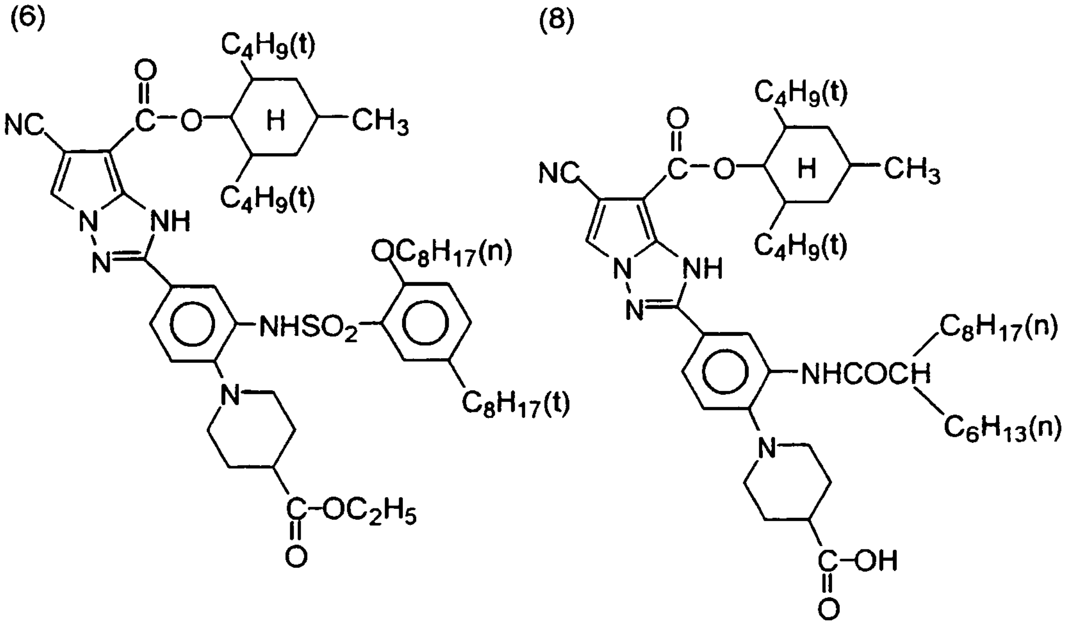

(8)

(7)

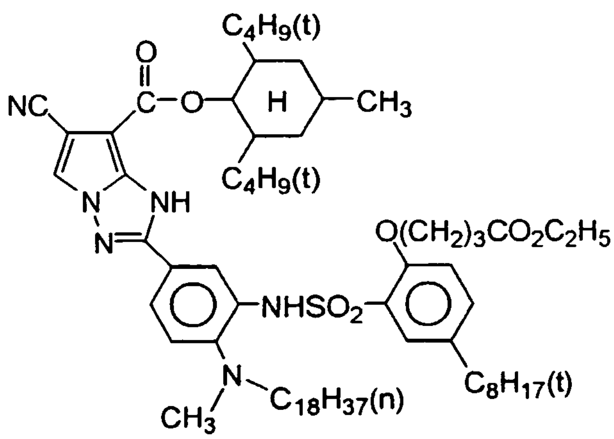

(9)

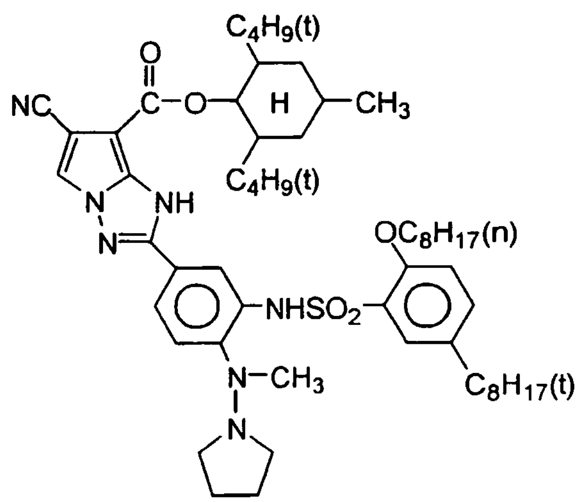

(10)

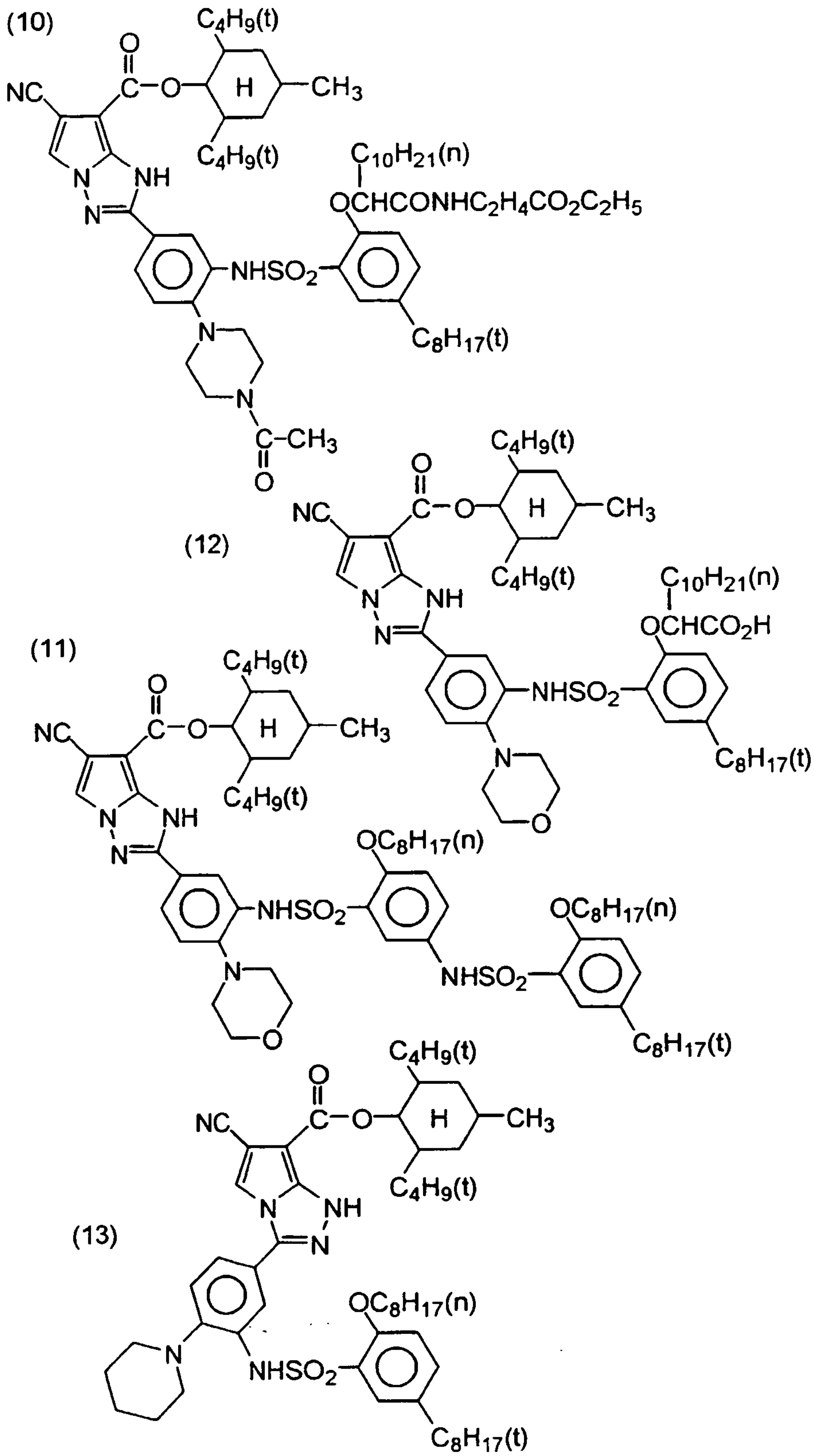

(12)

(11)

(13)

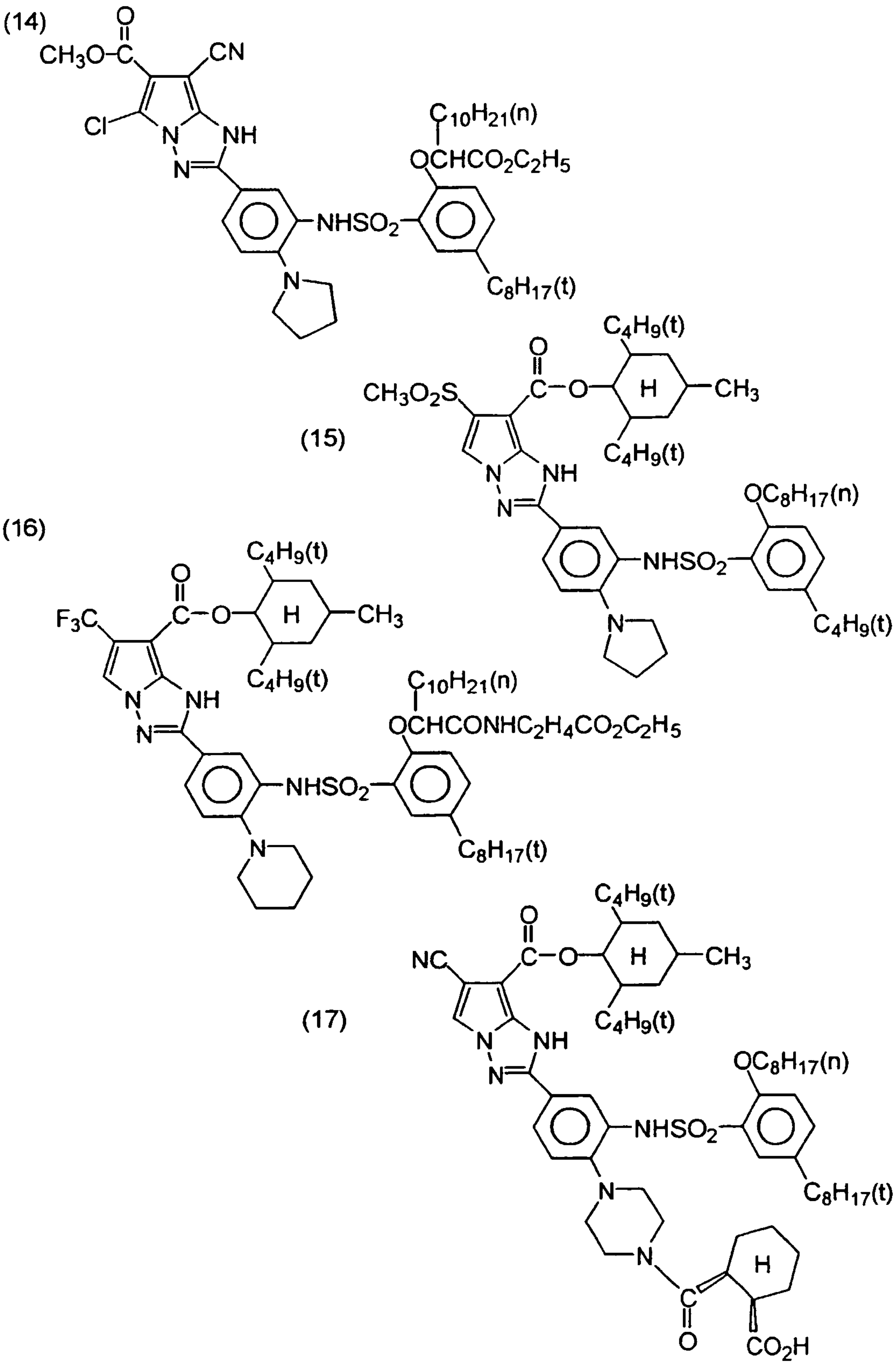

(14)

(15)

(16)

(17)

(18)

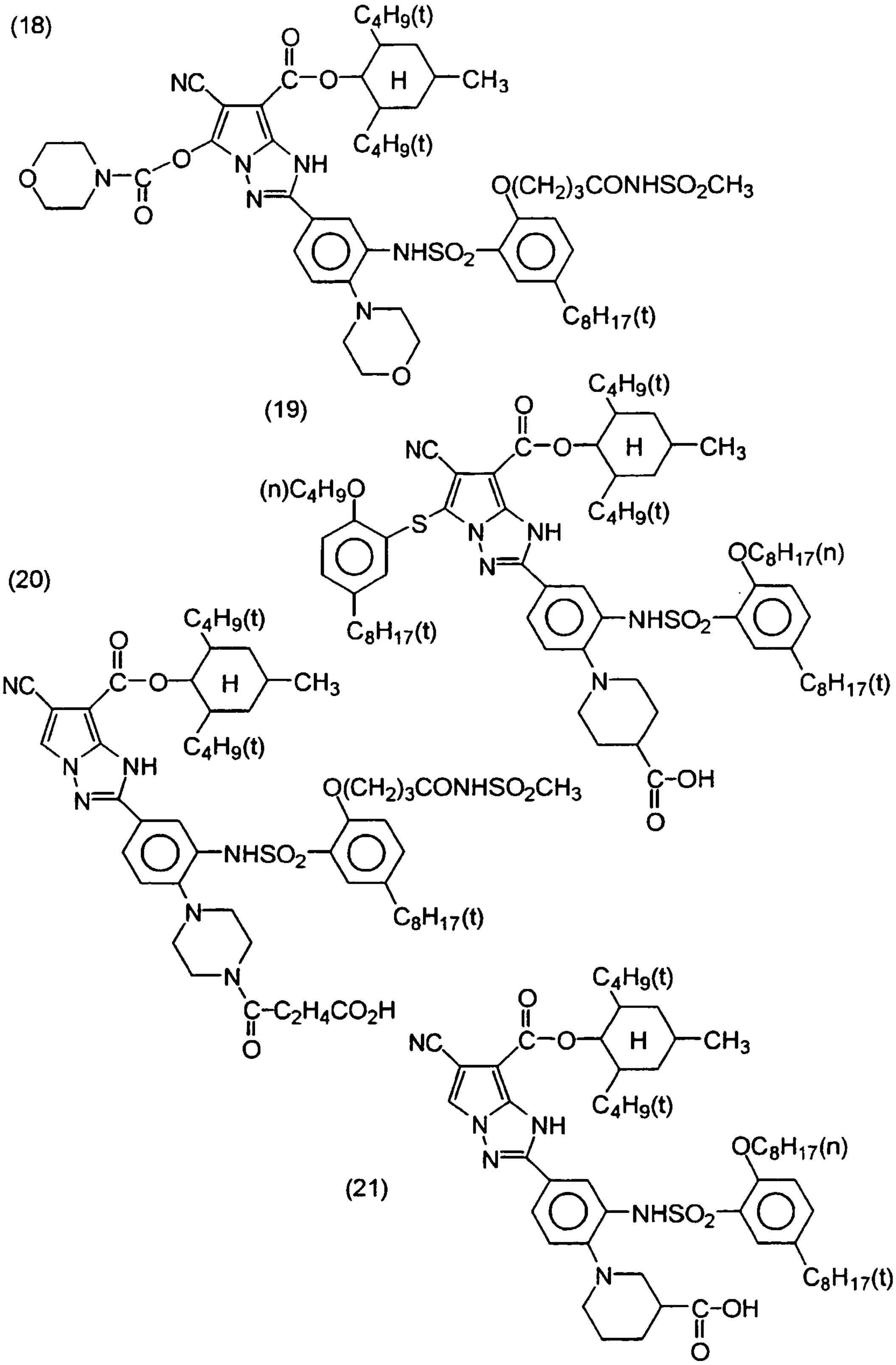

(19)

(20)

(21)

A synthetic example

A specific example of a synthetic method of the amide compound (compound 1) of the present invention will be set out below.

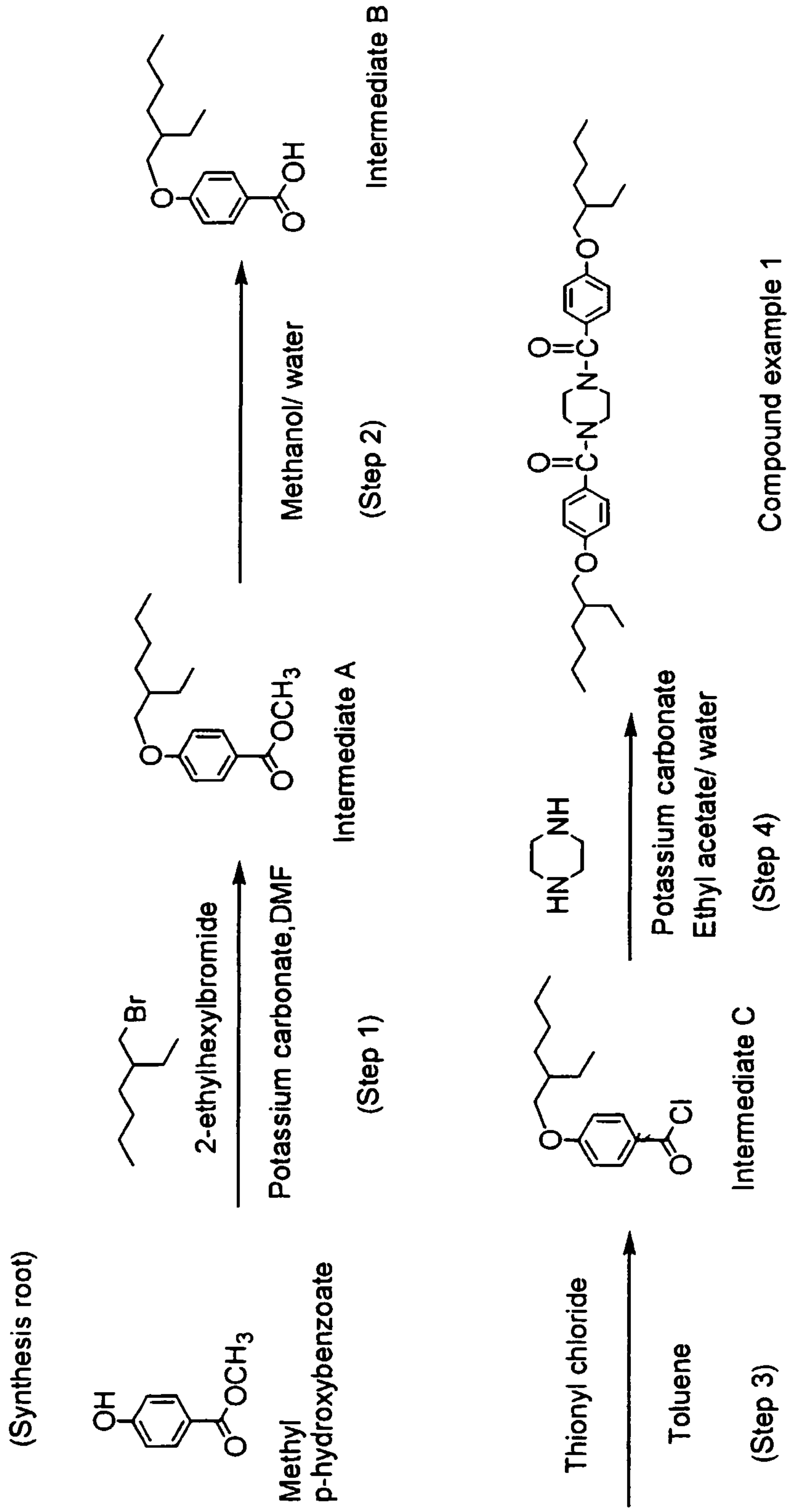


(Step 1)

456 g (2.36 mol) of 1-bromo-2-ethylhexane was dropped over a period of 1 hr into a mixture of 409 g (2.96 mol) of potassium carbonate, 300 g (1.97 mol) of methyl p-hydroxybenzoate and 900 mL of dimethylformamide under

agitation at 70˚C. After the dropping, reaction was conducted at 70˚C for 4 hr. The reaction mixture was cooled to 40˚C, and 500 mL of ethyl acetate was added to the cooled reaction mixture. Inorganic salts were filtered off, and the obtained reaction solution was washed with first 300 mL of water. subsequently 300 mL of a 1 N aqueous hydrochloric acid solution, and finally 200 mL of saturated saline solution. Solvent was distilled off in vacuum, thereby obtaining 530 g of intermediate A. The intermediate A left intact was used in the subsequent step.

(Step 2)

The whole amount of intermediate A synthesized in the step 1 was added to a mixture of 226 g (3.43 mol) of potassium hydroxide, 1.0 L of methanol and 100 mL of water under agitation at 60˚C. After the addition, reaction was conducted at 65˚C for 2 hr. The reaction mixture was cooled to 40˚C, and 450 mL of concentrated hydrochloric acid was added to the cooled reaction mixture. Further, 500 mL of acetonitrile was added, and agitated at 20˚C. Thus, crystal was precipitated. The crystal was collected by filtration, washed well with 500 mL of acetonitrile, and dried. Thus, 526 g of intermediate B was obtained (inorganic salts were mingled into the crystal, so that calculated yield exceeded 100%).

(Step 3)

286 g (2.4 mol) of thionyl chloride was dropped over a period of 30 min into a mixture of the whole amount of intermediate B synthesized in the preceding step, 900 mL of toluene and 2 mL of dimethylformamide under agitation at 60˚C. Further, reaction was conducted at 60˚C for 2 hr. The reaction mixture was concentrated in vacuum, and 250 mL of solvent was distilled off. Thereafter, 100 mL of ethyl acetate was added, and inorganic salts were filtered off. Thus, a toluene / ethyl acetate solution of intermediate C was obtained.

(Step 4)

The whole amount of toluene/ethyl acetate solution of intermediate C synthesized in the preceding step was dropped over a period of 30 min into a mixture of 108 g (1.25 mol) of piperazine anhydride, 414.6 g (3.0 mol) of potassium carbonate, 650 mL of water and 500 mL of ethyl acetate under agitation at 25˚C. After the dropping, reaction was conducted at 25˚C for 30 min, and the temperature was raised to 50˚C. The obtained reaction mixture was washed with 400 mL of 40˚C water and subsequently 400 mL of saturated saline solution. An organic layer was distilled off in vacuum, and 700 mL of ethyl acetate and 1.7 L of acetonitrile were added so as to effect recrystallization. Crystal was collected by filtration, washed well with acetonitrile, and dried. Thus, 300 g of compound 1 was obtained (total yield through four steps: 55.6%).

The structure thereof was identified by mass spectrometry and 300 MHz $^1$H-NMR.

NMR measurement data of the compound 1 will be set out below.
300 MHz $^1$H-NMR
Solvent used in measuring: deuterated chloroform
Internal standard: tetramethylsilane
$^1$H-NMR σ ($CDCl_3$): 0.85 - 0.98 (m, 6H), 1.22 - 1.61 (m, 8H), 1.66 - 1.80 (m, 1H), 3.50 - 3.80 (bs, 4H), 3.88 (d, J = 8.6 Hz, 2H), 6.92 (d, J = 8.6 Hz, 2H), 7.39 (d, J = 8.6, 2H).

The cyan coupler compounds of the general formula (II) can be synthesized by methods described in JP-A-2001-163887.

The coating amount of cyan coupler according to the present invention is preferably in the range of 0.01 to 2 g/m$^2$, more preferably 0.05 to 1.0 g/m$^2$.

In a light-sensitive material of the present invention, at least one sensitive layer need only be formed on a support. A typical example is a silver halide photographic light-sensitive material having, on a support, at least one sensitive layer consisting of a plurality of silver halide emulsion layers sensitive to substantially the same color but different in sensitivity. This sensitive layer is a unit sensitive layer sensitive to one of blue light, green light, and red light. In a multilayered silver halide color photographic light-sensitive material, sensitive layers are generally arranged in the order of red-, green-, and blue-sensitive layers from a support. However, according to the intended use, this order of arrangement can be reversed, or sensitive layers sensitive to the same color can sandwich another sensitive layer sensitive to a different color. Non-light-sensitive layers can be formed between the silver halide sensitive layers and as the uppermost layer and the lowermost layer. These non-light-sensitive layers can contain, e.g., couplers, DIR compounds, and color amalgamation inhibitors to be described later. As a plurality of silver halide emulsion layers constituting each unit sensitive layer, as described in DE1,121,470 or GB923,045, high- and low-speed emulsion layers are preferably arranged such that the sensitivity is sequentially decreased toward a support. Also, as described in JP-A's-57-112751, 62-200350, 62-206541, and 62-206543, layers can be arranged such that a low-speed emulsion layer is formed apart from a support and a high-speed layer is formed close to the support.

More specifically, layers can be arranged, from the one farthest from a support, in the order of a low-speed blue-sensitive layer (BL)/high-speed blue-sensitive layer (BH)/high-speed green-sensitive layer (GH)/low-speed green-sensitive layer (GL)/high-speed red-sensitive layer (RH)/low-speed red-sensitive layer (RL), the order of BH/BL/GL/GH/RH/RL, or the order of BH/BL/GH/GL/RL/RH.

In addition, as described in Jpn. Pat. Appln. KOKOKU Publication No. (hereinafter referred to as JP-B-) 55-34932, layers can be arranged in the order of a blue-sensitive layer/GH/RH/GL/RL from the one farthest from a support. Furthermore, as described in JP-A's-56-25738 and 62-63936, layers can be arranged in the order of a blue-sensitive layer/GL/RL/GH/RH from the one farthest from a support.

As described in JP-B-49-15495, three layers can be arranged such that a silver halide emulsion layer having the highest sensitivity is arranged as an upper layer, a silver halide emulsion layer having sensitivity lower than that of the upper layer is arranged as an interlayer, and a silver halide emulsion layer having sensitivity lower than that of the interlayer is arranged as a lower layer, i.e., three layers having different sensitivities can be arranged such that the sensitivity is sequentially decreased toward a support. When the layer structure is thus constituted by three layers having different sensitivities, these three layers can be arranged, in the same color-sensitive layer, in the order of a medium-speed emulsion layer/high-speed emulsion layer/low-speed emulsion layer from the one farthest from a support as described in JP-A-59-202464.

In addition, the order of a high-speed emulsion layer/low-speed emulsion layer/medium-speed emulsion layer or low-speed emulsion layer/medium-speed emulsion layer/high-speed emulsion layer can be used. Furthermore, the arrangement can be changed as described above even when four or more layers are formed.

To improve the color reproducibility, as described in US4,663,271, US4,705,744, US4,707,436, and JP-A's-62-160448 and 63-89850, a donor layer (CL) with an interlayer effect, which has a different spectral sensitivity distribution from that of a main sensitive layer such as BL, GL, or RL, is preferably formed adjacent to, or close to, this main sensitive layer.

A silver halide used in the present invention is silver iodobromide, silver iodochloride, or silver bromochloroiodide containing about 30 mol% or less of silver iodide. A silver halide is most preferably silver iodobromide or silver bromochloroiodide containing about 2 to about 10 mol% of silver iodide.

Silver halide grains contained in a photographic emulsion can have regular crystals such as cubic, octahedral, or tetradecahedral crystals, irregular crystals such as spherical or tabular crystals, crystals having crystal defects such as twin planes, or composite shapes thereof.

A silver halide can consist of fine grains having a grain size of about 0.2 $\mu$m or less or large grains having a projected area diameter of about 10 $\mu$m, and an emulsion can be either a polydisperse or monodisperse emulsion.

A silver halide photographic emulsion usable in the present invention can be prepared by methods described in, e.g., "I. Emulsion preparation and types," Research Disclosure (RD) No. 17643 (December, 1978), pp. 22 and 23, RD No. 18716 (November, 1979), p. 648, and RD No. 307105 (November, 1989), pp. 863 to 865; P. Glafkides, "Chemie et Phisique Photographique", Paul Montel, 1967; G.F. Duffin, "Photographic Emulsion Chemistry", Focal Press, 1966; and V.L. Zelikman et al., "Making and Coating Photographic Emulsion", Focal Press, 1964.

Monodisperse emulsions described in, e.g., US3,574,628, US3,655,394, and GB1,413,748 are also favorable.

Tabular grains having an aspect ratio of about 3 or more can also be used in the present invention. Tabular grains can be easily prepared by methods described in Gutoff, "Photographic Science and Engineering", Vol. 14, pp. 248 to 257 (1970); and US4,434,226, US4,414,310, US4,433,048, US4,439,520, and GB2,112,157.

A crystal structure can be uniform, can have different halogen compositions in the interior and the surface layer thereof, or can be a layered structure. Alternatively, a silver halide having a different composition can be bonded by an epitaxial junction, or a compound except for a silver halide such as silver rhodanide or lead oxide can be bonded. A mixture of grains having various types of crystal shapes can also be used.

The above emulsion can be any of a surface latent image type emulsion which mainly forms a latent image on the surface of a grain, an internal latent image type emulsion which forms a latent image in the interior of a grain, and another type of emulsion which has latent images on the surface and in the interior of a grain. However, the emulsion must be a negative type emulsion. The internal latent image type emulsion can be a core/shell internal latent image type emulsion described in JP-A-63-264740. A method of preparing this core/shell internal latent image type emulsion is described in JP-A-59-133542. Although the thickness of a shell of this emulsion depends on the development conditions and the like, it is preferably 3 to 40 nm, and most preferably, 5 to 20 nm.

A silver halide emulsion layer is normally subjected to physical ripening, chemical ripening, and spectral sensitization steps before it is used. Additives for use in these steps are described in RD Nos. 17643, 18716, and 307105 and they are summarized in a table to be presented later.

In a light-sensitive material of the present invention, it is possible to mix, in the same layer, two or more types of emulsions different in at least one of the characteristics, i.e., the grain size, grain size distribution, halogen composition, grain shape, and sensitivity, of a photosensitive silver halide emulsion.

It is also possible to preferably use surface-fogged silver halide grains described in US4,082,553, internally

fogged silver halide grains described in US4,626,498 and JP-A-59-214852 and colloidal silver, in photosensitive silver halide emulsion layers and/or substantially non-light-sensitive hydrophilic colloid layers. The internally fogged or surface-fogged silver halide grain means a silver halide grain which can be developed uniformly (non-imagewise) regardless of whether the location is a non-exposed portion or an exposed portion of the light-sensitive material. A method of preparing the internally fogged or surface-fogged silver halide grain is described in US4,626,498 and JP-A-59-214852. A silver halide which forms the core of an internally fogged core/shell type silver halide grain can have a different halogen composition. As the internally fogged or surface-fogged silver halide, any of silver chloride, silver chlorobromide, silver bromoiodide, and silver bromochloroiodide can be used. The average grain size of these fogged silver halide grains is preferably 0.01 to 0.75 μm, and most preferably, 0.05 to 0.6 μm. The grain shape can be a regular grain shape. Although the emulsion can be a polydisperse emulsion, it is preferably a monodisperse emulsion (in which at least 95% in weight or number of grains of silver halide grains have grain sizes falling within the range of ±40% of the average grain size).

In the present invention, it is preferable to use a non-light-sensitive, fine-grain silver halide. The non-light-sensitive, fine-grain silver halide preferably consists of silver halide grains which are not exposed during imagewise exposure for obtaining a dye image and are not substantially developed during development. These silver halide grains are preferably not fogged in advance. In the fine-grain silver halide, the content of silver bromide is 0 to 100 mol%, and silver chloride and/or silver iodide can be added if necessary. The fine-grain silver halide preferably contains 0.5 to 10 mol% of silver iodide. The average grain size (the average value of the equivalent-circle diameters of projected areas) of the fine-grain silver halide is preferably 0.01 to 0.5 μm, and more preferably, 0.02 to 2 μm.

The fine-grain silver halide can be prepared following the same procedures as for a common sensitive silver halide. The surface of each silver halide grain need not be optically sensitized nor spectrally sensitized. However, before the silver halide grains are added to a coating solution, it is preferable to add a well-known stabilizer such as a triazole-based compound, azaindene-based compound, benzothiazolium-based compound, mercapto-based compound, or zinc compound. Colloidal silver can be added to this layer containing fine silver halide grains.

The silver coating amount of a light-sensitive material of the present invention is preferably 6.0 $g/m^2$ or less, and most preferably, 4.5 $g/m^2$ or less.

Photographic additives usable in the present invention are also described in RDs, and the relevant portions are summarized in the following table.

| | Additives | RD17643 | RD18716 |
|---|---|---|---|
| 1. | Chemical sensitizers | page 23 | page 648, right column |
| 2. | Sensitivity increasing agents | | do |
| 3. | Spectral sensitizers, super sensitizers | pages 23 - 24 | page 648, right column to page 649, right column |
| 4. | Brighteners | page 24 | page 647, right column |
| 5. | Light absorbents, filter dyes, ultraviolet absorbents | pages 25 - 26 | page 649, right column to page 650, left column |
| 6. | Binders | page 26 | page 651, left column |
| 7. | Plasticizers, lubricants | page 27 | page 650, right column |
| 8. | Coating aids, surface active agents | pages 26 - 27 | do |
| 9. | Antistatic agents | page 27 | do |
| 10. | Matting agents Additives | RD307105 | |
| 1. | Chemical sensitizers | page 866 | |
| 2. | Sensitivity increasing agents | | |
| 3. | Spectral sensitizers, super sensitizers | pages 866 - 868 | |
| 4. | Brighteners | page 868 | |
| 5. | Light absorbent, filter dye, ultra-violet absorbents | page 873 | |
| 6. | Binder | pages 873 - 874 | |
| 7. | Plasticizers, lubricants | page 876 | |
| 8. | Coating aids, surface active agents | pages 875 - 876 | |
| 9. | Antistatic agents | pages 876 - 877 | |
| 10. | Matting agent | pages 878 - 879 | |

Various dye forming couplers can be used in the lightsensitive material of the present invention, and the following couplers are particularly preferred.

Yellow couplers: couplers represented by formulae (I) and (II) described in EP 502,424A; couplers represented by formulae (1) and (2) described in EP 513,496A (particularly Y-28 on page 18); couplers represented by formula (I) of claim 1 described in EP 568,037A; couplers represented by general formula (I) described in column 1, lines 45 to 55, of U.S.P. 5,066,576; couplers represented by general formula (I) described in paragraph 0008 of JP-A-4-274425; couplers described in claim 1 on page 40 of EP 498,381A1 (particularly D-35 on page 18); couplers represented by formula (Y) described on page 4 of EP 447,969A1 (particularly Y-1 (page 17) and Y-54 (page 41)); and couplers represented by formulae (II) to (IV) described in column 7, lines 36 to 58, of U.S.P. 4,476,219 (particularly II-17, 11-19 (column 17) and II-24 (column 19)).

Magenta couplers: JP-A-3-39737 (L-57 (page 11, lower right column), L-68 (page 12, lower right column) and L-77 (page 13, lower right column); A-4-63 (page 134), and A-4-73 and -75 (page 139) described in EP 456,257; M-4 and -6 (page 26), and M-7 (page 27) described in EP 486,965; M-45 (page 19) described in EP 571,959A; (M-1) (page 6) described in JP-A-5-204106; and M-22 described in paragraph 0237 of JP-A-4-362631.

Cyan couplers: CX-1, -3, -4, -5, -11, -12, -14 and -15 (pages 14 to 16) described in JP-A-4-204843; C-7 and -10 (page 35), C-34 and -35 (page 37), and (I-1) and (I-17) (pages 42 and 43) described in JP-A-4-43345; and couplers represented by general formulae (Ia) and (Ib) described in claim 1 of JP-A-6-67385.

Polymer couplers: P-1 and P-5 (page 11) described in JP-A-2-44345.

Couplers for forming a color forming dye of appropriate diffusivity are preferably those described in U.S.P. 4,366,237, GB 2,125,570, EP 96,873B and DE 3,234,533.

As couplers for correcting the unnecessary absorption of a color forming dye, preferred use is made of yellow colored cyan couplers represented by formulae (CI), (CII), (CIII) and (CIV) described on page 5 of EP 456,257A1 (particularly YC-86 on page 84); yellow colored magenta couplers ExM-7 (page 202), Ex-1 (page 249) and EX-7 (page 251) described in EP 456,257A1; magenta colored cyan couplers CC-9 (column 8) and CC-13 (column 10) described in U.S.P. 4,833,069; coupler (2) (column 8) described in U.S.P. 4,837,136; and colorless masking couplers represented by formula (A) described in claim 1 of WO 92/11575 (particularly compound examples on pages 36 to 45).

Examples of couplers which release a photographically useful group are as follows. Development inhibitor release compounds: compounds represented by formulae (I), (II), (III) and (IV) described on page 11 of EP 378,236A1 (particularly T-101 (page 30), T-104 (page 31), T-113 (page 36), T-131 (page 45), T-144 (page 51) and T-158 (page 58)); compounds represented by formula (I) described on page 7 of EP 436,938A2 (particularly D-49 (page 51)); compounds represented by formula (1) in EP 568,037A (particularly compound (23) (page 11)); and compounds represented by formulae (I), (II) and (III) described on pages 5 and 6 of EP 440,195A2 (particularly compound I-(1) on page 29). Bleaching accelerator release compounds: compounds represented by formulae (I) and (I') described on page 5 of EP 310,125A2 (particularly compounds (60) and (61) on page 61); and compounds represented by formula (I) in claim 1 of JP-A-6-59411 (particularly compound (7) (page 7)). Ligand release compounds: compounds represented by LIG-X described in claim 1 of U.S.P. 4,555,478 (particularly compounds in column 12, lines 21 to 41). Leuco dye release compounds: compounds 1 to 6 described in columns 3 to 8 of U.S.P. 4,749,641. Fluorescent dye release compounds: compounds represented by COUP-DYE described in claim 1 of U.S.P. 4,774,181 (particularly compounds 1 to 11 in columns 7 to 10). Development accelerator or fogging agent release compounds: compounds represented by formulae (1), (2) and (3) described in column 3 of U.S.P. 4,656,123 (particularly compound (I-22) in column 25); and ExZK-2 described on page 75, lines 36 to 38, in EP 450,637A2. Compounds which release a group becoming a dye only when split off: compounds represented by formula (I) described in claim 1 of U.S.P. 4,857,447 (particularly compounds Y-1 to Y-19 in columns 25 to 36).

Preferable examples of additives other than the couplers are as follows.

Dispersion mediums of an oil-soluble organic compound: P-3, -5, -16, -19, -25, -30, -42, -49, -54, -55, -66, -81, -85, -86 and -93 (pages 140 to 144) described in JP-A-62-215272. Impregnating latexes of an oil-soluble organic compound: latexes described in U.S.P. 4,199,363. Developing agent oxidation product scavengers: compounds represented by formula (I) described in column 2, lines 54 to 62, of U.S.P. 4,978,606 (particularly I-(1), -(2), -(6) and -(12) (columns 4 and 5)), and formulae described in column 2, lines 5 to 10, of U.S.P. 4,923,787 (particularly compound 1 (column 3)). Stain inhibitors: formulae (I) to (III) described on page 4, lines 30 to 33, particularly I-47, I-72, III-1 and III-27 (pages 24 to 48) in EP 298321A. Antifading agents: A-6, -7, -20, -21, -23, -24, -25, -26, -30, -37, -40, -42, -48, -63, -90, -92, -94 and -164 (pages 69 to 118) in EP 298321A; II-1 to III-23, particularly III-10, described in columns 25 to 38 of U.S.P. 5,122,444; I-1 to III-4, particularly II-2, described on pages 8 to 12 in EP 471347A; and A-1 to A-48, particularly A-39 and A-42, described in columns 32 to 40 of U.S.P. 5,139,931. Materials which reduce the use amount of a color enhancer or a color mixing prevention agent: I-1 to II-15, particularly I-46, described on pages 5 to 24 of EP 411324A. Formalin scavengers: SCV-1 to SCV-28, particularly SCV-8, described on pages 24 to 29 of EP 477932A. Film hardeners: H-1, -4, -6, -8 and -14 described on page 17 in JP-A-1-214845; compounds (H-1 to -54) represented by formulae (VII) to (XII) described in columns 13 to 23 of U.S.P. 4,618,573;, compounds (H-1 to -76), particularly H-14, represented by formula (6) described on page 8, lower right column, in JP-A-2-214852; and compounds described in claim 1 of U.S.P. 3,325,287. Development inhibitor precursors: P-24, -37 and -39 (pages 6 and 7) described in JP-A-62-168139; and

compounds described in claim 1, particularly compounds 28 and 29 in column 7, of U.S.P. 5,019,492. Antiseptic agents and mildewproofing agents; I-1 to III-43, particularly II-1, -9, -10, -18 and III-25, described in columns 3 to 15 of U.S.P. 4,923,790. Stabilizers and antifoggants: I-1 to (14), particularly I-1, I-60, (2) and (13), described in columns 6 to 16 of U.S.P. 4,923,793; and compounds 1 to 65, particularly compound 36, described in columns 25 to 32 of U.S.P. 4,952,483. Chemical sensitizers: triphenylphosphine selenide and compound 50 described in JP-A-5-40324. Dyes: a-1 to b-20, particularly a-1, -12, -18, -27, -35, -36 and b-5, described on pages 15 to 18, and V-1 to -23, particularly V-1, described on pages 27 to 29 in JP-A-3-156450; F-I-1 to F-II-43, particularly F-I-11 and F-II-8, described on pages 33 to 55 of EP 445627A; III-1 to -36, particularly III-1 and -3, described on pages 17 to 28 in EP 457153A; microcrystalline dispersions of Dye-1 to -124 described on pages 8 to 2 of WO 88/04794; compounds 1 to 22, particularly compound 1, described on pages 6 to 11 in EP 319999A; compounds D-1 to -87 (pages 3 to 28) represented by formulae (1) to (3) described in EP 519306A; compounds 1 to 22 (columns 3 to 10) represented by formula (I) described in U.S.P. 4,268,622; and compounds (1) to (31) (columns 2 to 9) represented by formula (I) described in U.S.P. 4,923,788. UV absorbers: compounds (18b) to (18r) and 101 to 427 (pages 6 to 9) represented by formula (1) described in JP-A-46-3335; compounds (3) to (66) (pages 10 to 44) represented by formula (I) and compounds HBT-1 to -10 (page 14) represented by formula (III) described in EP 520938A; and compounds (1) to (31) (columns 2 to 9) represented by formula (1) described in EP 521823A.

The present invention can be applied to various color lightsensitive materials such as color negative films for general purposes or cinemas, color reversal photographic films for slides and TV, color paper, color positive films and color reversal paper. Moreover, the present invention is suitable to lens equipped film units described in Jpn. Pat. Appln. KOKOKU Publication No. 2-32615 and Jpn. Utility Model Appln. KOKOKU Publication No. 3-39784.

Supports which can be suitably used in the present invention are described in, e.g., RD No. 17643, page 28; RD No. 18716, from the right column of page 647 to the left column of page 648; and RD No. 307105, page 879.

In the lightsensitive material of the present invention, the sum of the thicknesses of all hydrophilic colloid layers on its side having emulsion layers is preferably 28 μm or less, more preferably 23 μm or less, still more preferably 18 μm or less, and most preferably 16 μm or less. Film swelling speed $T_{1/2}$ is preferably 30 sec or less, more preferably 20 sec or less. The film swelling speed $T_{1/2}$ is defined as the time that when the saturation film thickness refers to 90% of the maximum swollen film thickness attained by the processing in a color developer at 30°C for 3 min 15 sec, spent for the film thickness to reach 1/2 of the saturation film thickness. The film thickness means one measured under moisture conditioning at 25°C in a relative humidity of 55% (two days). The film swelling speed $T_{1/2}$ can be measured by using a swellometer described in A. Green et al., Photogr. Sci. Eng., Vol. 19, No. 2, pp. 124 to 129. The film swelling speed $T_{1/2}$ can be regulated by adding a film hardener to gelatin as a binder, or by changing aging conditions after coating. The swelling ratio preferably ranges from 150 to 400%. The swelling ratio can be calculated from the maximum swollen film thickness measured under the above conditions in accordance with the formula:

(maximum swollen film thickness - film thickness) / film thickness.

In the lightsensitive material of the present invention, hydrophilic colloid layers (called "back layers") having a total dried film thickness of 2 to 20 μm are preferably formed on the side opposite to the side having emulsion layers. The back layers preferably contain the above light absorber, filter dye, ultraviolet absorber, antistatic agent, film hardener, binder, plasticizer, lubricant, coating aid and surfactant. The swelling ratio of the back layers is preferably in the range of 150 to 500%.

The lightsensitive material according to the present invention can be developed by conventional methods described in RD No. 17643, pages 28 and 29; RD No. 18716, page 651, left to right columns; and RD No. 307105, pages 880 and 881.

Color negative film processing solutions used in the present invention will be described below.

Compounds described in JP-A-4-121739, page 9, upper right column, line 1 to page 11, lower left column, line 4 can be used in a color developer of the present invention. As a color developing agent used when particularly rapid processing is to be performed, 2-methyl-4-[N-ethyl-N-(2-hydroxyethyl)amino]aniline, 2-methyl-4-[N-ethyl-N-(3-hydroxypropyl)amino]aniline, or 2-methyl-4-[N-ethyl-N-(4-hydroxybutyl)amino]aniline is preferred.

The use amount of any of these color developing agents is preferably 0.01 to 0.08 mol, more preferably, 0.015 to 0.06 mol, and most preferably, 0.02 to 0.05 mol per liter (to be referred to as "L" hereinafter) of a color developer. Also, a replenisher of a color developer preferably contains a color developing agent at a concentration 1.1 to 3 times, particularly 1.3 to 2.5 times the above concentration.

As a preservative of a color developer, hydroxylamine can be extensively used. If higher preservability is necessary, the use of a hydroxylamine derivative having a substituent such as an alkyl group, hydroxylalkyl group,

sulfoalkyl group, or carboxyalkyl group is preferable. Examples are N,N-di(sulfoethyl)hydroxylamine, monomethylhydroxylamine, dimethylhydroxylamine, monoethylhydroxylamine, diethylhydroxylamine, and N,N-di(carboxylethyl)hydroxylamine. Of these derivatives, N,N-di(sulfoethyl)hydroxylamine is particularly preferred. Although these derivatives can be used together with hydroxylamine, it is preferable to use one or two types of these derivatives instead of hydroxylamine.

The use amount of a preservative is preferably 0.02 to 0.2 mol, more preferably, 0.03 to 0.15 mol, and most preferably, 0.04 to 0.1 mol per L. As in the case of a color developing agent, a replenisher preferably contains a preservative at a concentration 1.1 to 3 times that of a mother solution (processing tank solution).

A color developer contains sulfite as an agent for preventing an oxide of a color developing agent from changing into tar. The use amount of this sulfite is preferably 0.01 to 0.05 mol, and more preferably, 0.02 to 0.04 mol per L. Sulfite is preferably used at a concentration 1.1 to 3 times the above concentration in a replenisher.

The pH of a color developer is preferably 9.8 to 11.0, and more preferably, 10.0 to 10.5. In a replenisher, the pH is preferably set to be higher by 0.1 to 1.0 than these values. To stably maintain this pH, a known buffering agent such as carbonate, phosphate, sulfosalicylate, or borate is used.

The replenishment rate of a color developer is preferably 80 to 1,300 mL per $m^2$ of a light-sensitive material. However, the replenishment rate is preferably smaller in order to reduce environmental pollution. For example, the replenishment rate is preferably 80 to 600 mL, and more preferably, 80 to 400 mL.

The bromide ion concentration in the color developer is usually 0.01 to 0.06 mol per L. However, this bromide ion concentration is preferably set at 0.015 to 0.03 mol per L in order to suppress fog and improve discrimination and graininess while maintaining sensitivity. To set the bromide ion concentration in this range, it is only necessary to add bromide ions calculated by the following equation to a replenisher. If C takes a negative value, however, no bromide ions are preferably added to a replenisher.

$$C = A - W/V$$

where

C : the bromide ion concentration (mol/L) in a color developer replenisher

A : the target bromide ion concentration (mol/L) in a color developer

W : the amount (mol) of bromide ions dissolving into the color developer from 1 $m^2$ of a light-sensitive material when the sensitive material is color-developed

V : the replenishment rate (L) of the color developer replenisher for 1 $m^2$ of the light-sensitive material

As a method of increasing the sensitivity when the replenishment rate is decreased or high bromide ion concentration is set, it is preferable to use a development accelerator such as pyrazolidones represented by 1-phenyl-3-pyrazolidone and 1-phenyl-2-methyl-2-hydroxylmethyl-3-pyrazolidone, or a thioether compound represented by 3,6-dithia-1,8-octandiol.

Compounds and processing conditions described in JP-A-4-125558, page 4, lower left column, line 16 to page 7, lower left column, line 6 can be applied to a processing solution having bleaching capacity in the present invention. This bleaching agent preferably has an oxidation-reduction potential of 150 mV. Favored practical examples of the bleaching agent are described in JP-A's-5-72694 and 5-173312. In particular, 1,3-diaminopropane tetraacetic acid and ferric complex salt of a compound as practical example 1 in JP-A-5-173312, page 7 are preferred.

To improve the biodegradability of a bleaching agent, it is preferable to use compound ferric complex salts described in JP-A-4-251845, JP-A-4-268552, EP588,289, EP591,934, and JP-A-6-208213 as the bleaching agent. The concentration of any of these bleaching agents is preferably 0.05 to 0.3 mol per L of a solution having bleaching capacity. To reduce the amount of waste to the environment, the concentration is preferably designed to be 0.1 to 0.15 mol per L of the solution having bleaching capacity. When the solution having bleaching capacity is a bleaching solution, preferably 0.2 to 1 mol, and more preferably, 0.3 to 0.8 mol of a bromide is added per L.

A replenisher of the solution having bleaching capacity basically contains components at concentrations calculated by the following equation. This makes it possible to maintain the concentrations in a mother solution constant.

$$C_R = C_T \times (V_1 + V_2)/V_1 + C_P$$

where

$C_R$ : the concentrations of components in a replenisher
$C_T$ : the concentrations of components in a mother solution (processing tank solution)
$C_P$ :the concentrations of components consumed during processing
$V_1$ : the replenishment rate (mL) of a replenisher having bleaching capacity per $m^2$ of a light-sensitive material
$V_2$ : an amount (mL) carried over from a pre-bath by $m^2$ of the light-sensitive material

Additionally, a bleaching solution preferably contains a pH buffering agent, and more preferably contains succinic acid, maleic acid, malonic acid, glutaric acid, adipic acid, or dicarboxylic acid with little odor. Also, the use of known bleaching accelerators described in JP-A-53-95630, RD No. 17129, and US3,893,858 is preferable.

It is preferable to replenish 50 to 1,000 mL of a bleaching replenisher to a bleaching solution per $m^2$ of a light-sensitive material. The replenishment rate is more preferably 80 to 500 mL, and most preferably, 100 to 300 mL. Aeration of a bleaching solution is also preferable.

Compounds and processing conditions described in JP-A-4-125558, page 7, lower left column, line 10 to page 8, lower right column, line 19 can be applied to a processing solution with fixing capacity.

To improve the fixing rate and preservability, compounds represented by formulas (I) and (II) described in JP-A-6-301169 are preferably added singly or together to a processing solution with fixing capacity. To improve the preservability, the use of sulfinic acid such as p-toluenesulfinate described in JP-A-1-224762 is also preferable.

To improve the desilvering characteristics, ammonium is preferably used as a cation in a solution with bleaching capacity or in a solution with fixing capacity. However, the amount of ammonium is preferably reduced, or zero, to reduce environmental pollution.

In the bleaching, bleach-fixing, and fixing steps, it is particularly preferable to perform jet stirring described in JP-A-1-309059.

The replenishment rate of a replenisher in the bleach-fixing or fixing step is preferably 100 to 1,000 mL, more preferably, 150 to 700 mL, and most preferably, 200 to 600 mL per $m^2$ of a light-sensitive material. In the bleach-fixing or fixing step, an appropriate silver collecting apparatus is preferably installed either in-line or off-line to collect silver. When the apparatus is installed in-line, processing can be performed while the silver concentration in a solution is reduced, so the replenishment rate can be reduced. It is also preferable to install the apparatus off-line to collect silver and reuse the residual solution as a replenisher.

The bleach-fixing or fixing step can be performed by using a plurality of processing tanks, and these tanks are preferably cascaded to form a multistage counterflow system. To balance the size of a processor, a two-tank cascade system is generally efficient. The processing time ratio of the front tank to the rear tank is preferably 0.5 : 1 to 1 : 0.5, and more preferably, 0.8 : 1 to 1 : 0.8.

In a bleach-fixing or fixing solution, the presence of free chelating agents which are not metal complexes is preferable to improve the preservability. As these chelating agents, the use of the biodegradable chelating agents previously described in connection to a bleaching solution is preferred.

Contents described in aforementioned JP-A-4-125558, page 12, lower right column, line 6 to page 13, lower right column, line 16 can be preferably applied to the washing and stabilization steps. To improve the safety of the work environment, it is preferable to use azolylmethylamines described in EP504,609 and EP519,190 or N-methylolazoles described in JP-A-4-362943 instead of formaldehyde in a stabilizer and to make a magenta coupler divalent to form a solution of surfactant containing no image stabilizing agent such as formaldehyde.

To reduce adhesion of dust to a magnetic recording layer formed on a light-sensitive material, a stabilizer described in JP-A-6-289559 can be preferably used.

The replenishment rate of washing water and a stabilizer is preferably 80 to 1,000 mL, more preferably, 100 to 500 mL, and most preferably, 150 to 300 mL per $m^2$ of a light-sensitive material in order to maintain the washing and stabilization functions and at the same time reduce the waste liquors for environmental protection. In processing performed with this replenishment rate, it is preferable to prevent the propagation of bacteria and mildew by using known mildewproofing agents such as thiabendazole, 1,2-benzoisothiazoline-3-one, and 5-chloro-2-methylisothiazoline-3-one, antibiotics such as gentamicin, and water deionized by an ion exchange resin or the like. It is more effective to use deionized water together with a mildewproofing agent or an antibiotic.

The replenishment rate of a solution in a washing water tank or stabilizer tank is preferably reduced by performing reverse permeable membrane processing described in JP-A-3-46652, JP-A-3-53246, JP-A-3-55542, JP-A-3-121448, and JP-A-3-126030. A reverse permeable membrane used in this processing is preferably a low-pressure reverse permeable membrane.

In the processing of the present invention, it is particularly preferable to perform processing solution evaporation correction disclosed in JIII Journal of Technical Disclosure No. 94-4992. In particular, a method of performing correction on the basis of (formula-1) on page 2 by using temperature and humidity information of an environment in which a

processor is installed is preferred. Water for use in this evaporation correction is preferably taken from the washing water replenishment tank. If this is the case, deionized water is preferably used as the washing replenishing water.

Processing agents described in aforementioned JIII Journal of Technical Disclosure No. 94-4992, page 3, right column, line 15 to page 4, left column, line 32, are preferably used in the present invention. As a processor for these processing agents, a film processor described on page 3, right column, lines 22 to 28 is preferred.

Practical examples of processing agents, automatic processors, and evaporation correction methods suited to practicing the present invention are described in the same JIII Journal of Technical Disclosure No. 94-4992, page 5, right column, line 11 to page 7, right column, last line.

Processing agents used in the present invention can be supplied in any form: a liquid agent having the concentration of a solution to be used, concentrated liquid agent, granules, powder, tablets, paste, and emulsion. Examples of such processing agents are a liquid agent contained in a low-oxygen-permeable vessel disclosed in JP-A-63-17453, vacuum-packed powders and granules disclosed in JP-A-4-19655 and JP-A-4-230748, granules containing a water-soluble polymer disclosed in JP-A-4-221951, tablets disclosed in JP-A-51-61837 and JP-A-6-102628, and a paste disclosed in International Patent Laid-Open No. 57-500485. Although any of these processing agents can be preferably used, the use of a liquid adjusted to have the concentration of a solution to be used is preferable for the sake of convenience in use.

As a vessel for containing these processing agents, polyethylene, polypropylene, polyvinylchloride, polyethyleneterephthalate, and nylon are used singly or as a composite material. These materials are selected in accordance with the level of necessary oxygen permeability. For a readily oxidizable solution such as a color developer, a low-oxygen-permeable material is preferred. More specifically, polyethyleneterephthalate or a composite material of polyethylene and nylon is favorable. A vessel made of any of these materials preferably has a thickness of 500 to 1,500 μm and an oxygen permeability of 20 $mL/m^2 \cdot 24$ hrs·atm or less.

Color reversal film processing solutions used in the present invention will be described below.

Processing for a color reversal film is described in detail in Aztech Ltd., Known Technology No. 6 (1991, April 1), page 1, line 5 to page 10, line 5 and page 15, line 8 to page 24, line 2, and any of the contents can be preferably applied.

In this color reversal film processing, an image stabilizing agent is contained in a control bath or a final bath. Preferable examples of this image stabilizing agent are formalin, sodium formaldehyde-bisulfite, and N-methylolazole. Sodium formaldehyde-bisulfite or N-methylolazole is preferred in terms of work environment, and N-methyloltriazole is particularly preferred as N-methylolazole. The contents pertaining to a color developer, bleaching solution, fixing solution, and washing water described in the color negative film processing can be preferably applied to the color reversal film processing.

Preferred examples of color reversal film processing agents containing the above contents are the E-6 processing agent manufactured by Eastman Kodak Co. and the CR-56 processing agent manufactured by Fuji Photo Film Co., Ltd.

The present invention will be described in more detail below by way of its examples. However, the present invention is not limited to these examples.

(Example 1)

Preparation of sample 101

A multilayer color lightsensitive material consisting of a support of 127 μm thick undercoated triacetylcellulose film and, superimposed thereon, a given number of layers of the following compositions was prepared and designated sample 101. The figures are for the addition amount per $m^2$. The effects of added compounds are not limited to described uses.

With respect to the following gelatins, use was made of those of 100 thousand to 200 thousand molecular weight (mass average molecular weight). With respect to the contents of major metal ions therein, the content of calcium was in the range of 2500 to 3000 ppm; the content of iron was in the range of 1 to 7 ppm; and the content of sodium was in the range of 1500 to 3000 ppm.

Moreover, gelatin of 1000 ppm or less calcium content was used in combination therewith.

In the preparation of each of the layers, the organic compounds to be added were formed into gelatinous emulsified dispersions (surfactants W-2, W-3 and W-4 used). Also, lightsensitive emulsions and yellow colloidal silver were formed into respective gelatinous dispersions. These dispersions were mixed together, thereby obtaining a coating liquid so formulated as to realize described addition amounts. The obtained coating liquid was subjected to coating operation. Compounds Cpd-H, O, P and Q and dyes D-1, 2, 3, 5, 6, 8 and 9, H-1, P-3 and F-1 to 9 were dissolved in water or appropriate water miscible organic solvents, such as methanol, dimethylformamide, ethanol and dimethylacetamide, and added to the coating liquid for each of the layers.

The gelatin concentration of each of the thus prepared layers (mass of gelatin solid contents / volume of coating liquid) was in the range of 2.5 to 15.0%. The pH value of each of the coating liquids was in the range of 5.0 to 8.5. With

respect to the coating liquids for the layers containing silver halide emulsions, the pAg value upon regulation to pH 6.0 and 40˚C was in the range of 7.0 to 9.5.

The coating operation was followed by drying operation through a multi-stage drying process wherein the temperature was controlled so as to be in the range of 10 to 45˚C. Thus, the desired sample was obtained.

1st layer: Antihalation layer

| | |
|---|---|
| Black colloidal silver | 0.20 g |
| Gelatin | 2.20 g |
| Compound Cpd-B | 0.010 g |
| Ultraviolet absorbent U-1 | 0.050 g |
| Ultraviolet absorbent U-3 | 0.020 g |
| Ultraviolet absorbent U-4 | 0.020 g |
| Ultraviolet absorbent U-5 | 0.010 g |
| Ultraviolet absorbent U-2 | 0.070 g |
| Compound Cpd-F | 0.20 g |
| Compound Cpd-R | 0.020 g |
| Compound Cpd-S | 0.020 g |
| High-boiling organic solvent Oil-2 | 0.020 g |
| High-boiling organic solvent Oil-6 | 0.020 g |
| High-boiling organic solvent Oil-8 | 0.020 g |
| Dye D-4 | 1.0 mg |
| Dye D-8 | 1.0 mg |
| Fine crystal solid dispersion of dye E-1 | 0.05 g |

2nd layer: Interlayer

| | |
|---|---|
| Gelatin | 0.40 g |
| Compound Cpd-F | 0.050 g |
| High-boiling organic solvent Oil-6 | 0.010 g |

3rd layer: Sensitive emulsion layer

| | | | |
|---|---|---|---|
| Emulsion R | silver | | 0.20 g |
| Emulsion S | silver | | 0.10 g |
| Fine-grain silver iodide (cubic, av. equivalent-sphere diameter 0.05 μm) | | silver | 0.050 g |
| Gelatin | | | 0.50 g |
| Compound Cpd-M | | | 0.030 g |
| High-boiling organic solvent Oil-6 | | | 0.030 g |
| High-boiling organic solvent Oil-7 | | | 5.0 mg |
| Dye D-7 | | | 4.0 mg |

4th layer: Interlayer

| | |
|---|---|
| Gelatin | 1.50 g |

(continued)

| | |
|---|---|
| Compound Cpd-M | 0.10 g |
| Compound Cpd-F | 0.030 g |
| Compound Cpd-D | 0.010 g |
| Compound Cpd-K | 3.0 mg |
| Ultraviolet absorbent U-6 | 0.010 g |
| High-boiling organic solvent Oil-6 | 0.10 g |
| High-boiling organic solvent Oil-3 | 0.010 g |
| High-boiling organic solvent Oil-4 | 0.010 g |

5th layer: Low-speed red-sensitive emulsion layer

| | | |
|---|---|---|
| Emulsion A | silver | 0.15 g |
| Emulsion B | silver | 0.10 g |
| Emulsion C | silver | 0.15 g |
| Yellow colloidal silver | silver | 1.0 mg |
| Gelatin | | 0.60 g |
| Coupler C-1 | | 0.15 g |
| Coupler C-2 | | 7.0 mg |
| Coupler C-9 | | 2.0 mg |
| Ultraviolet absorbent U-2 | | 3.0 mg |
| Compound Cpd-D | | 1.0 mg |
| Compound Cpd-J | | 2.0 mg |
| High-boiling organic solvent Oil-2 | | 0.040 g |

6th layer: Medium-speed red-sensitive emulsion layer

| | | |
|---|---|---|
| Emulsion C | silver | 0.20 g |
| Emulsion D | silver | 0.15 g |
| Silver bromide emulsion which internal thereof was fogged. (cubic, av. equivalent-sphere diameter 0.11 μm) | silver | 0.010 g |
| Gelatin | | 0.60 g |
| Coupler C-1 | | 0.15 g |
| Coupler C-2 | | 7.0 mg |
| Compound Cpd-D | | 1.5 mg |
| High-boiling organic solvent Oil-2 | | 0.045 g |
| Compound Cpd-T | | 2.0 mg |

7th layer: High-speed red-sensitive emulsion layer

| | | |
|---|---|---|
| Emulsion E | silver | 0.15 g |
| Emulsion F | silver | 0.20 g |
| Fine-grain silver iodobromide (av. silver iodide content 0.1 mol%, av. equivalent-sphere diameter 0.05 μm) | silver | 0.03 g |
| Gelatin | | 1.50 g |

(continued)

| | |
|---|---|
| Coupler C-1 | 0.70 g |
| Coupler C-2 | 0.025 g |
| Coupler C-3 | 0.020 g |
| Coupler C-8 | 3.0 mg |
| Ultraviolet absorbent U-1 | 0.010 g |
| High-boiling organic solvent Oil-2 | 0.20 g |
| High-boiling organic solvent Oil-9 | 0.030 g |
| Compound Cpd-D | 5.0 mg |
| Compound Cpd-L | 1.0 mg |
| Compound Cpd-T | 0.020 g |
| Additive P-1 | 0.010 g |
| Additive P-3 | 0.030 g |

8th layer: Interlayer

| | |
|---|---|
| Gelatin | 0.50 g |
| Additive P-2 | 0.10 g |
| Dye D-5 | 0.020 g |
| Dye D-9 | 6.0 mg |
| Compound Cpd-I | 0.020 g |
| Compound Cpd-O | 3.0 mg |
| Compound Cpd-P | 5.0 mg |
| High-boiling organic solvent Oil-6 | 0.050 g |

9th layer: Interlayer

| | | |
|---|---|---|
| Yellow colloidal silver | silver | 0.010 g |
| Gelatin | | 1.00 g |
| Additive P-2 | | 0.05 g |
| Compound Cpd-A | | 0.050 g |
| Compound Cpd-D | | 0.030 g |
| Compound Cpd-M | | 0.10 g |
| High-boiling organic solvent Oil-6 | | 0.10 g |
| High-boiling organic solvent Oil-3 | | 0.010 g |

10th layer: Low-speed green-sensitive emulsion layer

| | | |
|---|---|---|
| Emulsion G | silver | 0.15 g |
| Emulsion H | silver | 0.15 g |
| Emulsion I | silver | 0.15 g |
| Gelatin | | 1.00 g |
| Coupler C-4 | | 0.040 g |
| Coupler C-5 | | 0.080 g |
| Compound Cpd-B | | 0.010 g |
| Compound Cpd-G | | 2.5 mg |

(continued)

| | | |
|---|---|---|
| Compound Cpd-K | | 2.0 mg |
| Compound Cpd-U | | 3.0 mg |
| High-boiling organic solvent Oil-2 | | 0.020 g |
| High-boiling organic solvent Oil-5 | | 0.020 g |
| Additive P-1 | | 5.0 mg |

11th layer: Medium-speed green-sensitive emulsion layer

| | | |
|---|---|---|
| Emulsion I | silver | 0.10 g |
| Emulsion J | silver | 0.20 g |
| Gelatin | | 0.50 g |
| Coupler C-4 | | 0.10 g |
| Coupler C-5 | | 0.050 g |
| Coupler C-6 | | 0.010 g |
| Compound Cpd-B | | 0.020 g |
| High-boiling organic solvent Oil-2 | | 0.020 g |
| High-boiling organic solvent Oil-5 | | 0.020 g |

12th layer: High-speed green-sensitive emulsion layer

| | | |
|---|---|---|
| Emulsion K | silver | 0.40 g |
| Silver bromide emulsion which internal thereof was fogged. (cubic, av. equivalent-sphere diameter 0.11 μm) | silver | 5.0 mg |
| Gelatin | | 1.20 g |
| Coupler C-4 | | 0.60 g |
| Coupler C-5 | | 0.30 g |
| Coupler C-7 | | 0.050 g |
| Compound Cpd-B | | 0.030 g |
| High-boiling organic solvent Oil-2 | | 0.020 g |
| High-boiling organic solvent Oil-5 | | 0.050 g |
| Additive P-1 | | 0.020 g |

13th layer: Yellow filter layer

| | | |
|---|---|---|
| Yellow colloidal silver | silver | 2.0 mg |
| Gelatin | | 1.0 g |
| Compound Cpd-C | | 0.010 g |
| Compound Cpd-M | | 0.020 g |
| High-boiling organic solvent Oil-1 | | 0.020 g |
| High-boiling organic solvent Oil-6 | | 0.020 g |
| Fine-crystal solid dispersion of dye E-2 | | 0.25 g |

14th layer: Sensitive emulsion layer

| | | |
|---|---|---|
| Emulsion T | silver | 0.20 g |
| Gelatin | | 0.40 g |
| Coupler C-1 | | 5.0 mg |
| Coupler C-2 | | 0.5 mg |
| High-boiling organic solvent Oil-5 | | 2.0 mg |
| Compound Cpd-Q | | 0.20 g |
| Dye D-6 | | 4.0 mg |

15th layer: Low-speed blue-sensitive emulsion layer

| | | |
|---|---|---|
| Emulsion L | silver | 0.10 g |
| Emulsion M | silver | 0.10 g |
| Emulsion N | silver | 0.10 g |
| Gelatin | | 0.80 g |
| Coupler C-10 | | 0.35 mg |
| Compound Cpd-B | | 0.015 g |
| Compound Cpd-I | | 8.0 mg |
| Compound Cpd-K | | 1.0 mg |
| Ultraviolet absorbent U-5 | | 0.015 g |
| High-boiling organic solvent Oil-2 | | 0.015 g |
| Additive P-1 | | 0.020 g |

16th layer: Medium-speed blue-sensitive emulsion layer

| | | |
|---|---|---|
| Emulsion N | silver | 0.10 g |
| Emulsion O | silver | 0.20 g |
| Gelatin | | 0.80 g |
| Coupler C-8 | | 0.050 g |
| Coupler C-10 | | 0.30 g |
| Compound Cpd-B | | 0.010 g |
| Compound Cpd-E | | 0.020 g |
| Compound Cpd-N | | 2.0 mg |
| Compound Cpd-T | | 0.015 g |
| Ultraviolet absorbent U-5 | | 0.015 g |
| High-boiling organic solvent Oil-2 | | 0.015 g |
| Additive P-1 | | 0.020 g |

17th layer: High-speed blue-sensitive emulsion layer

| | | |
|---|---|---|
| Emulsion P | silver | 0.20 g |
| Emulsion Q | silver | 0.25 g |
| Gelatin | | 2.00 g |
| Coupler C-3 | | 0.20 g |

(continued)

| | |
|---|---|
| Coupler C-10 | 1.20 g |
| High-boiling organic solvent Oil-2 | 0.020 g |
| High-boiling organic solvent Oil-5 | 0.030 g |
| Ultraviolet absorbent U-2 | 0.030 g |
| Ultraviolet absorbent U-5 | 0.030 g |
| Compound Cpd-D | 3.0 mg |
| Compound Cpd-E | 0.020 g |
| Compound Cpd-F | 0.020 g |
| Compound Cpd-N | 5.0 mg |
| Compound Cpd-T | 0.050 g |
| Additive P-1 | 0.010 g |

18th layer: 1st protective layer

| | |
|---|---|
| Gelatin | 0.70 g |
| Ultraviolet absorbent U-1 | 0.030 g |
| Ultraviolet absorbent U-2 | 0.10 g |
| Ultraviolet absorbent U-5 | 0.050 g |
| Compound Cpd-B | 0.030 g |
| Compound Cpd-O | 5.0 mg |
| Compound Cpd-A | 0.030 g |
| Compound Cpd-H | 0.20 g |
| Dye D-1 | 8.0 mg |
| Dye D-2 | 0.010 g |
| Dye D-3 | 0.010 g |
| High-boiling organic solvent Oil-3 | 0.040 g |

19th layer: 2nd protective layer

| | | |
|---|---|---|
| Colloidal silver | silver | 2.5 mg |
| Fine-grain silver iodobromide emulsion (av. silver iodide content 1 mol%, av. equivalent-sphere diameter 0.06 μm) | | |
| silver | | 0.10 g |
| Gelatin | | 0.80 g |
| Ultraviolet absorbent U-2 | | 0.030 g |
| Ultraviolet absorbent U-5 | | 0.030 g |
| High-boiling organic solvent Oil-3 | | 0.010 g |

20th layer: 3rd protective layer

| | |
|---|---|
| Gelatin | 1.00 g |
| Polymethylmethacrylate (average grain size 1.5 μm) | 0.10 g |
| 6 : 4 copolymer of methylmethacrylate and methacrylic acid (average grain size 1.5 μm) | |

(continued)

| | |
|---|---|
| | 0.15 g |
| Silicone oil SO-1 | 0.20 g |
| Surfactant W-1 | 0.020 g |
| Surfactant W-2 | 0.040 g |

In addition to the above compositions, additives F-1 to F-9 were added to all emulsion layers. Also, a gelatin hardener H-1 and surfactants W-2, W-3 and W-4 for coating and emulsification were added to each layer.

Furthermore, phenol, 1,2-benzisothiazoline-3-one, 2-phenoxyethanol, phenethylalcohol, and p-benzoic butylester were added as antiseptic and mildewproofing agents.

The thus produced sample 101 in the dry state had a coating thickness of 25.5 μm, and exhibited a swelling ratio, upon swelling in distilled water at 25°C, of 1.85.

Table 1 Structures of silver halide emulsions

| Silver iodobromide emulsions used in sample 101 | | | | | | | | | | | | |
|---|---|---|---|---|---|---|---|---|---|---|---|---|
| Emulsion | Characteristics | Average equivalent-sphere diameter (μm) | Variation coefficient (%) | Average silver iodide content (mol%) | Halogen composition structure of silver halide grain | Silver iodide content of grain surface (mol%) | Other characteristics | | | | |
| | | | | | | | ① | ② | ③ | ④ | ⑤ |
| A | Monodisperse grain tetradecahedral grain | 0.18 | 10 | 3.5 | Triple | 1.5 | ○ | ○ | | ○ | |
| B | Monodisperse (111) tabular grain Average aspect ratio 3.0 | 0.20 | 10 | 2.5 | Quadruple | 1.5 | | | ○ | | ○ |
| C | Monodisperse (111) tabular grain Average aspect ratio 4.5 | 0.32 | 11 | 1.8 | Triple | 0.1 | | ○ | | ○ | ○ |
| D | Monodisperse (111) tabular grain Average aspect ratio 6.0 | 0.32 | 21 | 4.8 | Triple | 2.0 | | ○ | | ○ | ○ |
| E | Monodisperse (111) tabular grain Average aspect ratio 6.0 | 0.48 | 12 | 2.0 | Quadruple | 1.3 | | ○ | | | |
| F | Monodisperse (111) tabular grain Average aspect ratio 8.0 | 0.65 | 12 | 1.6 | Triple | 0.6 | | ○ | ○ | | ○ |
| G | Monodisperse cubic grain | 0.14 | 9 | 3.5 | Quadruple | 3.0 | ○ | | ○ | ○ | |
| H | Monodisperse cubic grain | 0.22 | 12 | 1.9 | Quadruple | 0.7 | | ○ | | | |

(continued)

| Silver iodobromide emulsions used in sample 101 | | | | | | | | | | | |
|---|---|---|---|---|---|---|---|---|---|---|---|
| Emulsion | Characteristics | Average equivalent-sphere diameter (μm) | Variation coefficient (%) | Average silver iodide content (mol%) | Halogen composition structure of silver halide grain | Silver iodide content of grain surface (mol%) | Other characteristics | | | | |
| | | | | | | | ① | ② | ③ | ④ | ⑤ |
| I | Monodisperse (111) tabular grain Average aspect ratio 4.0 | 0.35 | 12 | 3.5 | Quintuple | 4.5 | ○ | ○ | | ○ | ○ |
| J | Monodisperse (111) tabular grain Average aspect ratio 7.0 | 0.40 | 21 | 2.0 | Quadruple | 0.2 | | ○ | | ○ | ○ |
| K | Monodisperse (111) tabular grain Average aspect ratio 8.5 | 0.65 | 13 | 1.7 | Triple | 1.3 | ○ | ○ | ○ | | ○ |
| L | Monodisperse tetradecahedral grain | 0.30 | 9 | 7.5 | Triple | 7.0 | | | ○ | | ○ |
| M | Monodisperse tetradecahedral grain | 0.30 | 9 | 7.5 | Triple | 5.0 | | ○ | | ○ | ○ |
| N | Monodisperse (111) tabular grain Average aspect ratio 3.0 | 0.35 | 13 | 2.1 | Quintuple | 4.0 | ○ | ○ | ○ | | |
| O | Monodisperse (111) tabular grain Average aspect ratio 5.0 | 0.45 | 9 | 2.5 | Quadruple | 1.0 | | ○ | ○ | ○ | ○ |

(continued)

| Silver iodobromide emulsions used in sample 101 | | | | | | | | | | | |
|---|---|---|---|---|---|---|---|---|---|---|---|
| Emulsion | Characteristics | Average equivalent-sphere diameter (μm) | Variation coefficient (%) | Average silver iodide content (mol%) | Halogen composition structure of silver halide grain | Silver iodide content of grain surface (mol%) | Other characteristics | | | | |
| | | | | | | | ① | ② | ③ | ④ | ⑤ |
| P | Monodisperse (111) tabular grain Average aspect ratio 9.0 | 0.70 | 21 | 2.8 | Triple | 0.5 | ○ | ○ | | | ○ |
| Q | Monodisperse (111) tabular grain Average aspect ratio 9.0 | 0.85 | 8 | 1.0 | Quadruple | 0.5 | ○ | ○ | | | ○ |
| R | Monodisperse (111) tabular grain Average aspect ratio 5.0 | 0.50 | 11 | 8.0 | Quadruple | 4.0 | ○ | ○ | | | ○ |
| S | Monodisperse (111) tabular grain Average aspect ratio 4.0 | 0.85 | 13 | 12.5 | Quadruple | 3.0 | | ○ | ○ | | ○ |
| T | Monodisperse (111) tabular grain Average aspect ratio 4.0 | 0.55 | 13 | 10.5 | Quadruple | 2.8 | ○ | ○ | | | ○ |

(Other characteristics)
① A reduction sensitizer was added during grain formation.
② A selenium sensitizer was used as an after-ripening chemical.
③ A rhodium salt was added during grain formation.
④ Subsequently after-ripening, 10% silver nitrate based on silver molar ratio to the emulsion grain at that time and its equimolar potassium bromide were added and the shell formation was carried out.
⑤ It was observed by a transmission electron microscope that 10 or more of dislocation lines per one grain exist in average.

Further, all of the lightsensitive emulsions were post-ripened using sodium thiosulfate, potassium thiocyanate and sodium chloroaurate.

Further, an iridium salt was appropriately added during grain formation.

Further, a chemically modified gelatin in which a portion of the amino group of gelatin was converted to phthalic amide was added to the emulsion B, C, E, H, J, N, Q, R, S and T.

Table 2 Spectral sensitization of emulsions A to T

| Emulsion | Added sensitizing dye | Addition amount (g) per mol of silver halide | Addition timing of sensitizing dye |
|---|---|---|---|
| A | S-1 | 0.01 | Subsequently to after-ripening |
| | S-2 | 0.20 | Prior to after-ripening |
| | S-3 | 0.02 | Prior to after-ripening |
| | S-8 | 0.08 | Prior to after-ripening |
| | S-13 | 0.05 | Prior to after-ripening |
| B | S-2 | 0.20 | Prior to after-ripening |
| | S-8 | 0.08 | Prior to after-ripening |
| | S-13 | 0.05 | Prior to after-ripening |
| | S-14 | 0.01 | Prior to after-ripening |
| C | S-2 | 0.20 | Prior to after-ripening |
| | S-8 | 0.08 | Prior to after-ripening |
| | S-13 | 0.20 | Prior to after-ripening |
| D | S-2 | 0.20 | Subsequently to after-ripening |
| | S-3 | 0.05 | Subsequently to after-ripening |
| | S-8 | 0.08 | Prior to after-ripening |
| | S-13 | 0.25 | Prior to after-ripening |
| E | S-1 | 0.01 | Prior to after-ripening |
| | S-2 | 0.25 | Prior to after-ripening |
| | S-8 | 0.05 | Prior to after-ripening |
| | S-13 | 0.25 | Subsequently to after-ripening |
| F | S-2 | 0.25 | Prior to after-ripening |
| | S-3 | 0.02 | Prior to after-ripening |
| | S-8 | 0.05 | Prior to after-ripening |
| G | S-4 | 0.33 | Subsequently to after-ripening |
| | S-5 | 0.05 | Subsequently to after-ripening |
| | S-12 | 0.10 | Subsequently to after-ripening |
| H | S-4 | 0.25 | Prior to after-ripening |
| | S-5 | 0.05 | Subsequently to after-ripening |
| | S-9 | 0.10 | Prior to after-ripening |
| | S-14 | 0.02 | Subsequently to after-ripening |
| I | S-4 | 0.30 | Prior to after-ripening |
| | S-9 | 0.20 | Prior to after-ripening |
| | S-12 | 0.10 | Prior to after-ripening |

(continued)

| Emulsion | Added sensitizing dye | Addition amount (g) per mol of silver halide | Addition timing of sensitizing dye |
|---|---|---|---|
| J | S-4 | 0.35 | Prior to after-ripening |
| | S-5 | 0.05 | Subsequently to after-ripening |
| | S-12 | 0.10 | Prior to after-ripening |
| K | S-4 | 0.30 | Prior to after-ripening |
| | S-9 | 0.05 | Prior to after-ripening |
| | S-12 | 0.10 | Prior to after-ripening |
| | S-14 | 0.02 | Prior to after-ripening |
| L, M | S-6 | 0.10 | Subsequently to after-ripening |
| | S-10 | 0.20 | Subsequently to after-ripening |
| | S-11 | 0.05 | Subsequently to after-ripening |
| N | S-6 | 0.05 | Subsequently to after-ripening |
| | S-7 | 0.05 | Subsequently to after-ripening |
| | S-10 | 0.25 | Subsequently to after-ripening |
| | S-11 | 0.05 | Subsequently to after-ripening |
| O | S-10 | 0.40 | Subsequently to after-ripening |
| | S-11 | 0.15 | Subsequently to after-ripening |
| P | S-6 | 0.05 | Subsequently to after-ripening |
| | S-7 | 0.05 | Subsequently to after-ripening |
| | S-10 | 0.30 | Prior to after-ripening |
| | S-11 | 0.10 | Prior to after-ripening |
| Q | S-6 | 0.05 | Prior to after-ripening |
| | S-7 | 0.05 | Prior to after-ripening |
| | S-10 | 0.20 | Prior to after-ripening |
| | S-11 | 0.25 | Prior to after-ripening |
| R | S-15 | 0.35 | Subsequently to after-ripening |
| | S-4 | 0.15 | Subsequently to after-ripening |
| S | S-15 | 0.30 | Subsequently to after-ripening |
| | S-4 | 0.20 | Subsequently to after-ripening |
| | S-10 | 0.05 | Prior to after-ripening |
| T | S-6 | 0.05 | Prior to after-ripening |
| | S-7 | 0.05 | Prior to after-ripening |
| | S-10 | 0.30 | Prior to after-ripening |

Comparative compound A

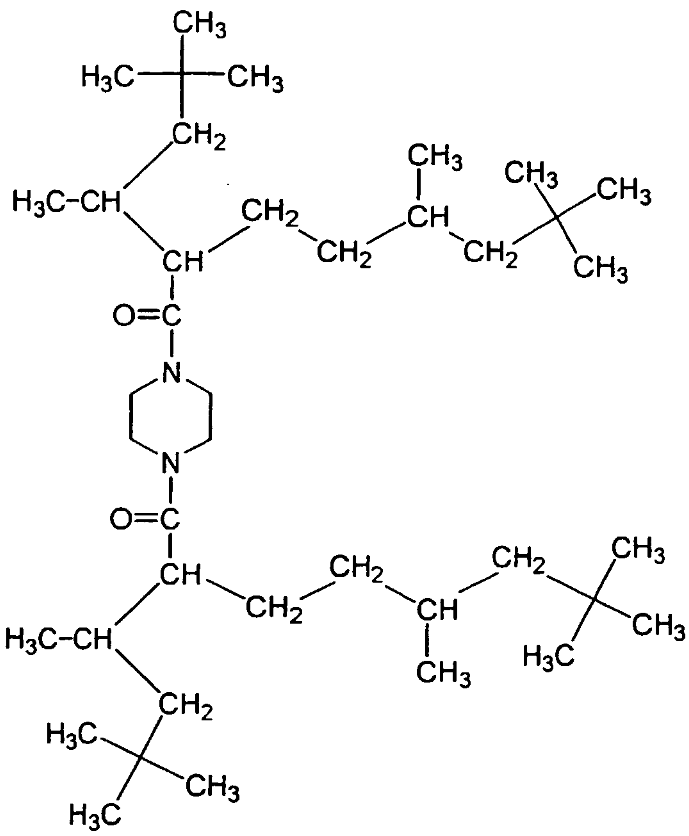

Compound described in JP-A-8-110624

C-1

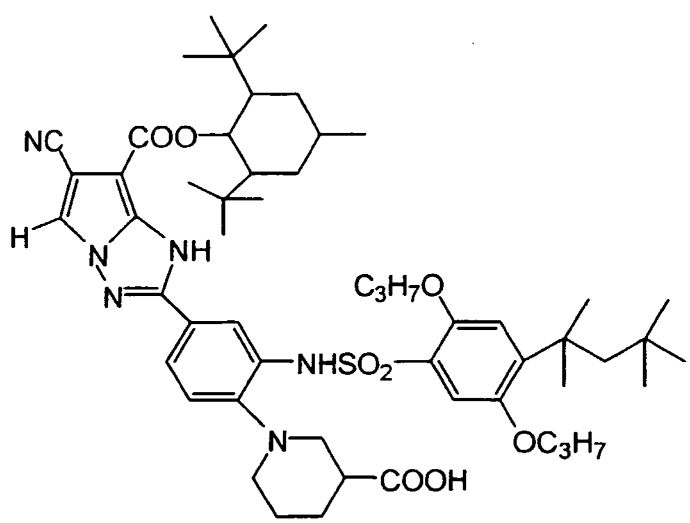

C-2

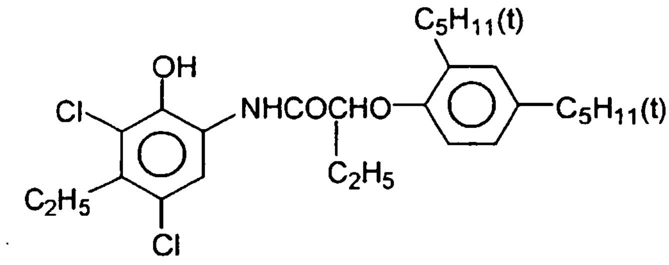

C-3

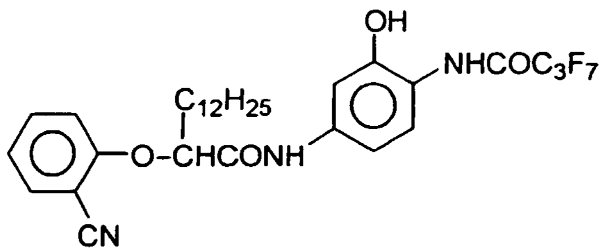

C-4

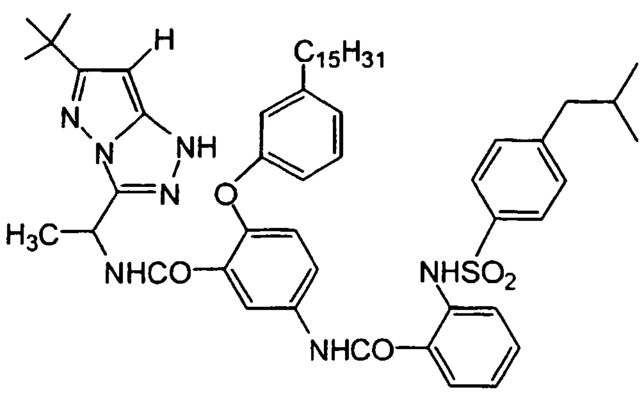

C-5

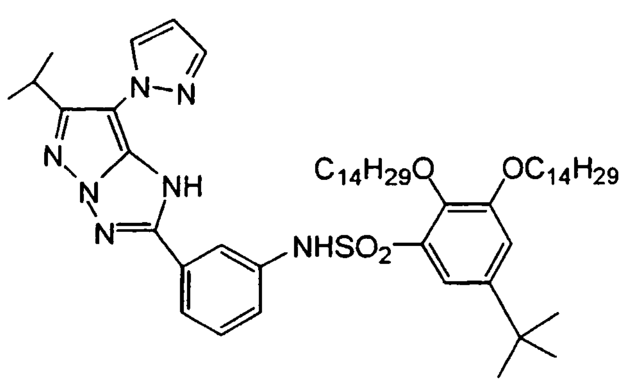

C-6

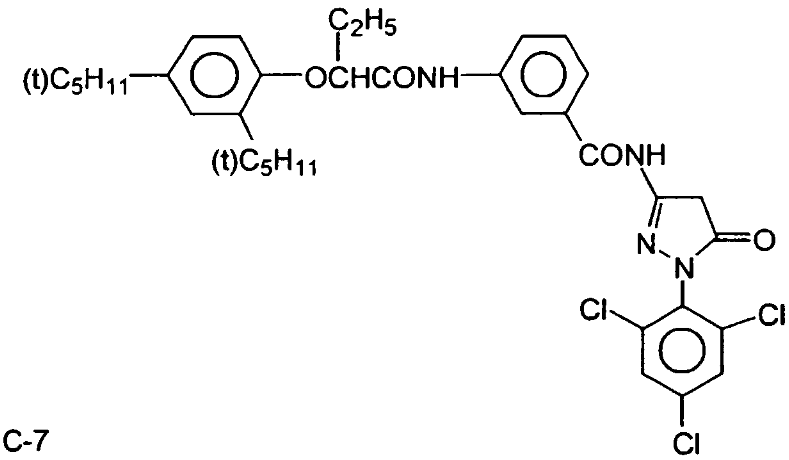

C-7

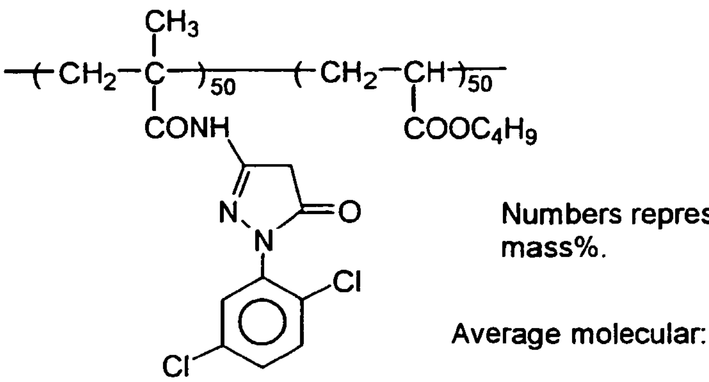

Numbers represent mass%.

Average molecular: about 25,000

C-8

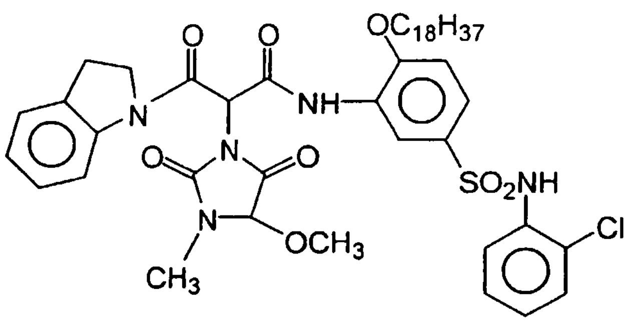

C-9

C-6

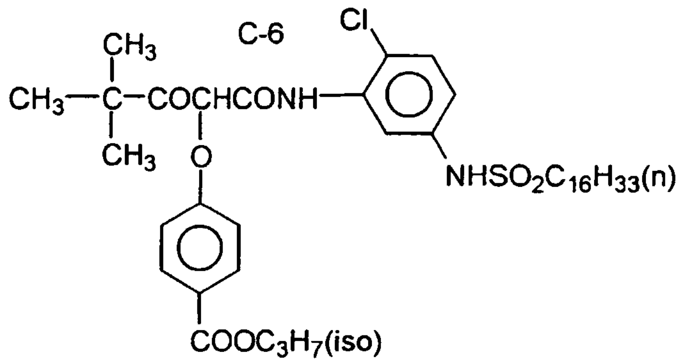

C-10

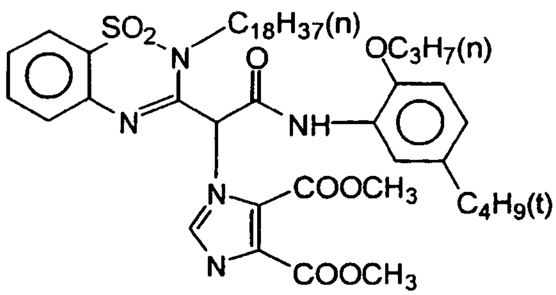

Oil-1 Tri-n-hexyl phosphate

Oil-2 Tricresyl phosphate

Oil-3

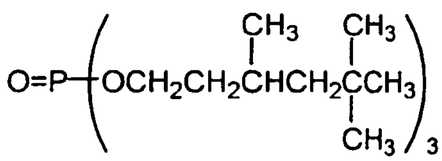

Oil-4 Tricyclohexyl phosphate

Oil-5

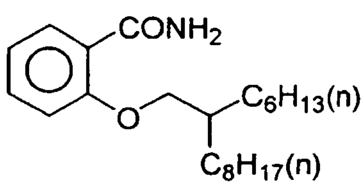

(continued)

Oil-6

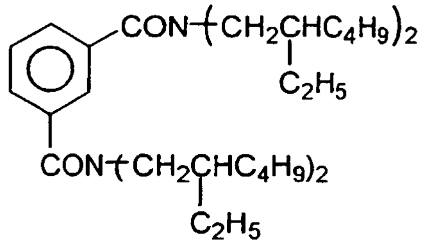

Oil-7

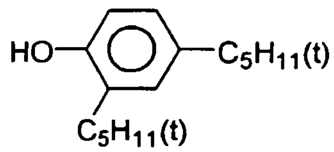

Oil-8

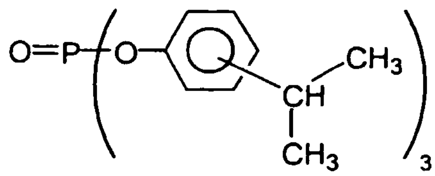

(Mixture of isomers)

Oil-9

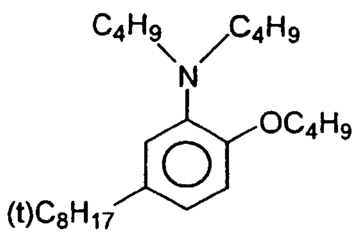

Cpd-A

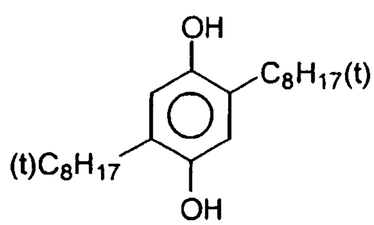

Cpd-B

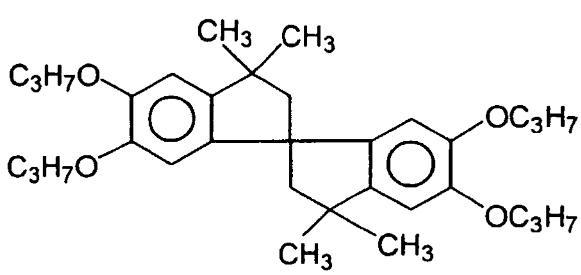

(continued)

Cpd-C

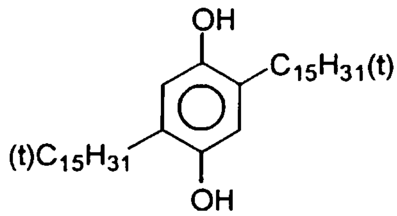

Cpd-D

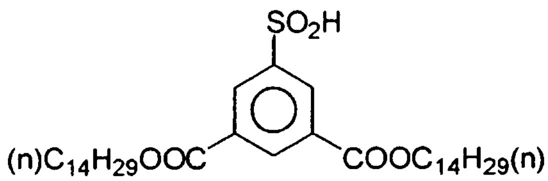

Cpd-E

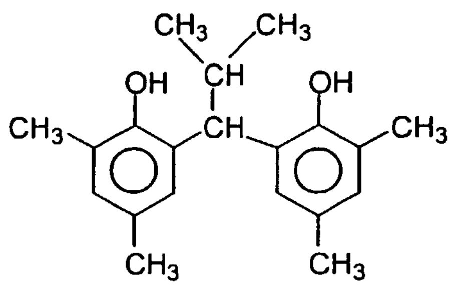

Cpd-F

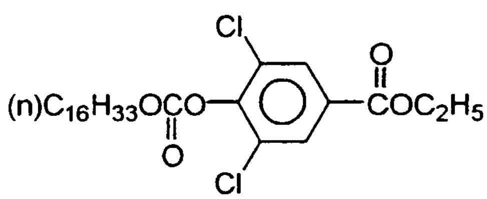

Cpd-G

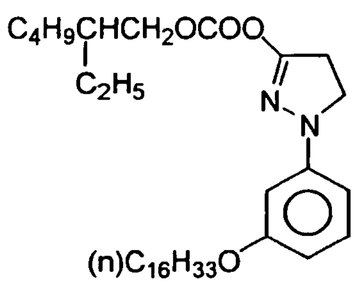

(continued)

Cpd-H

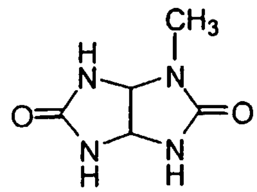

Cpd-I

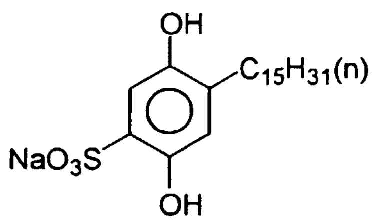

Cpd-J Cpd-K

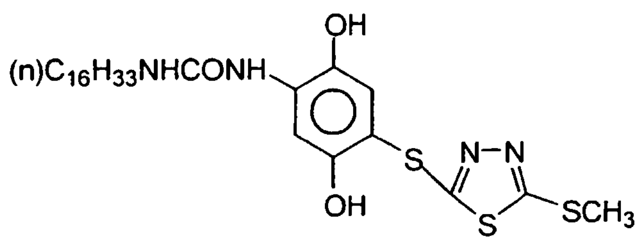

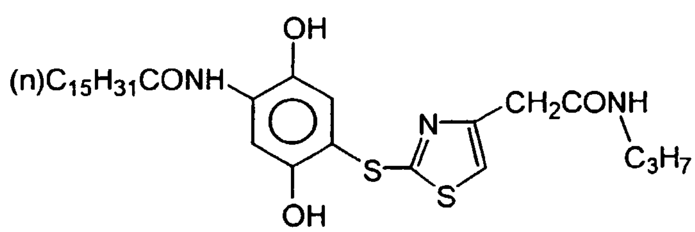

Cpd-L

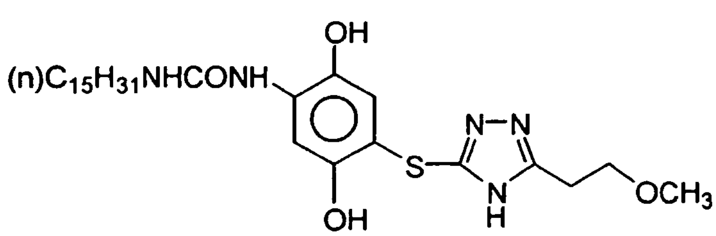

(continued)

Cpd-M

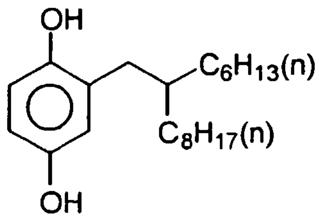

Cpd-N

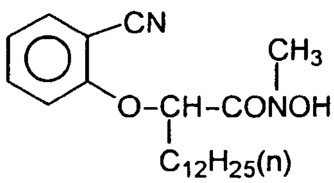

Cpd-O

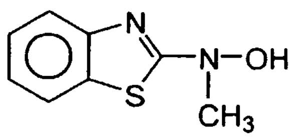

Cpd-P

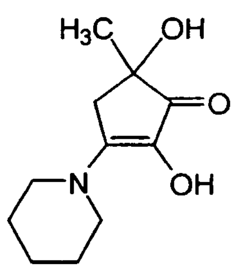

Cpd-Q

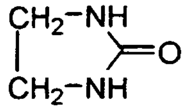

(continued)

Cpd-R

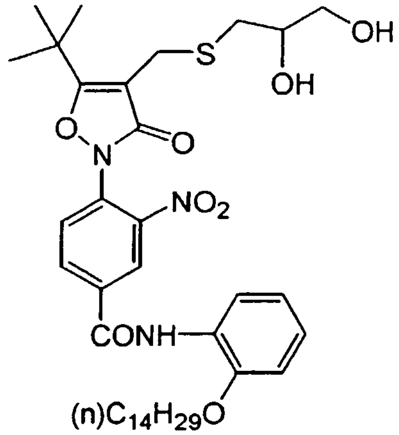

Cpd-S

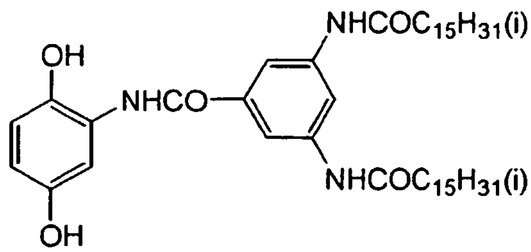

Cpd-T

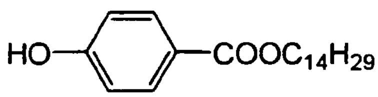

Cpd-U

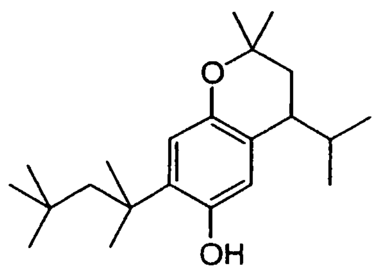

U-1

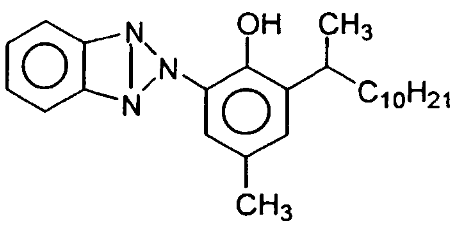

U-2

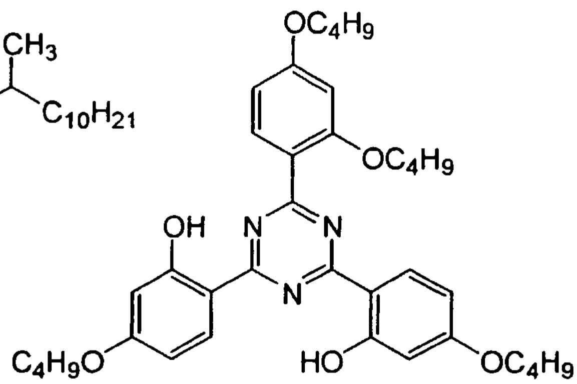

U-3

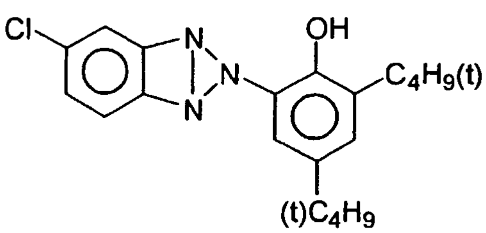

U-4

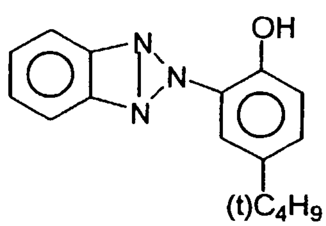

U-5

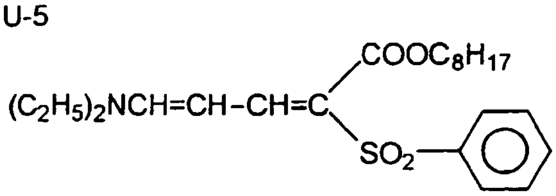

U-6

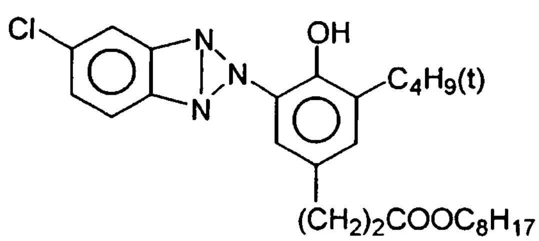

S-1

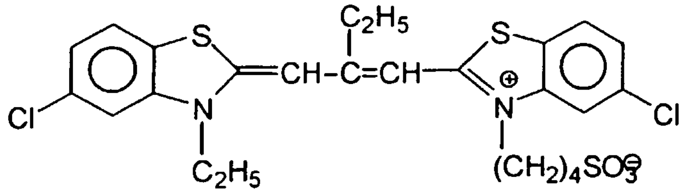

S-2

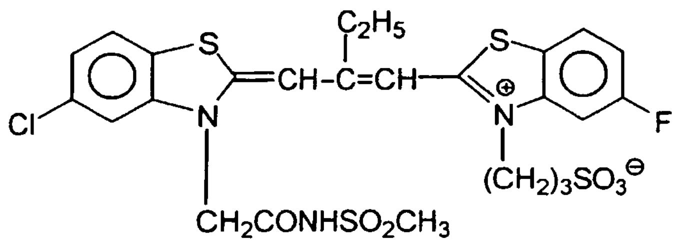

S-3

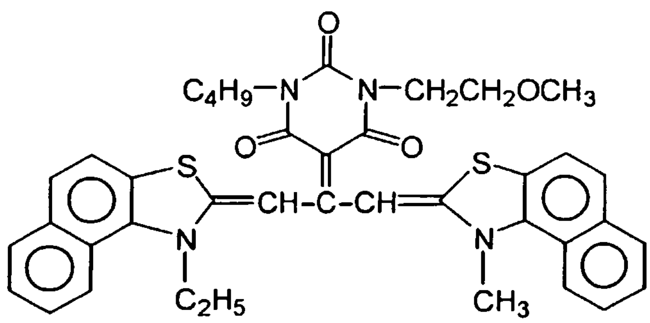

S-4

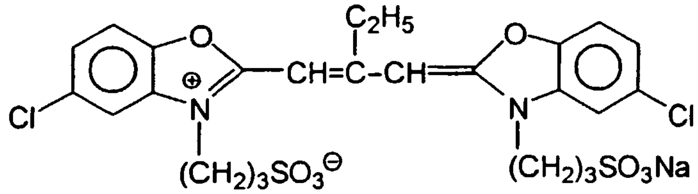

S-5

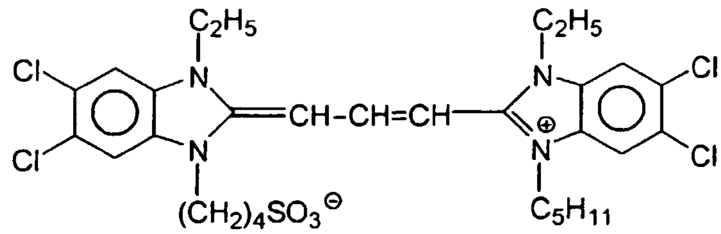

S-6

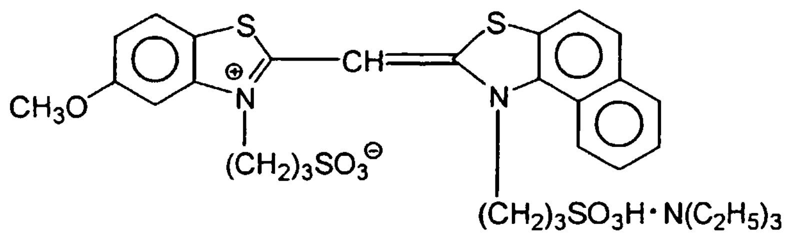

S-7

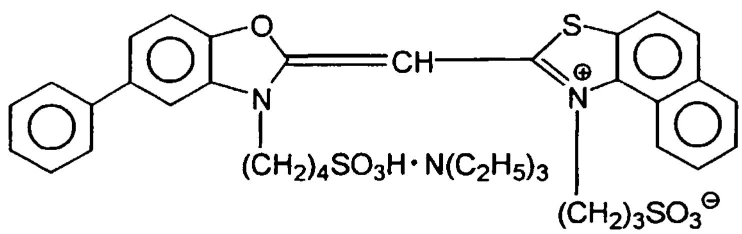

S-8

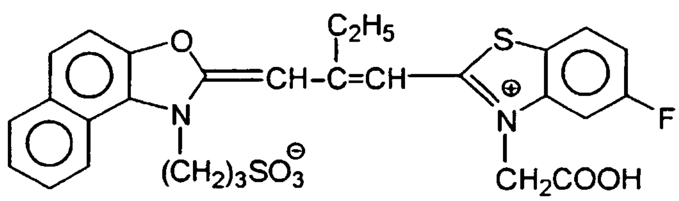

S-9

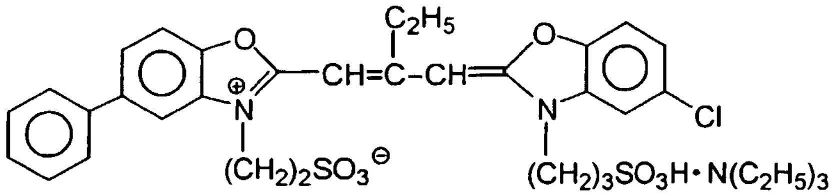

S-10

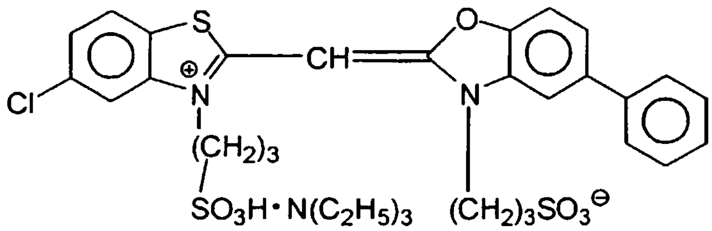

S-11

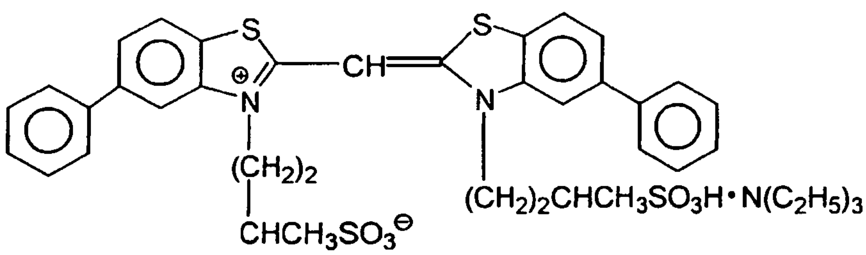

S-12

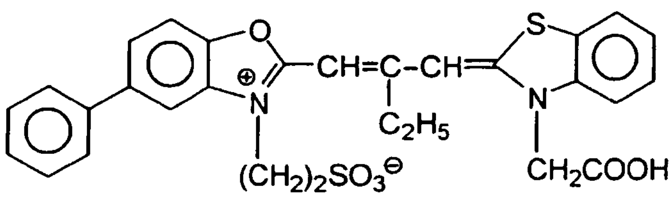

S-13

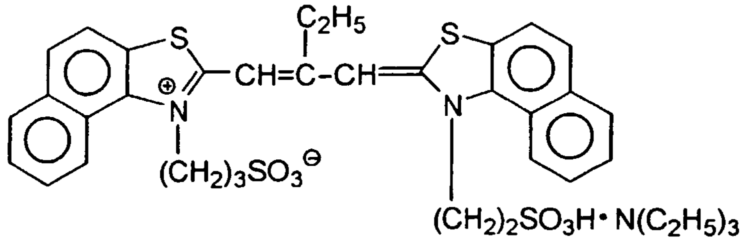

S-14

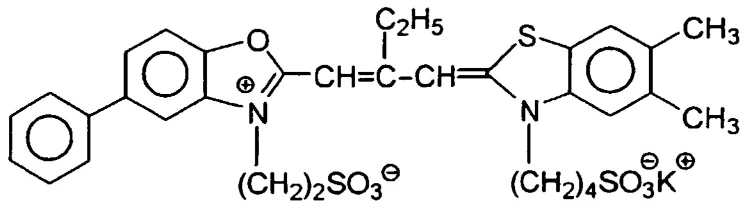

S-15

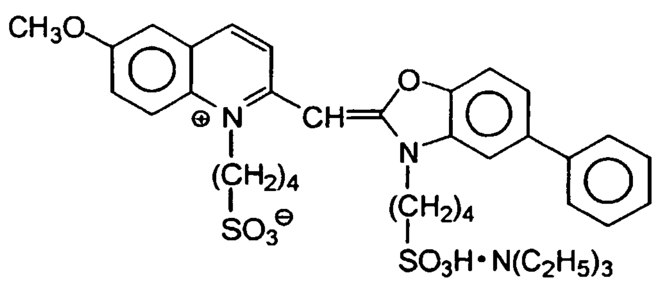

D-1

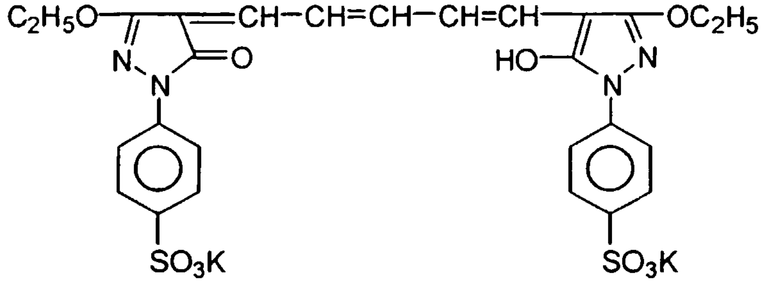

D-2

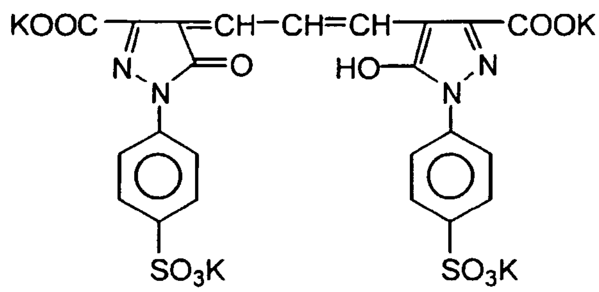

D-3

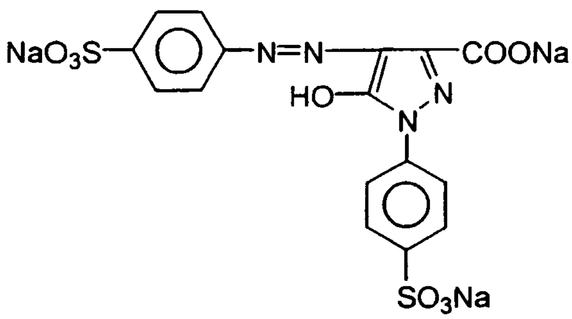

D-4

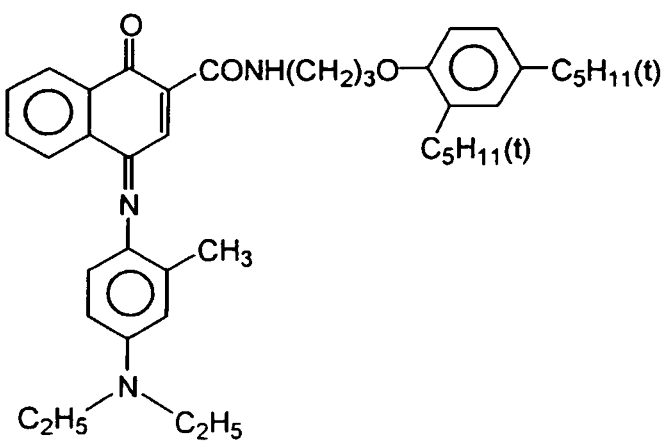

D-5

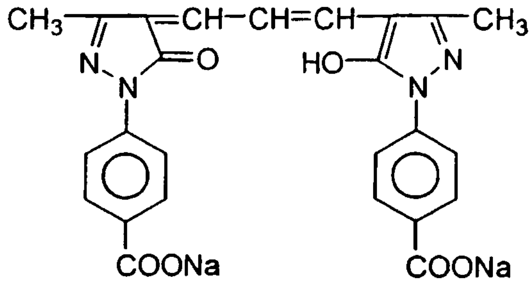

D-6

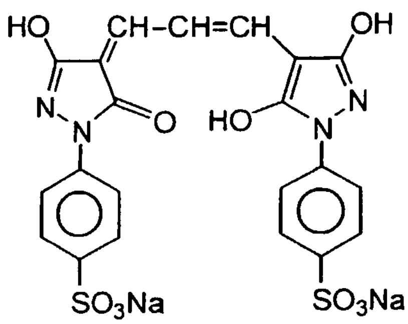

D-7

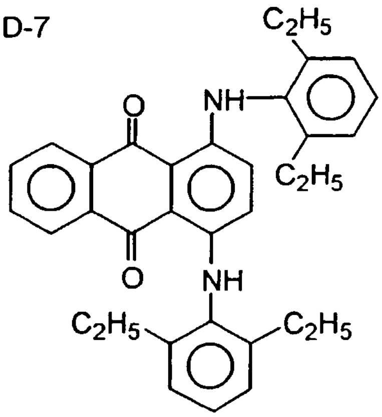

D-8

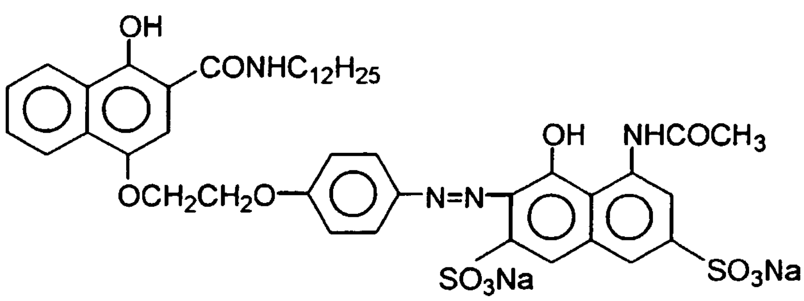

D-9

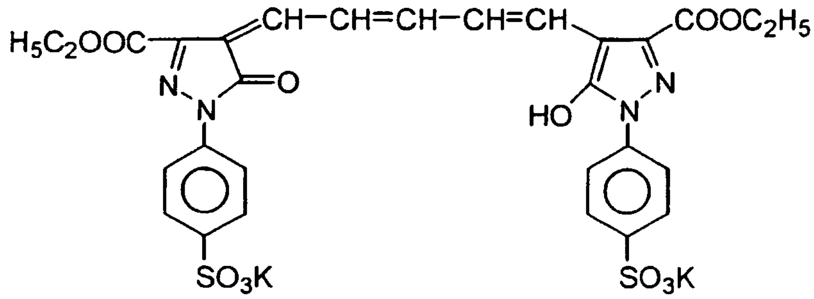

E-1

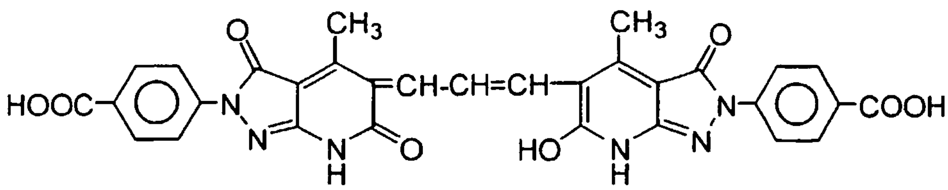

E-2

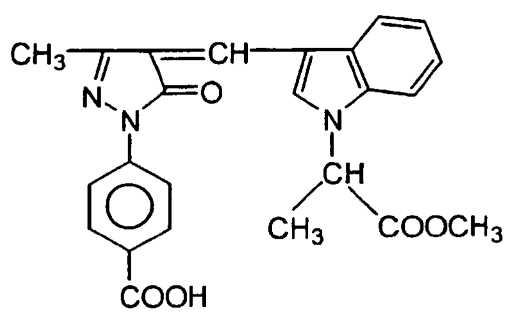

H-1

$CH_2=CH\text{-}SO_2\text{-}CH_2\text{-}CONH\text{-}CH_2$

$CH_2=CH\text{-}SO_2\text{-}CH_2\text{-}CONH\text{-}CH_2$

W-1

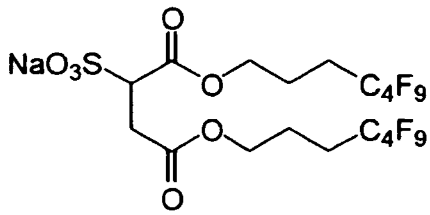

W-2

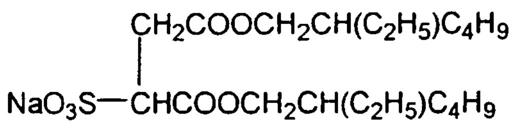

W-3

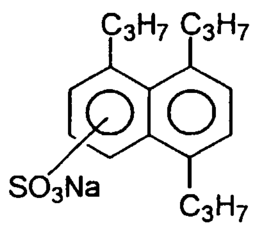

W-4

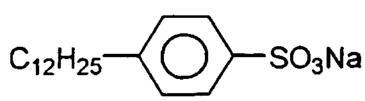

P-1

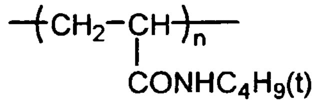

(n=100~1000)

P-2

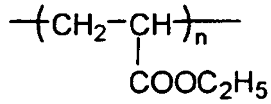

(n=100~1000)

P-3

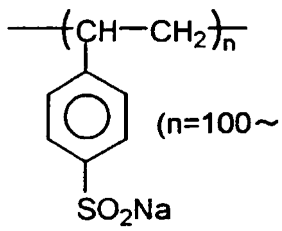

(n=100~1000)

F-1

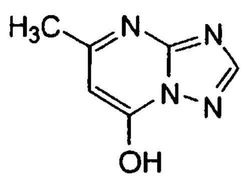

F-2

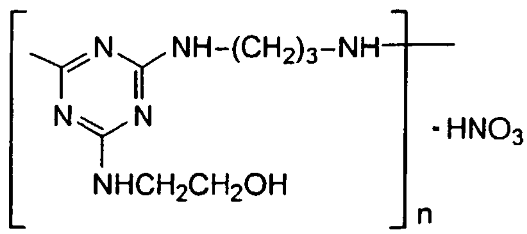

(n=3~4)

F-3

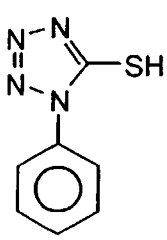

F-4

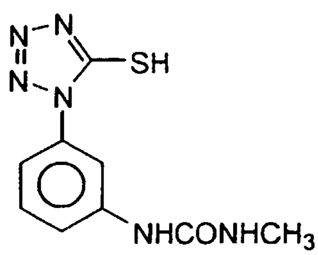

F-5

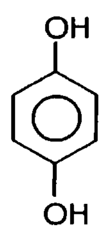

F-6

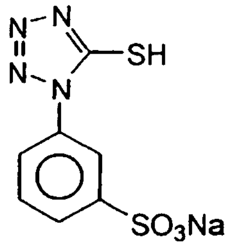

F-7

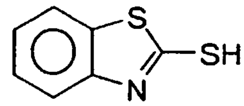

F-8

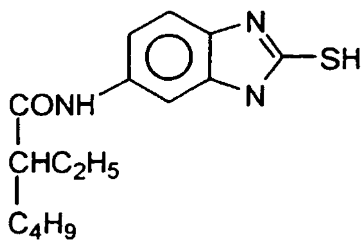

F-9

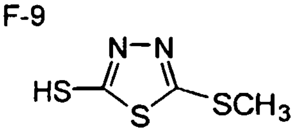

SO-1

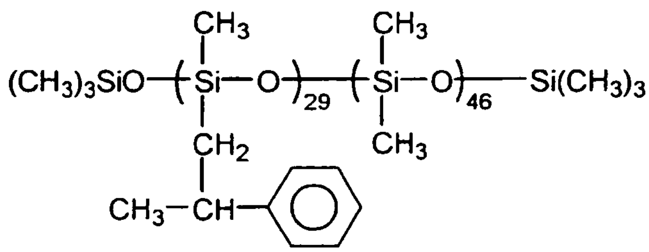

Preparation of dispersions of organic solid disperse dyes

(Preparation of fine-crystal solid dispersion of dye E-1)

100 g of Pluronic F88 (an ethylene oxide-propylene oxide block copolymer) manufactured by BASF CORP. and water were added to a wet cake of the dye E-1 (the net weight of E-1 was 270 g), and the resultant material was stirred to make 4,000 g. Next, the Ultra Visco Mill (UVM-2) manufactured by Imex K.K. was filled with 1,700 mL of zirconia beads with an average grain size of 0.5 mm, and the slurry was milled through the UVM-2 at a peripheral speed of approximately 10 m/sec and a discharge rate of 0.5 L/min for 2 hrs. The beads were filtered out, and water was added to dilute the material to a dye concentration of 3%. After that, the material was heated to 90°C for 10 hrs for stabilization. The average grain size of the obtained fine dye grains was 0.30 $\mu$m, and the grain size distribution (grain size standard deviation $\times$ 100/average grain size) was 20%.

(Preparation of solid dispersion of dye E-2)

Water and 270 g of W-4 were added to 1,400 g of a wet cake of E-2 containing 30 mass% of water, and the resultant material was stirred to form a slurry having an E-2 concentration of 40 mass%. Next, the Ultra Visco Mill (UVM-2) manufactured by Imex K.K. was filled with 1,700 mL of zirconia beads with an average grain size of 0.5 mm, and the slurry was milled through the UVM-2 at a peripheral speed of approximately 10 m/sec and a discharge rate of 0.5 L/min for 8 hr, thereby obtaining a solid fine-grain dispersion of E-2. This dispersion was diluted to 20 mass% by ion exchange water to obtain a fine-crystal solid dispersion. The average grain size was 0.15 $\mu$m.

Then, samples 102 to 113 were prepared in the same manner as in the preparation of the sample 101 except that supplemental addition or replacement by high-boiling organic solvents of the present invention or comparative high-boiling organic solvents were carried out in lightsensitive emulsion layers, as specified in Table 3.

Table 3 Structures of samples

| Sample | Remark | High-boiling organic solvent used | Layers changed | Manner of supplemental addition or replacement by the high-boiling organic solvent used |
|---|---|---|---|---|
| 101 | Comp. | the same as described in the specification | the same as described in the specification | the same as described in the specification |
| 102 | Comp. | Comp. Compound A | 5th, 6th and 7th layers | Supplemental addition in amount corresponding to 10 mass % of coupler C-1 contained in each layer. |
| 103 | Inv. | 1 | 5th, 6th and 7th layers | Supplemental addition in amount corresponding to 10 mass % of coupler C-1 contained in each layer. |
| 104 | Inv. | 4 | 5th, 6th and 7th layers | Supplemental addition in amount corresponding to 10 mass % of coupler C-1 contained in each layer. |
| 105 | Inv. | 6 | 5th, 6th and 7th layers | Supplemental addition in amount corresponding to 10 mass % of coupler C-1 contained in each layer. |
| 106 | Comp. | Comp. compound A | 10th, 11th and 12th layers | Supplemental addition in amount corresponding to 20 mass % of coupler C-4 contained in each layer. |
| 107 | Inv. | 1 | 10th, 11th and 12th layers | Supplemental addition in amount corresponding to 20 mass % of coupler C-4 contained in each layer. |
| 108 | Inv. | 4 | 10th, 11th and 12th layers | Supplemental addition in amount corresponding to 20 mass % of coupler C-4 contained in each layer. |
| 109 | Inv. | 1 | 10th, 11th and 12th layers | Replacement of high-boiling organic solvent Oil-2 contained in each layer at an equal mass. |
| 110 | Comp. | Comp. Compound A | 15th, 16th and 17th layers | Replacement of high-boiling organic solvent Oil-2 contained in each layer at an equal mass. |
| 111 | Inv. | 1 | 15th, 16th and 17th layers | Replacement of high-boiling organic solvent Oil-2 contained in each layer at an equal mass. |

(continued)

| Sample | Remark | High-boiling organic solvent used | Layers changed | Manner of supplemental addition or replacement by the high-boiling organic solvent used |
|---|---|---|---|---|
| 112 | Inv. | 4 | 15th, 16th and 17th layers | Replacement of high-boiling organic solvent Oil-2 contained in each layer at an equal mass. |
| 113 | Inv. | 1 | 5th, 6th 7th, 10th, 11th 12th, 15th, 16th and 17th layers | With respect to 5th, 6th and 7th layers, supplemental addition in amount corresponding to 10 mass % of coupler C-1 contained in each layer. coupler C-1 contained in each layer. With respect to 10th, 11th, 12th, 15th, 16th and 17th layers, replacement of high-boiling organic solvent Oil-2 contained in each layer at an equal mass. |

Each of the thus obtained samples 101 to 113 was cut into two strips and exposed to light with such an exposure intensity that the minimum density of the respective sample was realized. One thereof was subjected to the following development processing A, while the other strip was subjected to processing which was the same as the development processing A except that the washing temperature of the second washing was changed to 15˚C (development processing B).

The absorption spectra of the two sample strips after developments were taken, and an extinction coefficient difference at 510 nm was determined.

Relative values providing that the extinction coefficient difference at 510 nm between development processing A and development processing B with respect to the sample 101 is 1.0 are listed in Table 4. The smaller the relative value, the more favorably higher the resistance to change of the water temperature of the second washing.

Table 4 Result of estimation

| Sample | Remark | Extinction coefficient difference at 510 nm between development processing A and development processing B (Relative value providing that extinction coefficient difference with respect to sample 101 is 1.0) |
|---|---|---|
| 101 | Comp. | 1.0 (Standard) |
| 102 | Comp. | 1.05 |
| 103 | Inv. | 0.85 |
| 104 | Inv. | 0.87 |
| 105 | Inv. | 0.90 |
| 106 | Comp. | 1.08 |
| 107 | Inv. | 0.83 |
| 108 | Inv. | 0.85 |
| 109 | Inv. | 0.87 |
| 110 | Comp. | 1.0 |
| 111 | Inv. | 0.86 |
| 112 | Inv. | 0.88 |

(continued)

| Sample | Remark | Extinction coefficient difference at 510 nm between development processing A and development processing B (Relative value providing that extinction coefficient difference with respect to sample 101 is 1.0) |
|---|---|---|
| 113 | Inv. | 0.60 |

It is seen that the amide compounds of the present invention exert an effect of reducing white background dyeing, while the effect of reducing the residual amount of sensitizing dye to thereby enable an improvement to white background dyeing cannot be attained by the use of comparative compound A being an amide compound similar to the amide compounds of the present invention. This result is unexpected from prior known compounds.

The hues of cyan and magenta formed dyes were sharp and excellent. Further, when the amide compounds of the present invention were employed, the fastness of color images to heat and humidity was also excellent.

The ISO speeds of lightsensitive material samples 101 to 113, when having been processed according to development processing A, were in the range of 80 to 150. The respective maximum densities were in the range of 3.3 to 4.2 with respect to all of yellow, magenta and cyan, while the respective minimum densities were less than 0.20.

The development processing step shown below is the development processing A.

With respect to processing, after running processing was carried out until replenishment amount becomes 4 times the tank volume at a ratio 1:1 of an unexposed one to a completely exposed one of Sample 101, the processing for evaluation was carried out.

| Processing Step | Time | Temperature | Tank volume | Replenishment rate |
|---|---|---|---|---|
| 1st development | 6 min | 38˚C | 12 L | 2,200 mL/$m^2$ |
| 1st washing | 2 min | 38˚C | 4 L | 7,500 mL/$m^2$ |
| Reversal | 2 min | 38˚C | 4 L | 1,100 mL/$m^2$ |
| Color development | 6 min | 38˚C | 12 L | 2,200 mL/$m^2$ |
| Pre-bleaching | 2 min | 38˚C | 4 L | 1,100 mL/$m^2$ |
| Bleaching | 6 min | 38˚C | 12 L | 220 mL/$m^2$ |
| Fixing 1,100 mL/$m^2$ | 4 min | 38˚C | 8 L | |
| 2nd washing 7,500 mL/$m^2$ | 4 min | 40˚C | 8 L | |
| Final rinsing 1,100 mL/$m^2$ | 1 min | 25˚C | 2 L | |

The compositions of the processing solutions were as follows.

| <1st developer> | <Tank solution> | <Replenisher> |
|---|---|---|
| Nitrilo-N,N,N-trimethylene phosphonic acid · pentasodium salt | 1.5 g | 1.5 g |
| Diethylenetriamine pentaacetic acid· pentasodium salt | 2.0 g | 2.0 g |
| Sodium sulfite | 30 g | 30 g |
| Hydroquinone-potassium monosulfonate | 20 g | 20 g |
| Potassium carbonate | 15 g | 20 g |
| Potassium bicarbonate | 12 g | 15 g |
| 1-phenyl-4-methyl-4-hydroxymethyl-3-pyrazolidone | 1.5 g | 2.0 g |
| Potassium bromide | 2.5 g | 1.4 g |
| Potassium thiocyanate | 1.2 g | 1.2 g |
| Potassium iodide | 2.0 mg | - |
| Diethyleneglycol | 13 g | 15 g |
| Water to make | 1,000 mL | 1,000 mL |
| pH | 9.60 | 9.60 |

The pH was adjusted by sulfuric acid or potassium hydroxide.

| <Reversal solution> | <Tank solution> | <Replenisher> |
|---|---|---|
| Nitrilo-N,N,N-trimethylene phosphonic acid· pentasodium salt | 3.0 g | the same as tank solution |
| Stannous chloride·dihydrate | 1.0 g | |

(continued)

| <Reversal solution> | <Tank solution> | <Replenisher> |
|---|---|---|
| p-aminophenol | 0.1 g | |
| Sodium hydroxide | 8 g | |
| Glacial acetic acid | 15 mL | |
| Water to make | 1,000 mL | |
| pH | 6.00 | |

The pH was adjusted by acetic acid or sodium hydroxide.

| <Color developer> | <Tank solution> | <Replenisher> |
|---|---|---|
| Nitrilo-N,N,N-trimethylene phosphonic acid· pentasodium salt | 2.0 g | 2.0 g |
| Sodium sulfite | 7.0 g | 7.0 g |
| Trisodium phosphate· dodecahydrate Potassium bromide | 30 g 1.0 g | 30 g - |
| Potassium iodide | 90 mg | - |
| Sodium hydroxide | 8.0 g | 8.0 g |
| Citrazinic acid | 0.5 g | 0.5 g |
| N-ethyl-N-(β-methanesulfon amidoethyl)-3-methyl-4 aminoaniline·3/2 sulfuric acid·monohydrate | 9.0 g | 9.0 g |
| 3,6-dithiaoctane-1,8-diol | 0.6 g | 0.7 g |
| Water to make | 1,000 mL | 1,000 mL |
| pH | 11.80 | 12.00 |

The pH was adjusted by sulfuric acid or potassium hydroxide.

| <Pre-bleaching solution> | <Tank solution> | <Replenisher> |
|---|---|---|
| Ethylenediaminetetraacetic acid·disodium salt· dihydrate | 8.0 g | 8.0 g |
| Sodium sulfite | 6.0 g | 8.0 g |
| 1-thioglycerol | 0.4 g | 0.4 g |
| Formaldehyde sodium bisulfite adduct | 30 g | 35 g |
| Water to make | 1,000 mL | 1,000 mL |
| pH | 6.3 | 6.10 |

The pH was adjusted by acetic acid or sodium hydroxide.

| <Bleaching solution> | <Tank solution> | <Replenisher> |
|---|---|---|
| Ethylenediaminetetraacetic acid·disodium salt· dihydrate | 2.0 g | 4.0 g |
| Ethylenediaminetetraacetic acid·Fe(III)·ammonium· dihydrate Potassium bromide | 120 g 100 g | 240 g 200 g |
| Ammonium nitrate | 10 g | 20 g |
| Water to make | 1,000 mL | 1,000 mL |
| pH | 5.70 | 5.50 |

The pH was adjusted by nitric acid or sodium hydroxide.

| <Fixing solution> | <Tank solution> | <Replenisher> |
|---|---|---|
| Ammonium thiosulfate | 80 g | the same as tank solution |
| Sodium sulfite | 5.0 g | |
| Sodium bisulfite | 5.0 g | |
| Water to make | 1,000 mL | |
| pH | 6.60 | |

The pH was adjusted by acetic acid or ammonia water.

| <Stabilizer> | <Tank solution> | <Replenisher> |
|---|---|---|
| 1,2-benzoisothiazoline-3-one 0.02 g | | 0.03 g |
| Polyoxyethylene-p-monononyl phenylether (average polymerization degree = 10) | 0.3 g | 0.3 g |
| Polymaleic acid (weight-average molecular weight = 2,000) | 0.1 g | 0.15 g |
| Water to make | 1,000 mL | 1,000 mL |
| pH | 7.0 | 7.0 |

(Example 2)

Samples 201 to 213 were prepared by coating a 97 μm thick triacetylcellulose support with the same lightsensitive emulsion layers as employed in the samples 101 to 113, respectively (the sides opposite to the lightsensitive emulsion layers were coated with back layers of the same particulars as those applied to base D in Example 1 of JP-A-2002-62624).

Further, samples 301 to 313 were prepared by coating a 205 μm thick triacetylcellulose support with the above lightsensitive emulsion layers in the same manner.

Still further, samples 401 to 413 were prepared by coating a 200 μm thick polyethylene terephthalate support with the above lightsensitive emulsion layers in the same manner (the sides opposite to the lightsensitive emulsion layers were coated with back layers of the same particulars as those applied to base E in Example 1 of JP-A-2002-62624).

Yet still further, samples 501 to 513 were prepared by coating a 190 μm thick polyethylene naphthalate support with the above lightsensitive emulsion layers in the same manner (the sides opposite to the lightsensitive emulsion layers were coated with back layers of the same particulars as those applied to base E in Example 1 of JP-A-2002-62624).

The thus obtained samples 201 to 213, 301 to 313, 401 to 413 and 501 to 513 were evaluated in the same manner as in Example 1. The results showed that the samples of the present invention exhibit reduced white background staining and are excellent in both the hue of formed dye and the durability of color image.

(Example 3)

Samples exhibiting swelling ratios, as measured by swelling in 25°C distilled water, of 1.50, 1.60, 1.70, 1.80, 1.90, 2.00 and 2.10 were prepared from the same coating materials as used in the samples 101, 201, 301, 401, 501, 113, 213, 313, 413 and 513 of Examples 1 and 2 except that only the addition amount of hardener H-1 was increased or decreased, and evaluated in the same manner as in Example 1. The results showed that the samples of the present invention, irrespective of the change of swelling ratio value, exhibit reduced white background staining and are excellent in both the hue of formed dye and the durability of color image.

(Example 4)

Samples 601 to 613 were prepared in the same manner as in the preparation of the samples 101 to 113, respectively, except that the couplers of specified layers were changed as follows.

Table 5 Preparation of samples 601 to 613

| Sample | Couplers contained in 5th, 6th and 7th layers | Couplers contained in 10th, 11th and 12th layers | Couplers contained in 15th, 16th and 17th layers |
|---|---|---|---|
| 601-613 | C-1 contained in each layer was replaced with coupler C-3. (twice mol) | C-4 and C-5 contained in each layer were replaced with coupler C-7 (1.7 times mol) | C-10 contained in each layer was replaced with coupler C-8. (1.1 times mol) |

In comparisons among the samples 601 to 613 as well, the results showed that the samples of the present invention exhibit reduced white background staining and are excellent in both the hue of formed dye and the durability of color image.

## Claims

1. A silver halide color photographic lightsensitive material, **characterized by** comprising a compound of the following general formula (I):

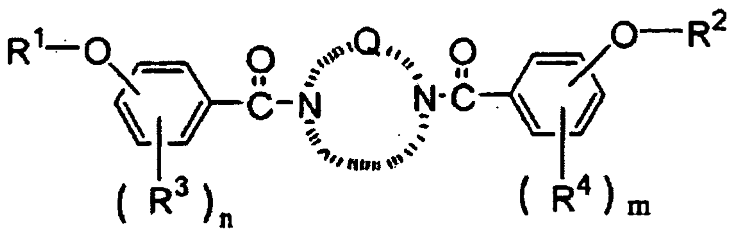

(I)

wherein:

each of $R^1$ and $R^2$ independently represents a substituted or unsubstituted alkyl group having 1 to 30 carbon atoms, or a substituted or unsubstituted alkenyl group having 3 to 30 carbon atoms;
each of $R^3$ and $R^4$ independently represents an unsubstituted alkyl group having 1 to 10 carbon atoms, a halogen atom or an unsubstituted alkoxy group having 1 to 10 carbon atoms;
each of n and m is independently an integer of 0 to 3; and
Q represents an atomic group needed to form a 5-membered or 6-membered heterocycle in cooperation with two nitrogen atoms.

2. The silver halide color photographic lightsensitive material according to claim 1, **characterized in that** a coupler of the following general formula (II) is contained:

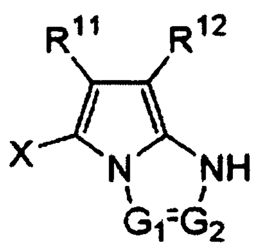

(II)

wherein:

X represents a hydrogen atom, or a group which may be split off at a coupling reaction with products of oxidation of an aromatic primary amine color developing agent;
each of $R^{11}$ and $R^{12}$ independently represents an electron withdrawing group whose Hammett substituent constant $\sigma p$ value is 0.20 or greater, provided that the sum of $R^{11}$ and $R^{12}$ $\sigma p$ values is 0.65 or greater; and
either of $G_1$ and $G_2$ is a nitrogen atom while the rest is $-C(R^{13})=$, wherein $R^{13}$ represents a hydrogen atom or a substituent.

3. A compound of the following general formula (I):

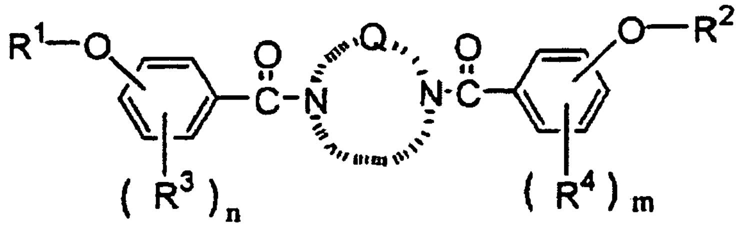

(I)

wherein:

each of $R^1$ and $R^2$ independently represents an unsubstituted alkyl group having 10 to 20 carbon atoms;
each of $R^3$ and $R^4$ independently represents an unsubstituted alkyl group having 1 to 10 carbon atoms, a halogen atom or an unsubstituted alkoxy group having 1 to 10 carbon atoms;
each of n and m is independently an integer of 0 to 2; and
Q represents an atomic group needed to form a 5-membered or 6-membered heterocycle in cooperation with two nitrogen atoms;
provided that the following compounds are not included:

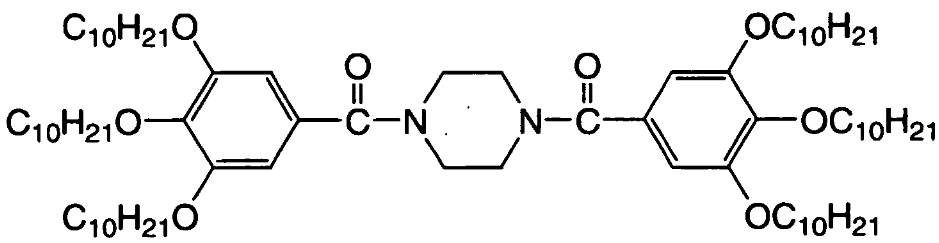

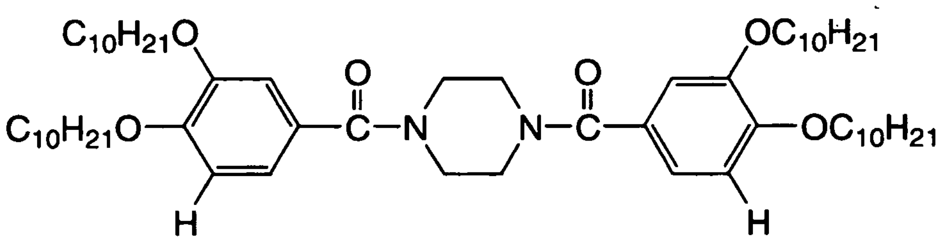

.

4. A method of forming an image,
**characterized by** comprising subjecting the silver halide color photographic lightsensitive material according to claim 2 to processes including reversal processing.

5. The silver halide color photographic lightsensitive material according to claim 1, wherein each of $R^1$ and $R^2$ independently represents an unsubstituted alkyl group having 10 to 20 carbon atoms.

6. The silver halide color photographic lightsensitive material according to claim 1 or 2, wherein m = n = O.

7. The compound according to claim 3, wherein m = n = O.

**Patentansprüche**

1. Farbfotografisches lichtempfindliches Silberhalogenidmaterial, **dadurch gekennzeichnet, daß** es eine Verbindung der folgenden allgemeinen Formel (I) umfaßt:

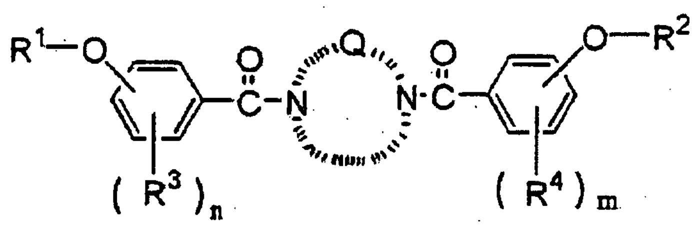

(I)

worin:

jedes von $R^1$ und $R^2$ unabhängig eine substituierte oder unsubstituierte Alkylgruppe mit 1 bis 30 Kohlenstoffatomen oder eine substituierte oder unsubstituierte Alkenylgruppe mit 3 bis 30 Kohlenstoffatomen repräsentiert;
jedes von $R^3$ und $R^4$ unabhängig eine unsubstituierte Alkylgruppe mit 1 bis 10 Kohlenstoffatomen, ein Halogenatom oder eine unsubstituierte Alkoxygruppe mit 1 bis 10 Kohlenstoffatomen repräsentiert;
jedes von n und m unabhängig eine ganze Zahl von 0 bis 3 ist; und
Q eine atomare Gruppe repräsentiert, die zum Bilden eines 5-gliedrigen oder 6-gliedrigen Heterocyclus zusammen mit zwei Stickstoffatomen erforderlich ist.

2. Farbfotografisches lichtempfindliches Silberhalogenidmaterial gemäß Anspruch 1, **dadurch gekennzeichnet, daß** ein Kuppler der folgenden allgemeinen Formel (II) enthalten ist:

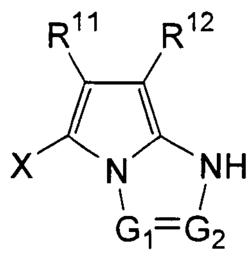

(II)

worin:

X ein Wasserstoffatom oder eine Gruppe, die bei einer Kupplungsreaktion mit Oxidationsproduktion eines aromatischen primären Amin-Farbentwicklungsmittels abgespalten werden kann, repräsentiert;
jedes von $R^{11}$ und $R^{12}$ unabhängig eine elektronenziehende Gruppe repräsentiert, deren Wert der Hammett-Substituentenkonstante $\sigma p$ 0,20 oder größer ist, vorausgesetzt, daß die Summe der $\sigma p$-Werte von $R^{11}$ und $R^{12}$ 0,65 oder größer ist; und
eines von $G_1$ und $G_2$ ein Stickstoffatom ist, während der Rest $-C(R^{13})=$ ist, worin $R^{13}$ ein Wasserstoffatom oder einen Substituenten repräsentiert.

3. Verbindung der folgenden allgemeinen Formel (I):

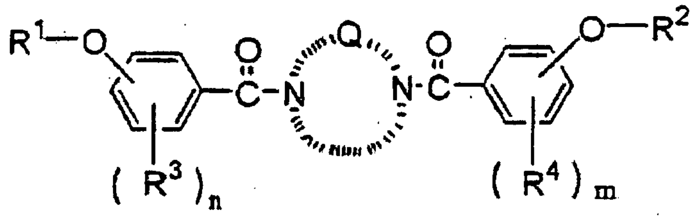

(I)

worin:

jedes von $R^1$ und $R^2$ unabhängig eine unsubstituierte Alkylgruppe mit 10 bis 20 Kohlenstoffatomen repräsentiert;
jedes von $R^3$ und $R^4$ unabhängig eine unsubstituierte Alkylgruppe mit 1 bis 10 Kohlenstoffatomen, ein Halogenatom oder eine unsubstituierte Alkoxygruppe mit 1 bis 10 Kohlenstoffatomen repräsentiert;
jedes von n und m unabhängig eine ganze Zahl von 0 bis 2 ist; und
Q eine atomare Gruppe repräsentiert, die zum Bilden eines 5-gliedrigen oder 6-gliedrigen Heterocyclus zusammen mit zwei Stickstoffatomen erforderlich ist;
vorausgesetzt, daß die folgenden Verbindungen nicht umfaßt sind:

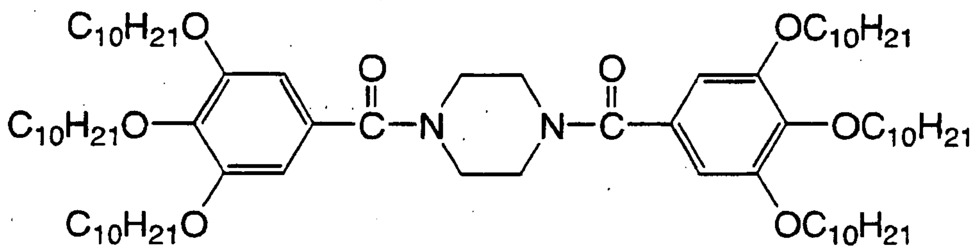

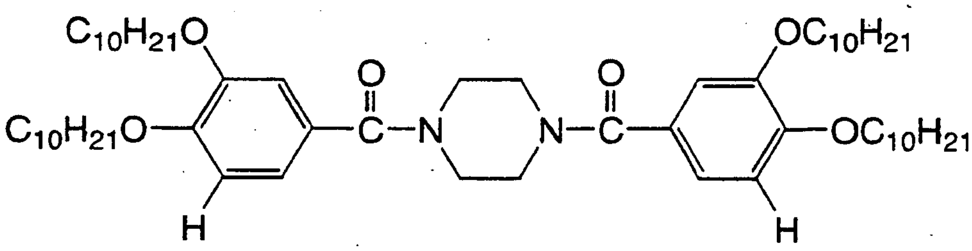

4. Verfahren zum Erzeugen eines Bildes, **dadurch gekennzeichnet, daß** es umfaßt, daß das farbfotografische lichtempfindliche Silberhalogenidmaterial gemäß Anspruch 2 Prozessen unterworfen wird, die ein Umkehrverarbeiten/entwickeln ("reversal processing") umfassen.

5. Farbfotografisches lichtempfindliches Silberhalogenidmaterial gemäß Anspruch 1, worin jedes von $R^1$ und $R^2$ unabhängig eine unsubstituierte Alkylgruppe mit 10 bis 20 Kohlenstoffatomen repräsentiert.

6. Farbfotografisches lichtempfindliches Silberhalogenidmaterial gemäß Anspruch 1 oder 2, worin m = n = 0.

7. Farbfotografisches lichtempfindliches Silberhalogenidmaterial gemäß Anspruch 3, worin m = n = 0.

## Revendications

1. Matériau photographique couleur à l'halogénure d'argent photosensible, **caractérisé en ce qu'**il comprend un composé ayant la formule générale (I) suivante :

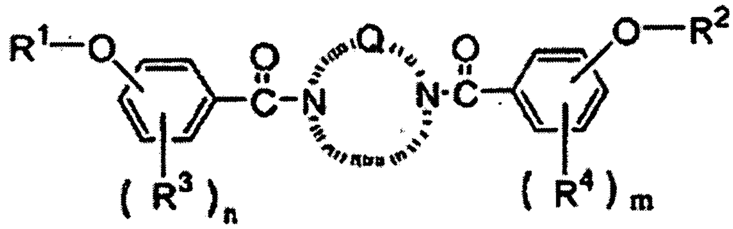

(I)

dans laquelle :

chacun de $R^1$ et $R^2$ indépendamment représente un groupe alkyle substitué ou non substitué ayant 1 à 30 atomes de carbone, ou un groupe alcényle substitué ou non substitué ayant 3 à 30 atomes de carbone ;
chacun de $R^3$ et $R^4$ indépendamment représente un groupe alkyle non substitué ayant 1 à 10 atomes de carbone, un atome d'halogène ou un groupe alcoxy non substitué ayant 1 à 10 atomes de carbone ;
chacun de n et m est indépendamment un nombre entier compris entre 0 et 3 ; et
Q représente un groupe atomique nécessaire pour former un hétérocycle à 5 ou à 6 membres en coopération avec deux atomes d'azote.

2. Matériau photographique couleur à l'halogénure d'argent photosensible selon la revendication 1, **caractérisé en ce qu'** un coupleur ayant la formule générale (II) suivante y est contenu :

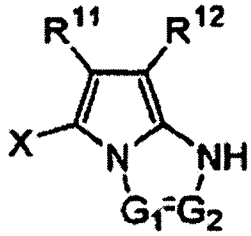

(II)

dans laquelle :

X représente un atome d'hydrogène, ou un groupe qui peut être séparé par scission lors d'une réaction de couplage avec des produits d'oxydation d'un agent révélateur de couleur à amine primaire aromatique ;
chacun de $R^{11}$ et $R^{12}$ indépendamment représente un groupe enlevant des électrons dont la valeur σp de la constante de Hammett de substituant est égale ou supérieure à 0,20, à la condition que la somme des valeurs σp de $R^{11}$ et $R^{12}$ soit égale ou supérieure à 0,65 ; et
l'un parmi $G_1$ et $G_2$ est un atome d'azote tandis que le reste est $-C(R^{13})=$, où $R^{13}$ représente un atome d'hydrogène ou un substituant.

3. Composé ayant la formule générale (I) suivante :

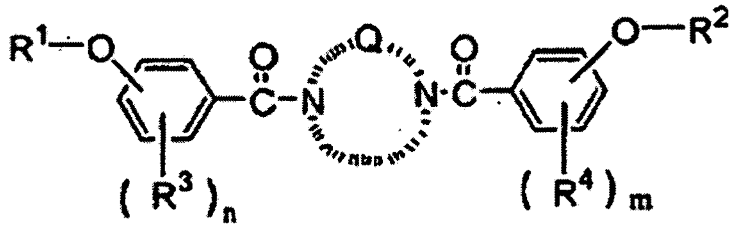

(I)

dans laquelle :

chacun de $R^1$ et $R^2$ indépendamment représente un groupe alkyle non substitué ayant 10 à 20 atomes de carbone ;
chacun de $R^3$ et $R^4$ indépendamment représente un groupe alkyle non substitué ayant 1 à 10 atomes de carbone, un atome d'halogène ou un groupe alcoxy non substitué ayant 1 à 10 atomes de carbone ;
chacun de n et m est indépendamment un nombre entier compris entre 0 et 2 ; et
Q représente un groupe atomique nécessaire pour former un hétérocycle à 5 ou à 6 membres en coopération avec deux atomes d'azote ;
à la condition que les composés suivants ne soient pas inclus:

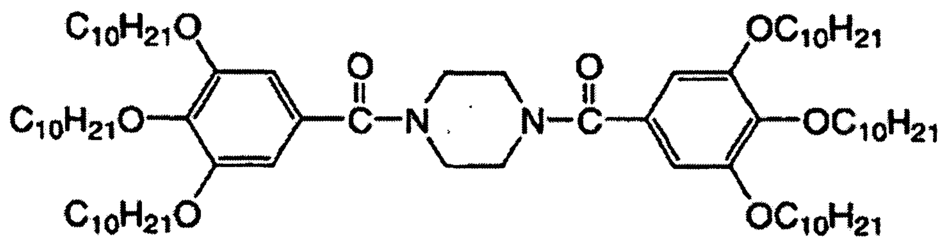

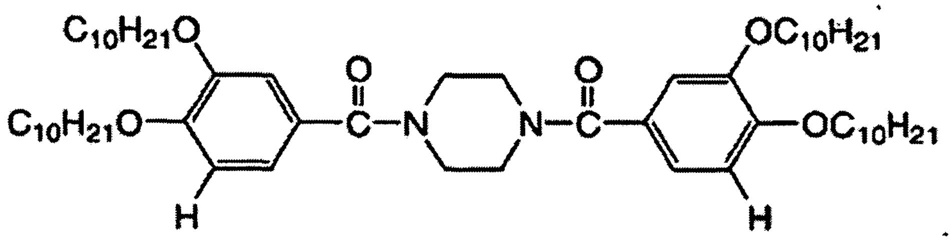

4. Procédé de formation d'une image, **caractérisé en ce qu'**il comprend la soumission du matériau photographique couleur à l'halogénure d'argent photosensible selon la revendication 2 à des procédés incluant un traitement d'inversion.

5. Matériau photographique couleur à l'halogénure d'argent photosensible selon la revendication 1, dans lequel chacun de $R^1$ et $R^2$ indépendamment représente un groupe alkyle non substitué ayant 10 à 20 atomes de carbone.

6. Matériau photographique couleur à l'halogénure d'argent photosensible selon la revendication 1 ou 2, dans lequel $m = n = 0$.

7. Composé selon la revendication 3, dans lequel $m = n = 0$.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2262654 A
- JP 8110624 A
- EP 0249453 A2
- US 5256526 A
- EP 0545300 A
- JP 06157442 B
- US 5418122 A
- JP 2001163887 A
- DE 1121470
- GB 923045 A
- JP 55034932 B
- JP 49015495 B
- JP 59202464 A
- US 4663271 A
- US 4705744 A
- US 4707436 A
- JP 62160448 A
- JP 63089850 A
- US 3574628 A
- US 3655394 A
- GB 1413748 A
- US 4434226 A
- US 4414310 A
- US 4433048 A
- US 4439520 A
- GB 2112157 A
- JP 63264740 A
- JP 59133542 A
- US 4082553 A
- US 4626498 A
- JP 59214852 A
- EP 502424 A
- EP 513496 A
- EP 568037 A
- US 5066576 A
- JP 4274425 A
- EP 498381 A1
- EP 447969 A1
- US 4476219 A
- JP 3039737 A
- EP 456257 A
- EP 486965 A
- EP 571959 A
- JP 5204106 A
- JP 4362631 A
- JP 4204843 A
- JP 4043345 A
- JP 6067385 A
- JP 2044345 A
- US 4366237 A
- GB 2125570 A
- EP 96873 B
- DE 3234533
- EP 456257 A1
- US 4833069 A
- US 4837136 A
- WO 9211575 A
- EP 378236 A1
- EP 436938 A2
- EP 440195 A2
- EP 310125 A2
- JP 6059411 A
- US 4555478 A
- US 4749641 A
- US 4774181 A
- US 4656123 A
- EP 450637 A2
- US 4857447 A
- JP 62215272 A
- US 4199363 A
- US 4978606 A
- US 4923787 A
- EP 298321 A
- US 5122444 A
- EP 471347 A
- US 5139931 A
- EP 411324 A
- EP 477932 A
- JP 1214845 A
- US 4618573 A
- JP 2214852 A
- US 3325287 A
- JP 62168139 A
- US 5019492 A
- US 4923790 A
- US 4923793 A
- US 4952483 A
- JP 5040324 A
- JP 3156450 A
- EP 445627 A
- EP 457153 A
- WO 8804794 A
- EP 319999 A
- EP 519306 A
- US 4268622 A
- US 4923788 A
- JP 46003335 A
- EP 520938 A

- EP 521823 A
- JP 4121739 A
- JP 4125558 A
- JP 5173312 A
- JP 4251845 A
- JP 4268552 A
- EP 588289 A
- EP 591934 A
- JP 6208213 A
- JP 53095630 A
- US 3893858 A
- JP 6301169 A
- JP 1224762 A
- JP 1309059 A
- EP 504609 A
- EP 519190 A
- JP 4362943 A
- JP 6289559 A
- JP 3046652 A
- JP 3053246 A
- JP 3055542 A
- JP 3121448 A
- JP 3126030 A
- JP 63017453 A
- JP 4019655 A
- JP 4230748 A
- JP 4221951 A
- JP 51061837 A
- JP 6102628 A
- JP 2002062624 A

**Non-patent literature cited in the description**


- **G. LATTERMAN et al.** Journal of the Chemical Society. *Chem. Communications,* 1992, 1091-1092
- Lange's Handbook of Chemistry. Kagaku no Ryoiki. Mc Graw-Hill, 1979, 96-103
- I. Emulsion preparation and types. *Research Disclosure,* December 1978, 22-23
- *RESEARCH DISCLOSURE,* November 1979, 648
- *RESEARCH DISCLOSURE,* November 1989, 863-865
- **P. GLAFKIDES.** *Chemie et Phisique Photographique,* 1967
- **G.F. DUFFIN.** Photographic Emulsion Chemistry. Focal Press, 1966
- **V.L. ZELIKMAN et al.** *Making and Coating Photographic Emulsion,* 1964
- **GUTOFF.** *Photographic Science and Engineering,* 1970, vol. 14, 248-257
- **A. GREEN et al.** *Photogr. Sci. Eng,* vol. 19 (2), 124-129